(19) Europäisches Patentamt
European Patent Office
Office européen des brevets

(11) **EP 2 024 022 B1**

(12) **EUROPÄISCHE PATENTSCHRIFT**

(45) Veröffentlichungstag und Bekanntmachung des Hinweises auf die Patenterteilung:
**08.12.2010 Patentblatt 2010/49**

(21) Anmeldenummer: **07764867.3**

(22) Anmeldetag: **26.06.2007**

(51) Int Cl.:
*A61N 1/36* (2006.01)     *G10L 15/20* (2006.01)
*H04R 25/00* (2006.01)

(86) Internationale Anmeldenummer:
**PCT/EP2007/005649**

(87) Internationale Veröffentlichungsnummer:
**WO 2008/000444 (03.01.2008 Gazette 2008/01)**

(54) **VORRICHTUNG UND VERFAHREN ZUM ERZEUGEN EINES GEFILTERTEN AKTIVITÄTSMUSTERS, QUELLENTRENNER, VERFAHREN ZUM ERZEUGEN EINES BEREINIGTEN AUDIOSIGNALS UND COMPUTERPROGRAMM**

DEVICE AND METHOD FOR PRODUCING A FILTERED ACTIVITY PATTERN, SOURCE SEPARATOR, METHOD FOR PRODUCING A CORRECTED AUDIO SIGNAL AND COMPUTER PROGRAM

DISPOSITIF ET PROCÉDÉ POUR GÉNÉRER UN MODÈLE D'ACTIVITÉ FILTRÉ, SÉPARATEUR DE SOURCES, PROCÉDÉ POUR GÉNÉRER UN SIGNAL AUDIO ÉPURÉ, ET PROGRAMME INFORMATIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **30.06.2006 DE 102006030276**

(43) Veröffentlichungstag der Anmeldung:
**18.02.2009 Patentblatt 2009/08**

(73) Patentinhaber: **Fraunhofer-Gesellschaft zur Förderung der angewandten Forschung e.V.
80686 München (DE)**

(72) Erfinder: **KLEFENZ, Frank
68159 Mannheim (DE)**

(74) Vertreter: **Zinkler, Franz et al
Schoppe, Zimmermann, Stöckeler & Zinkler
Patentanwälte
Postfach 246
82043 Pullach bei München (DE)**

(56) Entgegenhaltungen:
US-A- 5 434 924      US-A1- 2002 012 438
US-A1- 2003 115 054    US-A1- 2003 171 786
US-A1- 2005 069 162    US-A1- 2005 177 205
US-A1- 2005 192 646    US-B1- 6 987 856

**Beschreibung**

Die vorliegende Erfindung bezieht sich im Allgemeinen auf eine Vorrichtung und ein Verfahren zum Erzeugen eines gefilterten Aktivitätsmusters, auf einen Quellentrenner, auf ein Verfahren zum Erzeugen eines bereinigten Audiosignals und auf ein Computerprogramm, im Speziellen auf einem Konzept zur Rauschquellenfilterung.

Im Bereich der heutigen Medizintechnik stellt es eine wichtige Herausforderung dar, gehörgeschädigten Menschen eine Teilnahme am normalen Leben zu ermöglichen. Zu diesem Zweck sind aus der Medizintechnik eine große Anzahl an verschiedenen Hörhilfen bekannt. Eine besondere Herausforderung besteht, falls das Innenohr eines Patienten geschädigt ist. In diesem Fall ist es erforderlich, die Hörnerven des Patienten direkt anzuregen.

Wenngleich dies mit Hilfe von Cochlea-Implantaten bereits erfolgreich erreicht werden kann, so haben Patienten mit Cochlea-Implantaten doch besondere Schwierigkeiten, wenn sie sich in der Nähe mehrerer verschiedener Schallquellen befinden. In diesem Fall nimmt unter anderem eine Sprachverständlichkeit deutlich ab.

Aus diesem Grund besteht ein Bedürfnis, den Höreindruck von Patienten mit einem Cochlea-Implantat gerade in solchen Situationen, in denen mehrere Schallquellen vorhanden sind, zu verbessern.

Im Folgenden wird auf einige Dokumente eingegangen, die Hintergrundinformationen zu der genannten Problematik liefern.

So beschreibt der Artikel "A revised neurobiologically parameterized model of the cochlea and an attached auditory image processing network" von T. Harczos (Doktotstudent im zweiten Jahr) und seinem Betreuer, Dr. T. Roska (PPCU Multidisciplinary Doctoral School, 2005-2006 annual report) ein neurobiologisch parametrisiertes Model der Cochlea. In dem beschriebenen Model wird eine Basilarmembran-Modellierung gemäß einem erweiterten Zwicker/Baumgarte-Modell eingesetzt. Ferner wird eine innere Haarzelle unter Verwendung des Models von Meddis nachgebildet, wodurch eine Vesikel-Freisetzung berechnet wird. Ferner wird zusätzlich der synaptische Spalt modelliert, und es werden weiterhin auch Vorgänge nach dem synaptischen Spalt modelliert.

Weitere Informationen im Hinblick auf eine Verarbeitung von Schalldaten unter Verwendung einer Hough-Transformation finden sich beispielsweise in dem Artikel "A neurobiologically inspired vowel recognizer using Hough-transform" von T. Harczos, F. Klefenz und A. Kátai (veröffentlicht in den Proceedings VISAPP 2006, Setubal, Portugal, 25. - 28. Februar 2006).

Der Hauptbeitrag SCHALLANALYSE mit dem Titel "Neuronale Repräsentation des Hörvorgangs als Basis" von G. Szepannek, F. Klefenz und C. Weihs, online publiziert am 28. September 2005, Informatik-Spektrum, Springer-Verlag GmbH, ISSN: 0170-6012 (Paper), beschreibt eine Modellierung einer Antwort eines auditorischen Nervs sowie eine Informationsextraktion.

Weitere Informationen finden sich in dem Artikel "Feature Extraction for sound classification by means of a perceptionally motivated neurophysiologic parameterized auditory model" von T. Harczos, A. Katai, F. Klefenz, P. Schikowski und G. Szepannek (veröffentlicht auf der Konferenz der Gesellschaft für Klassifikation GfKl 2006 vom 8. März bis 10. März 2006 in Berlin).

Ferner beschreibt die nicht-vorveröffentlichte deutsche Patentanmeldung mit dem amtlichen Aktenzeichen DE 2005 10 030 327 ein Konzept zur Analyse eines Audiosignals. Die genannte Patentanmeldung, zu der im Übrigen eine internationale Anmeldung WO 2007/000231 A1 existiert, beschreibt ein Verfahren und ein Computerprogramm zur Analyse eines Audiosignals, um eine Analysedarstellung des Audiosignal zu erhalten. Ferner beschreibt die genannte Schrift ein Konzept zur neurophysiologisch parametrisierten Simulation der ersten Stufen des Hörsystems.

In ähnlicher Weise beschreibt die deutsche Patentanmeldung mit dem amtlichen Aktenzeichen 10 2005 030 327 einen Einsatz eines neurophysiologischen Gehörmodels und eine Erzeugung von Signalen basierend darauf. Zu der genannten Schrift existiert im übrigen eine parallele US-Anmeldung mit dem amtlichen Aktenzeichen 11/172,605.

Im übrigen beschreibt die US 6,442,510 B1 ein Konzept zur Bestimmung eines Laufzeit-Differentials für Signalwellenformen für eine Realzeit-Mustererkennung, Lokalisierung und Überwachung von optischen und akustischen Signalen. Das genannte Konzept umfasst Schritte eines segmentweisen Detektierens und In-Koinzidenz-Bringen von Signalwellenformen für eine Umwandlung in monotone und kontinuierliche Trajektorien, für eine Realzeit-Mustererkennung, sowie für eine Lokalisierung und Überwachung von optischen und akustischen Signalen. Das in der genannten Schrift beschriebene Verfahren bestimmt Laufzeit-Differentiale, wobei vorprogrammierte Schlüsselsignale durch eine Signalabtastung detektiert werden. Daten werden von den abgetasteten Signalen korrigiert und Paare von Signalkombinationen von gegebenen Signallaufzeitdifferenzialen werden aus der Koinzidenz der detektierten Signale bestimmt.

Die Vorrichtung umfasst, zumindest zwei Empfänger zum Erzeugen von Sequenzen von digitalen Werten aus einlaufenden akustischen Signalen. Die Vorrichtung umfasst ferner Vektor-Generatoren, um die digitalen Werte in Eingangsvektoren zu formen, eine Signaldetektionseinheit, die nach jedem Vektorgenerator angeordnet ist, und die parallele, programmierbare Signalflussketten sowie Addierer/Komparator-Einheiten aufweist. Die Addierer/Komparator-Einheiten sind senkrecht zu den Signalflussketten in äquidistanten Abständen angeordnet. Die Vorrichtung umfasst ferner eine Multi-Koinzidenz-Einheit, bestehend aus zwei anti-parallelen Schieberegistern, die Flip-Flop-Ketten bilden, sowie aus UND-Gattern.

In Anbetracht des bekannten Stands der Technik ist es die Aufgabe der vorliegenden Erfindung, ein Gehör-angepasstes Konzept zum Erzeugen einer gefilterten Darstellung eines Audiosignals zu schaffen, so dass ein Einfluss von Stör-Schallquellen in der gefilterten Darstellung verringert ist.

Diese Aufgabe wird durch eine Vorrichtung zum Erzeugen eines gefilterten Aktivitätsmusters gemäß dem Patentanspruch 1, einen Quellentrenner gemäß dem Patentanspruch 12, ein Verfahren zum Erzeugen eines gefilterten Aktivitätsmusters gemäß dem Patentanspruch 13, ein Verfahren zum Erzeugen eines bereinigten Audiosignals gemäß Anspruch 14, sowie durch ein Computerprogramm gemäß dem Patentanspruch 15 gelöst.

Die vorliegende Erfindung schafft eine Vorrichtung zum Erzeugen eines gefilterten Aktivitätsmusters gemäß dem Patentanspruch 1.

Es ist der Kerngedanke der vorliegenden Erfindung, dass ein gefiltertes Aktivitätsmuster in besonders zuverlässiger

Weise basierend auf einem ersten Aktivitätsmuster an einem Gehörmodel eines ersten Ohrs und einem zweiten Aktivitätsmuster an einem Gehörmodel eines zweiten Ohrs erzeugt werden kann, indem in dem ersten Aktivitätsmuster und in dem zweiten Aktivitätsmuster Trajektorien identifiziert werden, die einem gleichen Schallereignis zugeordnet sind, indem bestimmt wird, ob die zwei identifizierten Trajektorien einem Schallereignis einer Nutz-Schallquelle zugeordnet sind, und indem das erste Aktivitätsmuster oder das zweite Aktivitätsmuster basierend auf dem Ergebnis des Bestimmens, ob eine Trajektorie einem Schallereignis der Nutz-Schallquelle (oder einem Schallereignis einer Stör-Schallquelle) zugeordnet ist, erhalten wird.

Es hat sich nämlich gezeigt, dass durch die Verwendung von zwei Aktivitätsmustern, die an einem Gehörmodel eines ersten Ohres und einem Gehörmodel eines zweiten Ohres gebildet sind, eine besonders präzise Information darüber erzielbar ist, ob zwei Trajektorien, die in beiden Aktivitätsmustern auftreten, und die einem gleichen Schallereignis zugeordnet sind, von einer Nutz-Schallquelle oder einer Stör-Schallquelle stammen. Sind nämlich eine erste Trajektorie basierend auf dem Gehörmodel des ersten Ohres und eine zweite Trajektorie basierend auf dem Gehörmodel des zweiten Ohres bestimmt, wobei die Trajektorien zu dem gleichen Schallereignis gehören, so ist durch den Vergleich der beiden Trajektorien in besonders einfacher und zuverlässiger Weise erkennbar, ob die Trajektorien von einer Nutz-Schallquelle oder von einer Stör-Schallquelle stammen.

Der Grund dafür besteht darin, dass Trajektorien, die von einer Nutz-Schallquelle stammen, an den beiden Ohren typischerweise in anderer Weise gegeneinander verzerrt bzw. zeitlich verschoben sind als Trajektorien, die zu dem Stör-Schallereignis gehören. Augrund der Verzerrung bzw. Verschiebung von zwei Trajektorien, die zu dem gleichen Schallereignis gehören, in den beiden Aktivitätsmustern ist somit eine besonders einfache Zuordnung möglich, ob die Trajektorien einem Nutz-Schallereignis oder einer Stör-Schallereignis zugeordnet sind.

Eine Kenntnis darüber, ob eine Trajektorie einem Nutz-Schallereignis oder einem Stör-Schallereignis (bzw. einer Nutz-Schallquelle oder einer Stör-Schallquelle) zugeordnet ist, wird im übrigen im Rahmen des erfindungsgemäßen Filters ausgenutzt, um das gefilterte Aktivitätsmuster aus dem ersten Aktivitätsmuster oder aus dem zweiten Aktivitätsmuster derart zu erhalten, dass in dem gefilterten Aktivitätsmuster Aktivitätsereignisse, die einem Schallereignis der Nutz-Schallquelle zugeordnet sind, gegenüber Schallereignissen, die einer Stör-Schallquelle zugeordnet sind, dominieren, oder dass in dem gefilterten Aktivitätsmuster Aktivitätsereignisse, die nicht einem Schallereignis der Nutz-Schallquelle zugeordnet sind, nicht mehr vorhanden bzw. entfernt sind.

In anderen Worten, das erfindungsgemäße Konzept besteht im Wesentlichen darin, durch einen Vergleich bzw. durch eine Bestimmung einer Verzerrung oder zeitlichen Verschiebung von Trajektorien in dem ersten Aktivitätsmuster und in dem zweiten Aktivitätsmuster, die einem gleichen Schallereignis zugeordnet sind, zu bestimmen, ob die Trajektorien zu einem Nutz-Schallereignis von einer Nutz-Schallquelle oder zu einem Stör-Schallereignis von einer Stör-Schallquelle gehören, und um das erste Aktivitätsmuster oder das zweite Aktivitätsmuster basierend auf der genannten Information zu filtern, um das gefilterte Aktivitätsmuster zu erhalten.

Ein wesentlicher Vorteil der Vorliegenden Erfindung besteht somit darin, dass bei einer Erzeugung eines gefilterten Aktivitätsmuster von zwei Ohren herangezogen werden. Zur Bestimmung, ob eine Trajektorie einer Nutz-Schallquelle oder einer Stör-Schallquelle zugeordnet ist, wird somit die gesamte verfügbare Information von zwei Gehörmodellen (z.B. eines linken Ohres und eines rechten Ohres) herangezogen. Für die Identifizierung von Trajektorien von Nutz-Schallquellen bzw. von Stör-Schallquellen können somit Relationen zwischen zusammengehörigen (zu dem gleichen Schallereignis gehörigen) Trajektorien des ersten Aktivitätsmusters und des zweiten Aktivitätsmusters herangezogen. Dadurch wird eine besonders effiziente und zuverlässige Unterscheidung von Trajektorien von Nutz-Schallquellen und von Stör-Schallquellen erreicht, die im übrigen einer binauralen Verarbeitung von akustischen Signalen in dem menschlichen Gehirn nachempfunden ist.

Erfindungsgemäß beinhaltet die Vorrichtung einem Identifizierer, um eine zeitliche Verschiebung zwischen den zwei identifizierten Trajektorien, die dem gleichen Schallereignis zugeordnet sind, zu bestimmen. In diesem Fall ist der Bestimmer ausgelegt, um anhand der zeitlichen Verschiebung festzustellen, ob zwei Trajektorien, die dem gleichen Schallereignis zugeordnet sind, einem Schallereignis der Nutz-Schallquelle oder einem Schallereignis der Stör-Schallquelle zugeordnet sind.

Das beschriebene Konzept basiert auf der Erkenntnis, dass gerade eine zeitliche Verschiebung zwischen zwei Trajektorien ein besonders unterscheidungskräftiges Merkmal ist, das es erlaubt, Trajektorien die von einem Nutz-Schallereignis bzw. einer Nutz-Schallquelle stammen, von Trajektorien, die von einer Stör-Schallquelle bzw. einem Stör-Schallereignis stammen, zu trennen. Die zeitliche Verschiebung zwischen den Trajektorien ist nämlich ein Maß für eine räumliche Lage der Schallquelle. Eine zeitliche Verschiebung entsteht typischerweise durch eine Laufzeitdifferenz zwischen der Schallquelle und dem ersten Ohr sowie zwischen der Schallquelle und dem zweiten Ohr. Diese Laufzeitdifferenz hängt von einer Lage der Schallquelle relativ zu den beiden (zueinander beabstandeten) Ohren ab. Ferner hat sich im übrigen gezeigt, dass gerade die Laufzeitdifferenz, also die Richtung, aus der Schall von einer Nutz- Schallquelle bzw. Stör-Schallquelle eintrifft, ein besonders wichtiges und effizientes Merkmal zum Trennen von Nutz- Schallquellen und Stör-Schallquellen ist, da es typischerweise zu guten Resultaten führt, Schallereignisse aus genau einer Richtung bzw. aus einem begrenzten (gegebenenfalls auch unzusammenhängenden) Winkelbereich aufzunehmen, und Schallereignisse, die ihren Ursprung außerhalb des genannten Winkelbereichs haben, als Stör-Schallquellen zu betrachten. Daher wird es bevorzugt, Aktivitätsereignisse, die ihren Ursprung außerhalb des genannten Winkelbereichs haben, zu reduzieren oder zu unterdrücken.

Die vorliegende Erfindung schafft ferner ein entsprechendes Verfahren zum Erzeugen eines gefilterten Aktivitätsmusters gemäß dem Patentanspruch 13.

Im Hinblick auf die diesem Verfahren zugrundeliegende erfindungsgemäße Erkenntnis und auf die Vorteile des erfindungsgemäßen Verfahren gegenüber herkömmlichen Verfahren wird im übrigen auf die Ausführungen im Hinblick auf die Vorrichtung gemäß dem Anspruch 1 verwiesen.

Die vorliegende Erfindung schafft ferner einen Quellentrenner gemäß dem Anspruch 12.

Gemäß einem Kerngedanken der vorliegenden Erfindung hat es sich gezeigt, dass es zur Trennung von mehreren Quellen, beispielsweise einer Nutz-Schallquelle und einer Stör-Schallquelle, in einem zumindest zweikanaligen Audiosignal vorteilhaft ist, die beiden Kanäle des Audiosignals zunächst getrennt in Aktivitätsmuster an einem Gehörmodell eines ersten Ohres und an einem Gehörmodel eines zweiten Ohres zu wandeln. Basierend auf den Aktivitätsmustern werden sodann Trajektorien in den Aktivitätsmustern erkannt, die einem gleichen Schallereignis zugeordnet sind. Basierend auf der Erkennung bzw. Identifizierung von zwei Trajektorien, die dem gleichen Schallereignis zugeordnet sind, wird sodann, wie dies bereits oben erläutert wurde, bestimmt, ob die zwei Trajektorien einem Schallereignis einer Nutz-Schallquelle oder einem Schallereignis einer Stör-Schallquelle zugeordnet sind. Somit kann eine Quellentrennung erfindungsgemäßer Weise auf der Basis der Aktivitätsmuster erfolgen.

Es hat sich nämlich gezeigt, dass gerade unter Verwendung von Aktivitätsmustern an einem Gehörmodell Schallquellen in besonders effizienter Weise getrennt werden können, da die Schallereignisse von verschiedenen Schallquellen in dem Aktivitätsmuster als unterscheidbare Trajektorien repräsentiert sind. Während nämlich in einer zeitlichen Darstellung eines Audiosignals bzw. in einer Frequenzdarstellung des Audiosignals die zu verschiedenen Schallquellen gehörigen Signale sich überlappen, sind die verschiedenen Schallquellen bzw. die Informationsinhalte der verschiedenen Schallquellen in Aktivitätsmustern eines Gehörmodels durch getrennte, unterscheidbare Trajektorien repräsentiert. Aus diesem Grund ist eine Filterung auf der Basis der Aktivitätsmuster des Gehörmodels besonders effizient und im übrigen an eine Verarbeitung in einem menschlichen Gehirn angepasst, so dass in dem gefilterten Aktivitätsmuster Stör-Schallquellen mit besonders hoher Unterdrückung unterdrückbar bzw. entfernbar sind. Die Erkennung der Trajektorien, die zu Nutz-Schallereignissen bzw. Stör-Schallereignissen gehören, wird im übrigen, wie schon oben beschrieben, durch die Verwendung von zwei Aktivitätsmustern für Gehörmodelle von zwei Ohren in besonders zuverlässiger und effizienter Weise erreicht. Durch eine anschließende Rücktransformation des gefilterten Aktivitätsmusters in eine Zeitdarstellung, eine Frequenzdarstellung oder eine Subband-Darstellung des bereinigten Audiosignals, das durch das gefilterte Aktivitätsmuster beschrieben wird, wird eine weitere herkömmliche Nachbearbeitung des gefilterten Audiosignals (also des aus dem gefilterten Aktivitätsmuster durch Rücktransformation erhaltenem Audiosignals) ermöglicht.

Die vorliegende Erfindung schafft im Übrigen ein Verfahren zum Erzeugen eines bereinigten Audiosignals gemäß dem Patentanspruch 14. Das genannte Konzept zum Erzeugen eines bereinigten Aktivitätsmusters entspricht in seiner Funktionsweise der des erfindungsgemäßen Quellentrenners.

Die vorliegende Erfindung schafft ferner ein Computerprogramm gemäß dem Patentanspruch 15.

Im übrigen sei darauf hingewiesen, dass bevorzugte Ausführungsbeispiele der vorliegenden Erfindung durch die abhängigen Patentansprüche definiert sind.

Bevorzugte Ausführungsbeispiele der vorliegenden Erfindung werden nachfolgend bezugnehmend auf die beiliegenden Figuren näher erläutert. Es zeigen:

Fig. 1 ein Blockschaltbild einer erfindungsgemäßen Vor- richtung zum Erzeugen eines Aktivitätsmusters, gemäß einem Ausführungsbeispiel der vorliegenden Erfindung;

Fig. 2 ein Blockschaltbild einer erfindungsgemäßen Vor- richtung zum Erzeugen eines gefilterten Aktivi- tätsmusters, gemäß einem Ausführungsbeispiel der vorliegenden Erfindung;

Fig. 3a     eine schematische Darstellung von Trajektorien in einem Aktivitätsmuster, das eine Anzahl an Neu- rotransmitter-Vesikeln beschreibt;

Fig. 3b     eine schematische Darstellung von Trajektorien in einem Aktivitätsmuster an einer Mehrzahl von Ner- venfasern;

Fig. 3c     eine schematische Darstellung von digitalisierten Signalen, die die Aktivitätsmuster gemäß den Fig. 3a und 3b darstellen;

Fig. 3d     eine schematische Darstellung eines zweidimensio- nalen Musters, das die Aktivitätsmuster gemäß den Figuren 3a oder 3b beschreibt, und das auf dem digitalisierten Signalen gemäß der Fig. 3c ba- siert;

Fig. 4a     eine schematische Darstellung eines ersten Akti- vitätsmusters und eines zweiten Aktivitätsmus- ters, die eine Mehrzahl von Trajektorien umfas- sen;

Fig. 4b     eine schematische Darstellung eines gefilterten Aktivitätsmusters, das basierend auf den Aktivi- tätsmustern gemäß der Fig. 4a erzeugt ist;

Fig. 5a     ein Blockschaltbild einer erfindungsgemäßen Mul- ti-Koinzidenzeinrichtung gemäß einem Ausführung- beispiel der vorliegenden Erfindung;

Fig. 5b     ein detailliertes Schaltbild einer Spalte einer erfindungsgemäßen Multikoinzidenzeinrichtung ge- mäß der Fig. 5a.

Fig. 5c     ein Schaltbild einer Koinzidenzzelle zur Verwen- dung in einer Multikoinzidenzeinrichtung gemäß der Fig. 5a;

Fig. 6a     eine schematische Darstellung einer schrittweisen Verarbeitung von zwei Trajektorien gleicher Krüm- mung in einer erfindungsgemäßen Multikoinzidenz- einrichtung, ohne und mit Vorliegen einer zeitli- chen Verschiebung zwischen den Trajektorien;

Fig. 6b     eine schematische Darstellung einer schrittweisen Verarbeitung von zwei Trajektorien unterschiedli- cher Krümmung in einer erfindungsgemäßen Multiko- inzidenzeinrichtung;

Fig. 7     ein Blockschaltbild eines erfindungsgemäßen Iden- tifizierers zum Identifizieren von Trajektorien in zwei Aktivitätsmustern, gemäß einem Ausfüh- rungsbeispiel der vorliegenden Erfindung;

Fig. 8     ein Blockschaltbild einer Vorrichtung zur erfin- dungsgemäßen Durchführung einer Mustererkennung basierend auf einem Aktivitätsmuster;

Fig. 9     eine grafische Darstellung von Signalen in einer Vorrichtung zur erfindungsgemäßen Durchführung einer Mustererkennung;

Fig. 10     ein Schaltbild eines Hubel-Wiesen-Netzes zur er- findungsgemäßen Durchführung einer Mustererken- nung;

Fig. 11     eine schematische Darstellung eines Ablaufs bei einer Simulation eines menschlichen Gehörs sowie der bei der Simulation auftretenden Zwischen- und Endergebnisse;

Fig. 12     ein Flussdiagramm eines erfindungsgemäßen Verfah- rens zum Erzeugen eines gefilterten Aktivitäts- musters, gemäß einem Ausführungsbeispiel der vor- liegenden Erfindung;

Fig. 13     ein Blockschaltbild eines erfindungsgemäßen Quel- lentrenners, gemäß einem Ausführungsbeispiel der vor- liegenden Erfindung;

Fig. 14     ein Flussdiagramm eines erfindungsgemäßen Verfah- rens zum Erzeugen eines bereinigten Audiosignals, gemäß einem Ausführungsbeispiel der vorliedenden Erfindung;

Fig. 15     einen Auszug aus einem Blockschaltbild einer er- findungsgemäßen Vorrichtung, gemäß einem Ausfüh- rungsbeispiel der vorliegenden Erfindung; und

Fig. 16    eine schematische Darstellung einer menschlichen Cochlea sowie ausgewählter innerer Haarzellen.

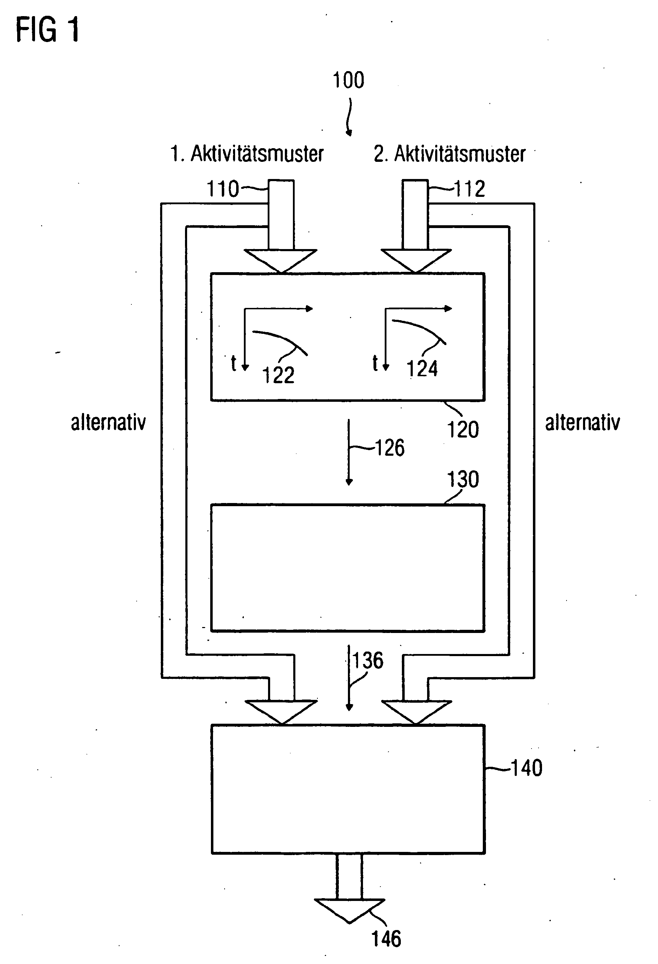

Fig. 1 zeigt ein Blockschaltbild einer erfindungsgemäßen Vorrichtung zum Erzeugen eines gefilterten Aktivitätsmusters basierend auf einem ersten Aktivitätsmuster an einem Gehörmodel eines ersten Ohrs, und eines zweiten Aktivitätsmusters an einem Gehörmodel eines zweiten Ohres. Die Vorrichtung gemäß der Fig. 1 ist in ihrer Gesamtheit mit 100 bezeichnet. Die Vorrichtung 100 ist ausgelegt, um ein erstes Aktivitätsmuster 110 von einem Gehörmodel eines ersten Ohres zu empfangen. Die Vorrichtung 100 ist ferner ausgelegt, um ein zweites Aktivitätsmuster 112 von einem Gehörmodel eines zweiten Ohres zu empfangen. Die Vorrichtung 100 umfasst einen Identifizierer 120, der ausgelegt ist, um das erste Aktivitätsmuster 110 und das zweite Aktivitätsmuster 112 zu empfangen. Der Identifizierer 120 ist ausgelegt, um eine erste Trajektorie in dem ersten Aktivitätsmuster und eine zweite Trajektorie in einem zweiten Aktivitätsmuster zu erkennen, die einem gleichen Schallereignis (z.B. einem Beginne eines Vokals, eines Konsonanten, eines Lauts, einem Knacken oder einem anderweitigen Schallereignis, das in einer Wanderwelle auf der Basilarmembran resultiert) zugeordnet sind. Unter einem gleichen Schallereignis wird dabei beispielsweise ein Schallereignis verstanden, das zu Trajektorien (zumindest näherungsweise bzw. innerhalb eines Toleranzbereichs) gleicher Krümmung und/oder gleicher Länge führt. Bevorzugt wird unter einem gleichen Schallereignis ein gleiches Ereignis in einem akustischen Signal, das von einer Schallquelle ausgeht, verstanden.

Der Identifizierer 120 ist ferner ausgelegt, um eine Information 126 zu liefern, die die erste Trajektorie 122 in dem ersten Aktivitätsmuster 110 und die zweite Trajektorie 124 in dem zweiten Aktivitätsmuster 112, die beide dem gleichen Schallereignis zugeordnet sind, beschreibt. Ein Bestimmer 130 empfängt die Information 126, und ist ausgelegt, um basierend auf der Information 126 über die erste Trajektorie 122 und die zweite Trajektorie 124 zu bestimmen, ob die zwei Trajektorien 122, 124, die dem gleichen Schallereignis zugeordnet sind, einem Schallereignis einer Nutz-Schallquelle oder einem Schallereignis einer Stör-Schallquelle zugeordnet sind. Der Bestimmer 130 liefert daher eine Information 136, die anzeigt, ob die Trajektorien 122, 124 einem Nutz-Schallereignis einer Nutz-Schallquelle oder einem Stör-Schallereignis einer Stör-Schallquelle zugeordnet sind.

Die Vorrichtung 100 umfasst ferner ein Filter 140 zum Filtern des ersten Aktivitätsmusters 110 oder des zweiten Aktivitätsmusters 112 basierend auf einem Ergebnis 136 des Bestimmens, ob eine Trajektorie einem Schallereignis der Nutz-Schallquelle oder einem Schallereignis der Stör-Schallquelle zugeordnet ist. Das Filter 140 empfängt somit entweder das erste Aktivitätsmuster 110, oder, alternativ, das zweite Aktivitätsmuster 112. Es ist allerdings ebenso möglich, dass das Filter 140 sowohl das erste Aktivitätsmuster 110 als auch das zweite Aktivitätsmuster 112 empfängt.

Das Filter 140 ist im übrigen ausgelegt, um ein gefiltertes Aktivitätsmuster 146 so zu erzeugen, dass in dem gefilterten Aktivitätsmuster Aktivitätsereignisse, die einem Schallereignis der Nutzschallquelle zugeordnet sind, dominieren, oder dass Aktivitätsereignisse, die nicht einem Schallereignis der Nutz-Schallquelle zugeordnet sind, in dem gefilterten Aktivitätsmuster nicht mehr vorhanden bzw. entfernt sind.

Basierend auf der obigen strukturellen Beschreibung wird im Folgenden die Funktionsweise der Vorrichtung 100 noch einmal näher erläutert. Der Identifizierer 120 empfängt ein erstes Aktivitätsmuster 110 sowie ein zweites Aktivitätsmuster 112. Bei den Aktivitätsmustern 110, 112 handelt es sich beispielsweise jeweils um eine Mehrzahl von parallelen Signalen, die eine Aktivität in oder an Hörzellen (bevorzugt inneren Hörzellen oder inneren Haarzellen) eines Gehörmodels beschreiben. In anderen Worten, das erste Aktivitätsmuster 110 umfasst eine Mehrzahl von parallelen Signalen bzw. Informationen, die eine Aktivität in oder an einer Mehrzahl von Hörzellen eines ersten Ohres (z.B. eines linken Ohres) beschreiben. Das zweite Aktivitätsmuster 112 umfasst ferner typischerweise eine Mehrzahl von parallelen Informationen oder Signalen, die eine Aktivität in oder an Hörzellen (bevorzugt inneren Hörzellen der inneren Haarzellen) eines zweiten Ohres beschreiben.

Alternativ dazu können die Aktivitätsmuster auch eine Aktivität auf Nervenfasern von Hörnerven beschreiben. Beispielsweise kann das erste Aktivitätsmuster 110 eine Aktivität (bzw. einen zeitlichen Verlauf einer Aktivität) auf einer Mehrzahl von Nervenfasern, die zu einem ersten Ohr (z.B. linken Ohr) gehören, beschreiben, während hingegen das zweite Aktivitätsmuster 112 eine Aktivität auf einer Mehrzahl von Nervenfasern, die zu einem zweiten Ohr (z.B. einem rechten Ohr) gehören, beschreibt.

Es wird im übrigen bevorzugt, dass das erste Aktivitätsmuster 110 und das zweite Aktivitätsmuster 112 durch die Verwendung von Gehörmodellen für das erste Ohr und das zweite Ohr gewonnen werden. In anderen Worten, das Gehörmodel für das erste Ohr empfängt ein erstes Audiosignal beispielsweise von einem ersten Mikrofon, das an einer linken Seite eines (beispielsweise menschlichen) Kopfes angeordnet ist, und liefert das erste Aktivitätsmüster.

Ferner wird es bevorzugt, dass das zweite Aktivitätsmuster durch ein Gehörmodel eines zweiten Ohrs (z.B. eines rechten Ohrs) basierend auf einem zweiten Audiosignal bestimmt wird. Das zweite Audiosignal kann beispielsweise von einem zweiten Mikrofon geliefert werden, das beispielsweise an der rechten Seite eines (beispielsweise menschlichen) Kopfes angeordnet ist.

Somit beschreiben das erste Aktivitätsmuster 110 und das zweite Aktivitätsmuster 112 typischerweise zwei Audiosignale von zwei verschiedenen Audiosignalquellen (z.B. von zwei verschieden angeordneten Mikrofonen). Die zwei Aktivitätsmuster können aber auch Audiosignale von zumindest zwei Kanälen eines mehrkanaligen Audiosignals beschreiben.

Es sei im übrigen darauf hingewiesen, dass das erste Aktivitätsmuster 110 und das zweite Aktivitätsmuster 112 typischerweise durch eine Mehrzahl von Zeitsignalen gebildet werden, die (beispielsweise bei einer zweidimensionalen Darstellung derselben) ein zweidimensionales Muster beschreiben. Beispielsweise kann das erste Aktivitätsmuster N Informationen bzw. parallele Zeitsignale enthalten, die eine Aktivität in oder an N verschiedenen Hörzellen oder an N verschiedenen Hörnerven als Funktion der Zeit beschreiben. In ähnlicher Weise kann das zweite Aktivitätsmuster 112 eine Mehrzahl von Informationen bzw. parallelen (Zeit-) Signalen umfassen, die einen zeitlichen Verlauf einer Aktivität in oder an einer Mehrzahl von Hörzellen oder an einer Mehrzahl von Hörnerven beschreiben.

Das Auftreten einer Aktivität (also beispielsweise eines aktiven Zustands, der beispielsweise durch eine charakteristische Veränderung einer Konzentration eines bestimmten Stoffes oder eines elektrischen Potentials angezeigt wird) wird im übrigen im Folgenden als ein Aktivitätsereignis bezeichnet, und ist aus den Aktivitätsmustern 110, 112 ersichtlich. Bei einem Aktivitätsereignis kann es sich beispielsweise um ein Neurotransmitter-Vesikel-Auftreten in einer Hörzelle (beispielsweise an einem synaptischen Spalt der Hörzelle) oder um ein Auftreten eines (aktiven) Aktionspotentials auf einer Nervenfaser handeln.

Der Identifizierer 120 ist ausgelegt, um das erste Aktivitätsmuster 110 und das zweite Aktivitätsmuster 112 zu empfangen, und um eine erste Trajektorie 122 in dem ersten Aktivitätsmuster 110 in eine zweite Trajektorie 124 in dem zweiten Aktivitätsmuster 112 zu identifizieren, die einem gleichen Schallereignis zugeordnet sind. Es sei diesbezüglich darauf hingewiesen, dass Trajektorien, die einem gleichen Schallereignis zugeordnet sind, typischerweise eine zumindest ähnliche Form und/oder eine ähnliche Länge aufweisen, und aufgrund der genannten Merkmale als zusammengehörig bzw. als zu dem gleichen Schallereignis gehörig identifiziert werden können. Ferner ist bekannt, dass Trajektorien, die einem gleichen Schallereignis zugeordnet sind, bzw. die zu einem gleichen Schallereignis gehören, typischerweise innerhalb eines vorbestimmten maximalen Zeitintervalls auftreten.

Daher ist der Identifizierer 120 bevorzugt ausgelegt, um zueinander ähnliche Trajektorien, also beispielsweise Trajektorien, deren Krümmung um weniger als eine vorgegebene maximal zulässige Abweichung voneinander abweicht, und die innerhalb eines vorgegebenen maximalen Zeitintervalls auftreten, als zu einem gleichen Schallereignis gehörige Trajektorien zu identifizieren. Alternativ oder zusätzlich kann der Identifizierer 120 ferner ausgelegt sein um eine Länge von Trajektorien bei der Bestimmung, ob zwei Trajektorien einem gleichen Schallereignis zugeordnet sind, zu berücksichtigen. In anderen Worten, der Identifizierer 120 kann beispielsweise ausgelegt sein, um anzuzeigen, dass zwei Trajektorien zu einem gleichen Schallereignis gehören, wenn diese innerhalb eines vorgegebenen maximalen Zeitintervalls auftreten, und wenn diese ferner, bis auf eine vorgegebene maximal zulässige Abweichung eine gleiche Länge aufweisen.

Basierend auf einer Erkennung, dass zwei Trajektorien 122, 124 einem gleichen Schallereignis zugeordnet sind, liefert der Identifizierer 120 die Information 126, die Information über die zwei identifizierten, zusammengehörigen Trajektorien 122, 124 umfasst. Die Information 126 kann beispielsweise eine Information umfassen, die es ermöglicht, zumindest eine der identifizierten, zusammengehörigen Trajektorien 122, 124 (bzw. zugehörige Aktivitätsereignisse) in dem ersten Aktivitätsmuster 110 oder in dem zweiten Aktivitätsmuster 112 zu finden.

Beispielsweise kann die Information 126 eine Information über einen Zeitpunkt, zu dem die zusammengehörigen Trajektorien 124 auftreten, umfassen. Ferner kann die Information 126 alternativ über zusätzlich eine Information über eine Länge der beiden Trajektorien 124, 126 umfassen. Alternativ oder zusätzlich umfasst die Information 126 ferner eine Aussage darüber, wie stark sich die beiden Trajektorien 122, 124 voneinander unterscheiden. Die genannte Information 126 kann beispielsweise eine Aussage darüber geben, wie stark sich die Krümmungen der beiden zusammengehörigen, dem gleichen Schallereignis zugeordneten Trajektorien 124, 126 unterscheiden. Ferner kann die Information 126 alternativ oder zusätzlich eine Information darüber tragen, wie groß eine zeitliche Verschiebung zwischen den beiden Trajektorien 124, 126 ist.

Der Bestimmer 130 ist bevorzugt ausgelegt, um die Information 126 von dem Identifizierer 120 zu empfangen, und um basierend darauf zu bestimmen, ob die zwei identifizierten, zusammengehörigen Trajektorien 122, 124 einem Nutz-Schallereignis bzw. einer Nutzschallquelle oder einem Stör-Schallereignis bzw. einer Stör-Schallquelle zugeordnet sind. Bei einem bevorzugten Ausführungsbeispiel ist der Bestimmer 130 ausgelegt, um die Information, ob die zwei zusammengehörigen identifizierten Trajektorien einer Nutz-Schallquelle oder einer Stör-Schallquelle zugeordnet sind, durch einen Vergleich von Eigenschaften bzw. Merkmalen der beiden zusammengehörigen identifizierten Trajektorien 122, 124 abzuleiten. So kann der Identifizierer 130 beispielsweise ausgelegt sein, um die Längen zweier als zusammengehörig identifizierter Trajektorien 122, 124 zu vergleichen. Weichen beispielsweise die Längen voneinander ab, so kann dies als ein Anzeichen dafür gewertet werden, dass die beiden identifizierten Trajektorien 122, 124 einer Stör-Schallquelle zugeordnet sind, wenn beispielsweise davon ausgegangen wird, dass die Längen von Trajektorien, die einer Nutz-Schallquelle zugeordnet sind, innerhalb eines vorgegebenen Toleranzbereichs zueinander gleich sind.

Ferner kann der Bestimmer 130 ausgelegt sein, um die Krümmungen der beiden als zusammengehörig identifizierten Trajektorien 122, 124 zu vergleichen, um basierend darauf eine Aussage zu liefern, ob die beiden Trajektorien 122, 124 einer Nutz-Schallquelle oder einer Stör-Schallquelle zugeordnet sind.

Bei einem weiteren bevorzugten Ausführungsbeispiel ist der Bestimmer ausgelegt um eine zeitliche Verschiebung zwischen den beiden als zusammengehörig identifizierten Trajektorien 122, 124 zu ermitteln, und um die Entscheidung, ob die als zusammengehörig identifizierten Trajektorien einem Schallergebnis einer Nutz-Schallquelle oder einer Stör-Schallquelle zugeordnet sind, basieren auf einer Größe der zeitlichen Verschiebung zwischen den als zusammengehörig identifizierten Trajektorien 122, 124 zu bestimmen.

Das Filter 140 ist bevorzugt ausgelegt, um eine Information darüber zu erhalten, welche der durch den Identifizierer 120 identifizierten Trajektorien einem Schallereignis einer Nutz-Schallquelle zugeordnet sind und/oder welche der von dem Bestimmer 130 identifizierten Trajektorien einem Schallereignis einer Stör-Schallquelle zugeordnet sind. Bei einem Ausführungsbeispiel ist das Filter 140 ausgelegt, um das erste Aktivitätsmuster oder das zweite Aktivitätsmuster zu empfangen, und um ferner eine Information darüber zu empfangen, welche der in dem ersten Aktivitätsmuster oder in dem zweiten Aktivitätsmuster enthaltenen Trajektorien einer Nutz-Schallquelle zugeordnet sind. Das Filter 140 ist in diesem Fall ausgelegt, um die durch die Information 136 bezeichneten Trajektorien (bzw. deren Aktivitätsereignisse), die einem Schallereignis einer Nutz-Schallquelle zugeordnet sind, in das gefilterte Aktivitätsmuster 146 zu übernehmen, und um beispielsweise die übrigen Trajektorien (bzw. deren Aktivitätsereignisse) in dem ersten Aktivitätsmuster 110 und dem zweiten Aktivitätsmuster 112 bei der Erzeugung des gefilterten Aktivitätsmusters 146 zu unterdrücken oder zumindest gegenüber den zu Nutz-Schallereignissen der Nutz-Schallquelle gehörigen Trajektorien und/oder Aktivitätsereignissen zu bedämpfen.

Bei einem weiteren Ausführungsbeispiel ist das Filter 140 ausgelegt, um als die Information 136 eine Information darüber zu erhalten, welche der durch den Identifizierer 120 identifizierten Trajektorien 122, 124 einem Schallereignis einer Stör-Schallquelle zugeordnet sind. In diesem Fall ist das Filter 140 bevorzugt ausgelegt, um aus dem ersten Aktivitätsmuster 110 oder aus dem zweiten Aktivitätsmuster 112 diejenigen Trajektorien (bzw. die zu den entsprechenden Trajektorien gehörigen Aktivitätsereignisse) zu entfernen oder zu bedämpfen, die gemäß der Information 136 zu einem Schallereignis der Stör-Schallquelle gehören.

In anderen Worten, das Filter 140 kann beispielsweise ausgelegt sein, um basierend auf dem ersten Aktivitätsmuster 110 oder auf dem zweiten Aktivitätsmuster 112 nur diejenigen Trajektorien bzw. Schallereignisse weiterzuleiten, die gemäß der Information 136 einem Schallereignis der Nutz-Schallquelle zugeordnet sind. Alternativ dazu kann das Filter 140 ausgelegt sein, um aus dem ersten Aktivitätsmuster 110 oder aus dem zweiten Aktivitätsmuster 112 diejenigen Trajektorien bzw. Aktivitätsereignisse zu entfernen, die gemäß der Information 136 zu einem Schallereignis einer Stör-Schallquelle gehören.

Somit entsteht an dem Ausgang des Filters 146 insgesamt ein gefiltertes Aktivitätsmuster, in dem Trajektorien bzw. Aktivitätsereignisse, die zu Schallereignissen der Stör-Schallquelle gehören, unterdrückt oder gedämpft sind, und in dem Aktivitätsereignisse oder Trajektorien, die zu Schallereignissen der Nutz-Schallquelle gehören, ungedämpft oder verstärkt enthalten sind.

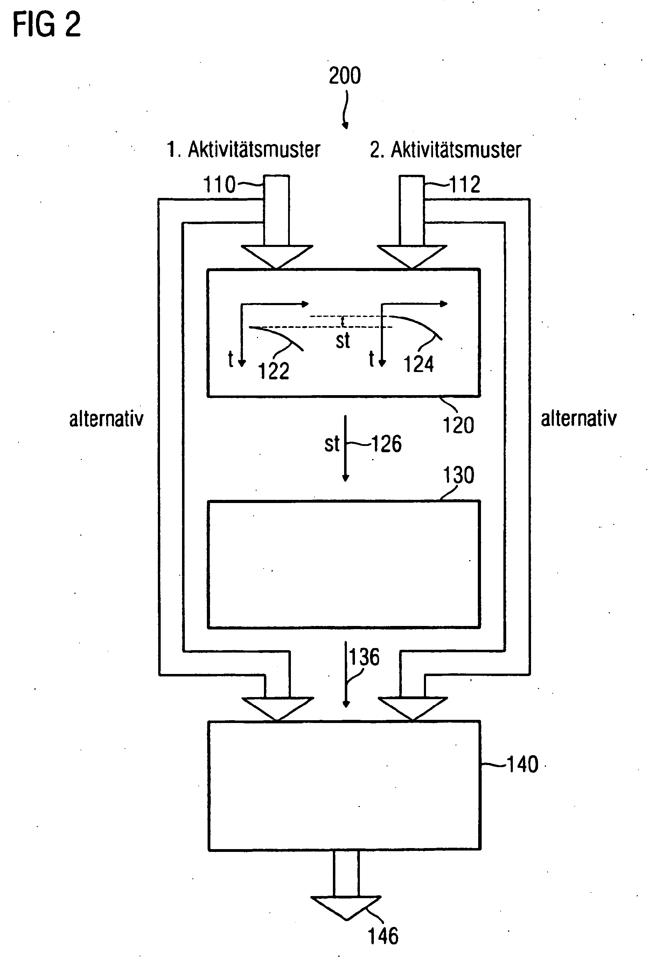

Fig. 2 zeigt ein Blockschaltbild einer erfindungsgemäßen Vorrichtung zum Erzeugen eines gefilterten Aktivitätsmusters basierend auf einem ersten Aktivitätsmuster an einem Gehörmodell eines ersten Ohres und einem zweiten Aktivitätsmuster an einem Gehörmodell eines zweiten Ohrs. Die Vorrichtung gemäß der Fig. 2 ist in ihrer Gesamtheit mit 200 bezeichnet. Da die Vorrichtung 200 gemäß der Fig. 2 der Vorrichtung 100 gemäß Fig. 1 sehr ähnlich ist, sind bei den Vorrichtungen 100, 200 gemäß den Fig. 1 und 2 gleiche Merkmale und Signale mit gleichen Bezugszeichen bezeichnet und werden hier nicht noch einmal erläutert.

Der Identifizierer 120 bei der Vorrichtung 200 ist ausgewählt, um eine erste Trajektorie 122 in dem ersten Aktivitätsmuster 110 und eine zweite Trajektorie 124 in dem zweiten Aktivitätsmuster 112 zu identifizieren, die dem gleichen Schallereignis zugeordnet sind. Diese Identifizierung kann beispielsweise, wie schon oben beschrieben, anhand eines Vergleichs der Krümmungen der beiden Trajektorien 122, 124 und/oder der Längen der beiden Trajektorien 122, 124 erfolgen. Im übrigen kann zum Identifizieren von zwei Trajektorien 122, 124, die einem gleichen Schallereignis zugeordnet sind, auch eine der im Folgenden beschriebenen Mustererkennungseinrichtungen verwendet werden.

Zusätzlich zu dem im Hinblick auf die Vorrichtung 100 beschriebenen Identifizierer ist der Identifizierer 120 der Vorrichtung 200 ausgelegt, um eine zeitliche Verschiebung zwischen den zwei zusammengehörigen, (d.h. dem gleichen Schallereignis zugeordneten) Trajektorien 122, 124 zu bestimmen. Der Identifizierer 120 liefert daher die Information über die zeitliche Verschiebung zwischen den beiden als zusammengehörig identifizierten Trajektorien 122, 124 als die Information 126 bzw. als Teil der Information 126 an den Bestimmer 130. Der Bestimmer 130 ist ausgelegt, um anhand der zeitlichen Verschiebung (bzw. zumindest basierend auf der zeitlichen Verschiebung) $\Delta t$ festzustellen, ob die zwei Trajektorien 122, 124 einem Schallereignis einer Nutz-Schallquelle zugeordnet sind.

Der Bestimmer 130 kann zu dem Zweck beispielsweise prüfen, ob die zeitliche Verschiebung $\Delta t$ zwischen den beiden Trajektorien 122, 124 innerhalb eines vorbestimmten zulässigen Bereichs liegt. Liegt die zeitliche Verschiebung $\Delta t$ zwischen den beiden Trajektorien 122, 124 innerhalb des vorgegebenen zulässigen Bereichs, so kann der Bestimmer 130 beispielsweise feststellen, dass die Trajektorien zu einem Schallereignis der Nutz-Schallquelle gehören. Liegt die zeitliche Verschiebung $\Delta t$ außerhalb des vorbestimmten zulässigen Bereichs, so kann der Bestimmer 130 feststellen, dass die Trajektorien 122, 124 einem Schallereignis der Stör-Schallquelle zugeordnet sind.

Der zulässige Bereich, der dadurch definiert ist, dass bei Vorliegen einer Zeitverschiebung Δt innerhalb des zulässigen Bereichs angenommen wird, dass die zwei Trajektorien 122, 124 zu dem Schallereignis einer Nutz-Schallquelle gehören, kann beispielsweise fest vorgegeben sein. Bei einem bevorzugten Ausführungsbeispiel wird davon ausgegangen, dass der zulässige Bereich für die Zeitverschiebung der Trajektorien 122, 124 Werte für die zeitliche Verschiebung umfasst, deren Betrag kleiner als eine vorgegebene obere Grenze ist. In anderen Worten, die zwei Trajektorien werden dann als ein Schallereignis einer Nutz-Schallquelle identifiziert, wenn eine zeitliche Verschiebung Δt zwischen den Trajektorien kleiner als ein vorgegebener maximaler Wert ist.

Wird davon ausgegangen, dass das erste Aktivitätsmuster beispielsweise von einem in der Nähe eines ersten Ohres angeordneten Mikrofon stammt, und dass das zweite Aktivitätsmuster beispielsweise von einem in der Nähe eines zweiten menschlichen Ohres angeordneten Mikrofon stammt, so weisen zeitgleich eintreffenden Trajektorien 122, 124 in den beiden Aktivitätsmustern 110, 112 beispielsweise darauf hin, dass die Schallquelle sich geradlinig vor dem Kopf befindet. Eine Definition, die einen maximalen Betrag der Zeitdifferenz Δt festlegt, um zwei Trajektorien 122, 124 als zu dem Schallereignis der Nutz-Schallquelle gehörig zu identifizieren, entspricht daher der Festlegung eines Winkelbereichs vor dem Kopf einer Person, in der Nähe von deren Ohren die Audiosignale aufgenommen werden, auf denen das erste Aktivitätsmuster 110 und das zweite Aktivitätsmuster 112 basieren.

Bei einem alternativen Ausführungsbeispiel wird es allerdings bevorzugt, den zulässigen Bereich basierend auf Eigenschaften der Signale, die den Aktivitätsmustern 110, 112 zugrunde liegen, zu bestimmen. Zu diesem Zweck kann beispielsweise für eine Mehrzahl von Bereichen von Zeitverschiebungen ermittelt werden, wann für jeden der Bereiche von Zeitverschiebungen zugehörige Trajektorien auftreten. In anderen Worten, es werden beispielsweise Paare von all denjenigen zusammengehörigen (eine gleiche Krümmung aufweisenden oder einem gleichen Schallereignis zugeordneten) Trajektorien ermittelt, deren Zeitverschiebung zueinander in einem Bereich zwischen $\Delta t_1$ und $\Delta t_2$ liegt. Basierend auf den Trajektorien mit Zeitverschiebungen in dem Bereich zwischen $\Delta t_1$ und $\Delta t_2$ wird dann ein Auftrittsmuster bestimmt, das angibt, zu welchem Zeitpunkt Trajektorien mit einer Zeitverschiebung in dem Bereich zwischen $\Delta t_1$ und $\Delta t_2$ auftreten. Aus dem Auftrittsmuster oder einer von dem Auftrittsmuster abgeleiteten Statistik wird dann beispielsweise ermittelt, ob die Trajektorien mit Zeitverschiebungen in dem Bereich zwischen $\Delta t_1$ und $\Delta t_2$ zu einem Sprachsignal gehören. In anderen Worten, es wird aus dem Auftrittsmuster von Trajektorien mit einer Zeitverschiebung in dem Bereich bzw. Intervall zwischen $\Delta t_1$ und $\Delta t_2$ eine Statistik ermittelt, die beispielsweise einen mittleren Zeitabstand zwischen Zeitpunkten, zu denen Trajektorien auftreten, eine Standardabweichung zwischen Zeitpunkten, zu denen Trajektorien auftreten, einem maximalen zeitlichen Abstand zwischen dem Auftreten von zwei Trajektorien, einem minimalen zeitlichen Abstand zwischen dem Auftreten von zwei Trajektorien oder eine andere statistische Größe (z.B. eine zugehörige Standardabweichung zu einem der oben genannten statistischen Werte) umfasst. Basierend auf einer der genannten statistischen Größen oder einer Kombination der genannten statistischen Größen kann dann ermittelt werden, ob die Trajektorien, deren Zeitverschiebung in dem Bereich zwischen $\Delta t_1$ und $\Delta t_2$ liegt, ein Schallereignis eines Nutzsignals eines Störsignals beschreiben.

Alternativ zu der Berechnung der Statistik kann im Übrigen auch eine Mustererkennung auf das Auftrittsmuster der Trajektorien angewandt werden. In anderen Worten, das zeitliche Muster, mit dem Trajektorien auftreten, deren zeitliche Verschiebung in dem Bereich zwischen $\Delta t_1$ und $\Delta t_2$ liegt, kann mit zumindest einem Vergleichsmuster verglichen werden, um festzustellen, ob die Trajektorien mit einer Zeitverschiebung in dem Bereich zwischen $\Delta t_1$ und $\Delta t_2$ ein Nutzsignal oder ein Störsignal beschreiben. Die Vergleichsmuster können dabei beispielsweise charakteristische Muster umfassen, die ein Auftreten von Trajektorien in einem typischen Nutzsignal (z.B. einem Sprachsignal) und/oder in einem typischen Störsignal charakterisieren.

Ist somit aufgrund der oben beschriebenen Vorgehensweise bekannt, dass zusammengehörige Trajektorien, die eine Zeitverschiebung in dem Bereich zwischen $\Delta t_1$ und $\Delta t_2$ aufweisen, Schallereignisse einer Nutzschallquelle oder einer Stör-Schallquelle beschreiben, so kann basierend darauf das Intervall zwischen den Verzögerungszeiten $\Delta t_1$ und $\Delta t_2$ dem zulässigen Bereich hinzugefügt oder von dem zulässigen Bereich entfernt werden.

In anderen Worten, der Bestimmer 130 umfasst in diesem Fall eine Bereichs-Einstelleinrichtung, die ausgelegt ist, um für eine Mehrzahl von Intervallen von Verzögerungszeiten (zwischen zwei zusammengehörigen Trajektorien) zu bestimmen, ob Trajektorien mit Verzögerungszeiten innerhalb der jeweiligen Intervalle einer Nutzschallquelle oder einer Störschallquelle zugeordnet sind, und um den zulässigen Bereich, als eine Kombination derjenigen Zeitintervalle (von Zeitverschiebungen zwischen zusammengehörigen Trajektorien), die Nutzschallquellen zugeordnet sind, zu erhalten.

Somit ist die Bereichs-Auswahleinrichtung ausgelegt, um den zulässigen Bereich (also den Bereich von Zeitverschiebungen Δt, so dass zusammengehörige Trajektorien 122, 124 mit einer Zeitverschiebung aus dem zulässigen Bereich als zu Schallereignissen einer Nutzschallquelle gehörige Trajektorien identifiziert werden) so auszuwählen, dass der zulässige Bereich ein oder mehrere (zusammenhängende oder nichtzusammenhängende) Zeitintervalle umfasst, die Zeitverschiebungen beschreiben, die zu Trajektorien gehören, die auf Schallereignissen von einer oder mehreren Nutzschallquellen basieren.

Die Auswahl des zulässigen Bereichs von Zeitverschiebungen Δt kann im Übrigen auch aufgrund anderer Eigenschaften der akustischen Signale, auf denen das erste Aktivitätsmuster 110 und/oder das zweite Aktivitätsmuster 112 basieren, bestimmt werden. Beispielsweise kann bestimmt werden, welche Trajektorien in den ersten Aktivitätsmustern zu einem lautesten Schallsignal gehören. Der zulässige Bereich kann daraufhin so eingestellt werden, dass die Trajektorien, die zu einem lautesten Schallsignal gehören, eine Zeitverschiebung $\Delta t$ aufweisen, die innerhalb des zulässigen Bereichs liegt.

In anderen Worten, für eine Einstellung des zulässigen Bereiches ist es beispielsweise vorteilhaft, aufgrund von bestimmbaren Eigenschaften eines Audiosignalanteils (z.B. Korrelationseigenschaften, Lautstärke, zeitlichem Verlauf der Intensität, Bandbreite, Auftretenszeitpunkte von Trajektorien) zu bestimmen, ob der entsprechende Audiosignalanteil als ein Nutzsignal von einer Nutzsignalquelle oder als ein Störsignal von einer Störsignalquelle anzusehen ist. Aufgrund der genannten Klassifizierung eines Signalanteils als Nutzsignals von einer Nutzsignalquelle oder als Störsignal von einer Störsignalquelle und einer Bestimmung einer zeitlichen Verschiebung $\Delta t$ zwischen zusammengehörigen Trajektorien 122, 124, die zu dem betreffenden Signalanteil gehören, wird dann ein Bereich von zeitlichen Verschiebungen zwischen $\Delta t_1$ und $\Delta t_2$ ermittelt, so dass Trajektorien, die zu einem Nutzsignal von einer Nutzsignalquelle gehören, eine zeitliche Verschiebung $\Delta t$ in dem Bereich zwischen $\Delta t_1$ und $\Delta t_2$ aufweisen, und dass Trajektorien, die zu einem Störsignal von einer Störsignalquelle gehören, eine zeitliche Verschiebung außerhalb des genannten Bereichs aufweisen.

Im Folgenden wird durch den Bestimmer 130 festgestellt, dass zusammengehörige Trajektorien, die eine zeitliche Verschiebung $\Delta t$ in dem Bereich zwischen $\Delta t_1$ und $\Delta t_2$ aufweisen, als zu dem Nutzsignal gehörige Trajektorien zu betrachten und weiterzuverarbeiten sind.

Somit ist insgesamt eine dynamische Anpassung des zulässigen Bereichs von zeitlichen Verzögerungen $\Delta t$ möglich, wobei Trajektorien mit einer zeitlichen Verzögerung innerhalb des zulässigen Bereichs als zu einem Nutzsignal von einer Nutzsignalquelle gehörige Trajektorie durch den Bestimmer 130 identifiziert werden.

Im Anschluss an die Bestimmung eines zulässigen Bereichs $\Delta t$ muss im Übrigen lediglich eine zeitliche Verschiebung zwischen zusammengehörigen Trajektorien 122, 124 ausgewertet werden, um zu entscheiden, ob die Trajektorien als einem Schallereignis der Nutzsignalquelle zugeordnet oder als einem Schallereignis der Störsignalquelle zugeordnet identifiziert werden. Somit ist nach der Einstellung des zulässigen Bereichs keine aufwendige Verarbeitung der Aktivitätsmuster 110, 112 (beispielsweise in Form einer kontinuierlichen Bestimmung von Korrelationseigenschaften) mehr erforderlich, um Trajektorien von Aktivitätsereignissen, die auf dem Schallereignis einer Nutzsignalquelle basieren, von Trajektorien von Aktivitätsereignissen, die auf einem Schallereignis einer Störsignalquelle basieren, zu trennen.

Die Trennung von Nutzsignalen und Störsignalen bleibt im Übrigen so lange erhalten, wie sich eine zeitliche Verschiebung zwischen zusammengehörigen Trajektorien, die einem Schallereignis von einer Nutzschallquelle zugeordnet sind, nicht wesentlich verändern. Erst wenn sich beispielsweise die Lage der Nutzschallquelle im Bezug auf die Mikrofone, die zur Aufnahme der Audiosignale dienen, auf denen das erste Aktivitätsmuster 110 und das zweite Aktivitätsmuster 112 basieren, verändert, ist eine Neueinstellung des zulässigen Bereichs erforderlich.

Im Folgenden wird beschrieben, wie das Vorhandensein einer ersten Trajektorie 122 in dem ersten Aktivitätsmuster 110 und das Vorhandensein einer zweiten Trajektorie 124 in dem zweiten Aktivitätsmuster 112, die einem gleichen Schallereignis zugeordnet sind, erkannt werden kann, und wie ferner die zeitliche Verschiebung $\Delta t$ zwischen den zweit Trajektorien 122, 124 in technisch vorteilhafter Weise ermittelt werden kann.

Diesbezüglich wird zunächst noch einmal kurz erläutert, was unter einer Trajektorie verstanden wird, und in welcher Form das erste Aktivitätsmuster 110, das zweite Aktivitätsmuster 112 und das gefilterte Aktivitätsmuster 146 dargestellt werden können.

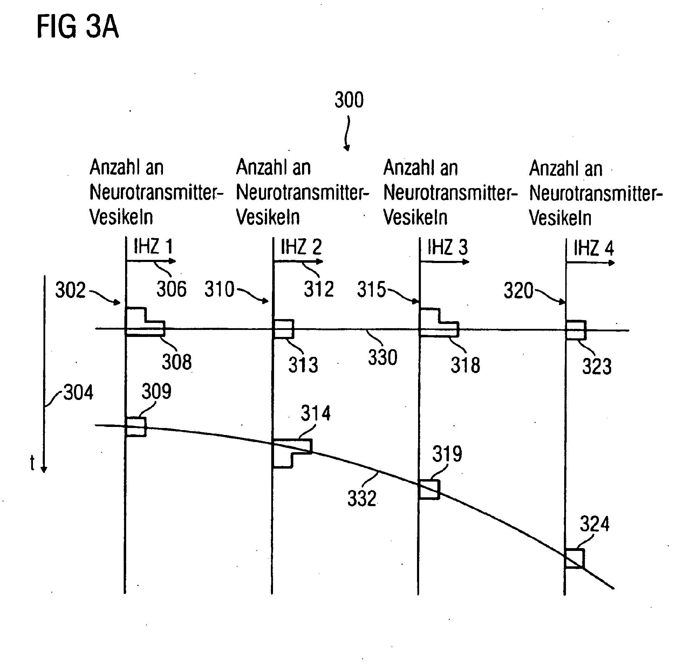

Zu diesem Zweck zeigt die Fig. 3a eine graphische Darstellung von zwei Trajektorien in einer Darstellung eines Neurotransmitter-Vesikel-Auftretens in bzw. an einer inneren Hörzelle.

Die graphische Darstellung der Fig. 3a ist in ihrer Gesamtheit mit 300 bezeichnet. Eine erste Darstellung 302 zeigt einen zeitlichen Verlauf einer Anzahl an Neurotransmitter-Vesikeln, die in oder an einer ersten inneren Hörzelle IHZ1 auftreten. Eine Zeitachse ist dabei mit 304 bezeichnet, während eine Anzahl-Achse 306 eine Anzahl an Neurotransmitter-Vesikeln beschreibt, die innerhalb einer Zeiteinheit freigesetzt werden, oder die zu einem bestimmten Zeitpunkt in oder an der betrachteten inneren Hörzelle (beispielsweise in freigesetzter Form) existieren. Die erste graphische Darstellung 302 zeigt ein erstes Neurotransmitter-Vesikel-Auftreten 308 sowie ein zweites Neurotransmitter-Vesikel-Auftreten 309. Das erste Neurotransmitter-Vesikel-Auftreten 308 kann beispielsweise als ein erstes Aktivitätsereignis betrachtet werden, und das zweite Neurotransmitter-Vesikel-Auftreten 309 kann beispielsweise ein zweites Aktivitätsereignis betrachtet werden. Eine zweite graphische Darstellung 310 zeigt ein Neurotransmitter-Vesikel-Auftreten an einer zweiten inneren Hörzelle IHZ2. Eine Anzahl-Achse 312 beschreibt eine Anzahl an pro Zeiteinheit freigesetzten oder zu einem bestimmten Zeitpunkt insgesamt (z.B. in freigesetzter Form) vorhandenen Neurotransmitter-Vesikeln in oder an der inneren Hörzelle IHZ2. Ein erstes Neurotransmitter-Vesikel-Auftreten an der inneren Hörzelle IHZ2 ist mit 313 bezeichnet, und ein zweites Neurotransmitter-Vesikel-Auftreten an der inneren Hörzelle IHZ2 ist mit 314 bezeichnet. Eine dritte graphische Darstellung 315 zeigt in ähnlicher Weise ein Neurotransmitter-Vesikel-Auftreten an einer dritten inneren Hörzelle IHZ3, wobei ein erstes Neurotransmitter-Vesikel-Auftreten an der inneren Hörzelle IHZ3 mit 318 bezeichnet ist, wobei ein zweites Neurotransmitter-Vesikel-Auftreten mit 319 bezeichnet ist. Eine vierte graphische Darstellung, 320 zeigt schließlich ein erstes Neurotransmitter-Vesikel-Auftreten 323 an einer vierten inneren Hörzelle IHZ4 sowie ein zweites Neurotransmitter-Vesikel-Auftreten 324 an der vierten inneren Hörzelle IHZ4.

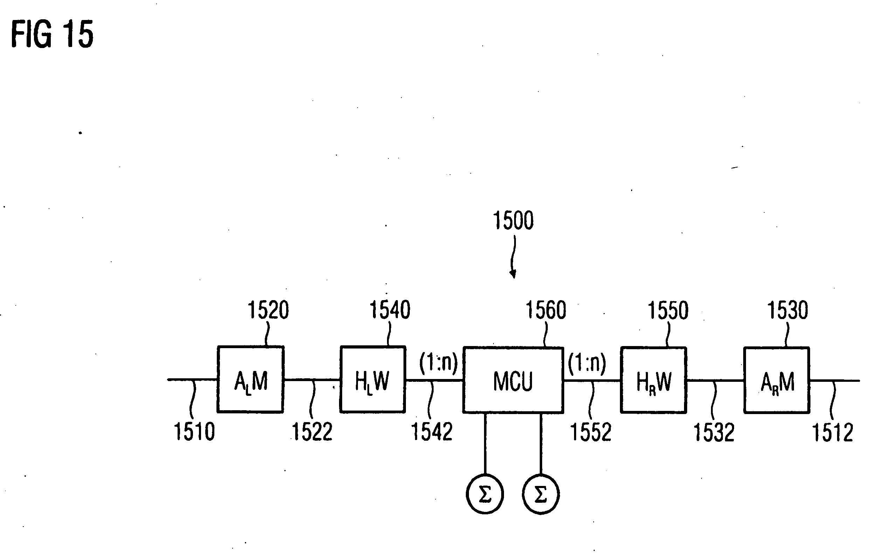

Es sei hierbei darauf hingewiesen, dass die inneren Hörzellen IHZ1, IHZ2, IHZ3, IHZ4 beispielsweise so angeordnet sind, wie dies im Folgenden noch anhand der Fig. 15 beschrieben wird.

Anhand der graphischen Darstellung 300 der Fig. 3a ist im Übrigen ersichtlich, dass das erste Aktivitätsereignis 308 an der ersten inneren Hörzelle IHZ1, das erste Aktivitätsereignis 313 an der zweiten inneren Hörzelle IHZ2, das erste Aktivitätsereignis 318 an der dritten inneren Hörzelle IHZ3 und das erste Aktivitätsereignis 323 an der vierten inneren Hörzelle IHZ4 in der zweidimensionalen Darstellung der Aktivitätsereignisse über der Zeit durch eine näherungsweise gerade Linie 330 miteinander verbunden werden. Bei der Linie 330 handelt es sich daher um eine erste Trajektorie. Das zweite Aktivitätsereignis 309 an oder in der ersten inneren Hörzelle IHZ1, das zweite Aktivitätsereignis 314 an oder in der zweiten inneren Hörzelle IHZ2, das zweite Aktivitätsereignis 319 an oder in der dritten inneren Hörzelle IHZ3 und das zweite Aktivitätsereignis 324 an oder in der vierten inneren Hörzelle IHZ4 sind im Übrigen in der graphischen Darstellung 300 der Fig. 3a durch eine zweite Linie 332 verbunden, die eine zweite Trajektorie bildet.

Eine Trajektorie ist dabei im Allgemeinen definiert als ein linienförmiger Verlauf, der zwei, bevorzugt aber mehr als zwei zusammengehörige Aktivitätsereignisse, die auf dem gleichen Schallereignis basieren, verbindet. Eine Trajektorie ist typischerweise entweder geradlinig oder in einer einzigen Richtung gekrümmt, wechselt also bevorzugt die Krümmungsrichtung nicht. Ferner ist eine Trajektorie typischerweise eine glatte Kurve, die also keine Knicke bzw. nicht-differenzierbare Schwellen aufweist. Im Übrigen wird im Rahmen der vorliegenden Beschreibung jeweils davon ausgegangen, dass einer Trajektorie eine Mehrzahl von Aktivitätsereignissen zugeordnet sind, so dass der Begriff Trajektorie auch die der Trajektorie zugeordneten Aktivitätsereignisse umfasst. Beispielsweise umfasst die Trajektorie 330 die Aktivitätsereignisse 308, 313, 318, 323. Die Trajektorie 332 umfasst im Übrigen die Aktivitätsereignisse 309, 314, 319, 324.

Ferner sei darauf hingewiesen, dass eine Trajektorie bevorzugt eine Mehrzahl von Aktivitätsereignissen umfasst, die auf einer Ausbreitung einer Wanderwelle auf einer Basilarmembran eines menschlichen Innenohrs basieren.

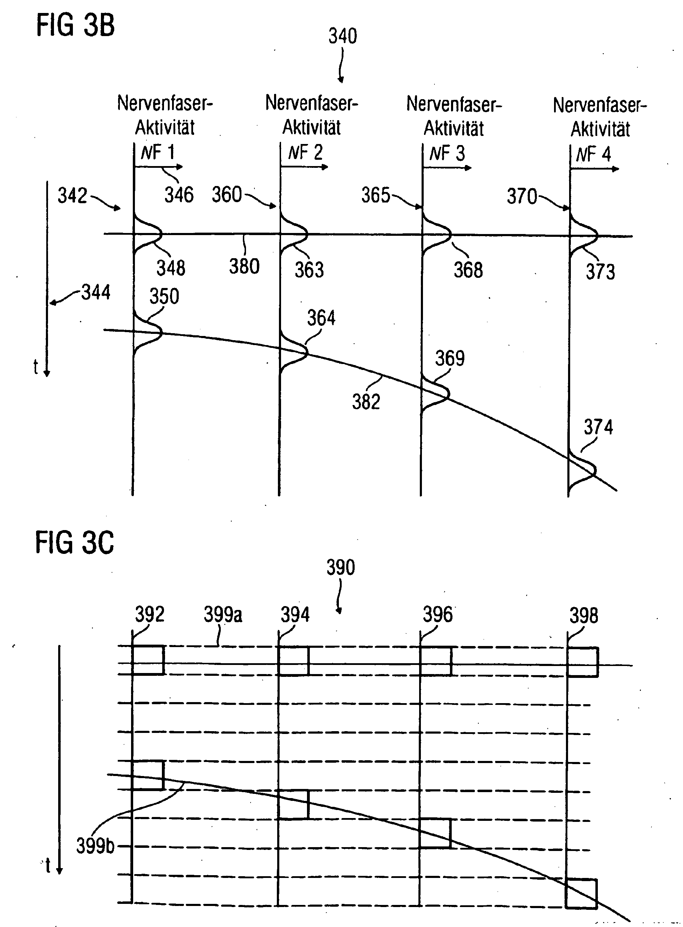

Fig. 3b zeigt eine graphische Darstellung einer Aktivität auf einer Mehrzahl von Nervenfasern, die mit inneren Hörzellen eines Gehörmodells gekoppelt sind. Die graphische Darstellung der Fig. 3b ist in ihrer Gesamtheit mit 340 bezeichnet. Eine erste graphische Darstellung 342 zeigt eine Aktivität einer ersten Nervenfaser, die beispielsweise mit der ersten inneren Hörzelle IHZ1 gekoppelt ist. Eine Zeitachse 344 beschreibt dabei die Zeit, während eine Potential-Achse 346 beispielsweise ein Potential auf einer ersten Nervenfaser NF1 beschreibt. Die graphische Darstellung 342 zeigt beispielsweise ein erstes Aktivitätsereignis 348, das eine Auslösung eines Aktionspotentials auf der ersten Nervenfaser NF1 umfasst. In anderen Worten, ein Potentialverlauf auf der ersten Nervenfaser NF1 weist eine Aktivität (zeitliche Veränderung bzw. Impuls) auf, die das Aktivitätsereignis 348 bildet. Die erste graphische Darstellung 342 zeigt weiterhin ein zweites Aktivitätsereignis 350 auf der ersten Nervenfaser NF1. Eine zweite graphische Darstellung 360 zeigt eine Aktivität einer zweiten Nervenfaser NF2, die beispielsweise mit einer zweiten inneren Hörzelle IHZ2 gekoppelt ist. Dabei sind zwei Aktivitätsereignisse 363, 364 auf der zweiten Nervenfaser NF2 ersichtlich. Eine dritte graphische Darstellung 365 zeigt zwei Aktivitätsereignisse 368, 369 auf einer dritten Nervenfaser NF3. Ferner zeigt eine vierte graphische Darstellung 370 zwei Aktivitätsereignisse 373, 374 auf einer vierten Nervenfaser NF4.

Ferner ist ersichtlich, dass in der graphischen Darstellung 340 der Fig. 3b, die eine Aktivität auf einer Mehrzahl von Nervenfasern NF1, NF2, NF3, NF4 als Funktion der Zeit beschreibt, die Aktivitätsereignisse 348, 363, 368, 373 mit einer ersten Trajektorie 380 verbunden sind bzw. alle auf der Trajektorie 308 liegen. Ferner sind die Aktivitätsereignisse 350, 364, 369, 374 durch die Trajektorie 382 verbunden bzw. liegen alle auf der Trajektorie 382.

Fig. 3c zeigt eine graphische Darstellung von zeitdiskretisierten und wertdiskretisierten Signalen, die beispielsweise auf den in den Fig. 3a oder 3b gezeigten Aktivitätsereignissen basieren. In anderen Worten, aus der zeitlichen Darstellung der Anzahl an Neurotransmitter-Vesikeln an einer Mehrzahl von inneren Hörzellen kann beispielsweise eine Mehrzahl von zeitdisktreten und wertdiskreten Signalen gewonnen werden, die die auftretenden Aktivitätsereignisse 308, 313, 318, 323, 309, 314, 319, 324 beschreiben. Beispielsweise beschreibt ein erstes Signal 392. Aktivitätsereignisse, die an oder in der ersten inneren Hörzelle IHZ1 auftreten. Ein zweites Signal 394 beschreibt beispielsweise Aktivitätsereignisse, die an oder in einer zweiten inneren Hörzelle IHZ2 auftreten. Ein drittes Signal 396 beschreibt beispielsweise Aktivitätsereignisse, die an oder in der dritten inneren Hörzelle IHZ3 auftreten, und ein viertes Signal 398 beschreibt beispielsweise Aktivitätsereignisse, die an oder in der vierten inneren Hörzelle IHZ4 auftreten.

Alternativ dazu können die vier Signale 392, 394, 396, 398 aber auch Aktivitätsereignisse auf den Nervenfasern NF1, NF2, NF3, NF4, beschreiben. In der zweidimensionalen zeitlichen Darstellung 390 der Fig. 3c liegen dabei Aktivitätsereignisse (bzw. aktive Zustände) auf den Signalen 392, 394, 396, 398 auf Trajektorien 398a, 399b.

In anderen Worten, die parallelen (beispielsweise binärwertigen) Signale 392, 394, 396, 398 stellen lediglich eine elektrische (beispielsweise binärwertige) Repräsentation der Aktivitätsereignisse 308, 313, 318, 323, 309, 314, 319, 324 bzw. Aktivitätsereignisse 348, 363, 368, 373, 350, 364, 369, 374 dar. Die Tatsache, dass Aktivitätsereignisse innerhalb einer zweidimensionalen Darstellung auf Trajektorien liegen, wird durch den Übergang in eine Darstellung als parallele Signale 392, 394, 396, 398 nicht beeinflusst.

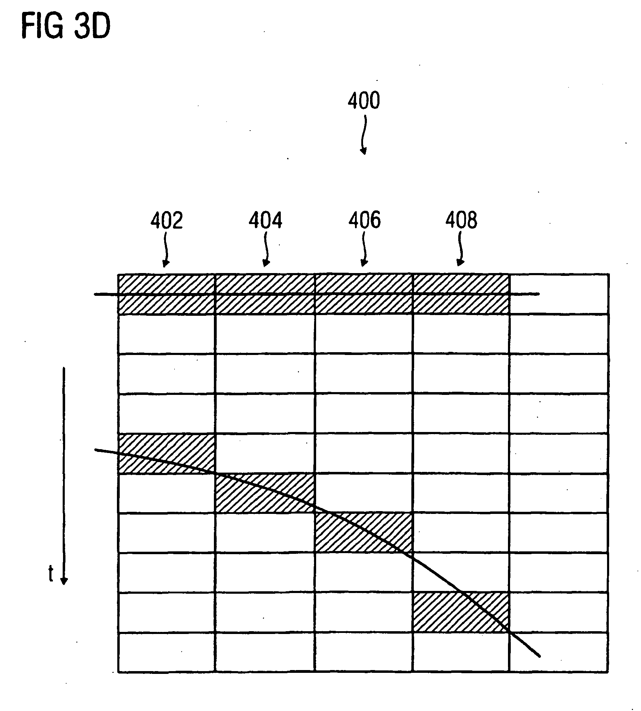

Fig. 3d zeigt ferner eine graphische Darstellung eines Musters, das den in der graphischen Darstellung 390 der Fig. 3c gezeigten elektrischen Signalen 392, 394, 396, 398 zugeordnet ist. Ein zeitlicher Verlauf des ersten Signals 392 entspricht dabei einer ersten Spalte 402 des in der graphischen Darstellung 400 gezeigten Musters. Ein zeitlicher Verlauf des zweiten Signals 394 entspricht einer zweiten Spalte 404 des in der graphischen Darstellung 400 gezeigten Musters. Ein zeitlicher Verlauf des dritten Signals 396 wird durch eine dritte Spalte 406 des Musters 400 repräsentiert. Ein zeitlicher Verlauf des vierten Signals 398 wird durch eine vierte Spalte 408 des Musters 400 repräsentiert.

In anderen Worten, das Muster 400 gemäß der Fig. 3d repräsentiert ein Auftreten von Aktivitätsereignissen 308, 309, 313, 314, 318, 319, 323, 324; 348, 350, 363, 364, 368, 369, 373, 374 in oder an einer Mehrzahl von (inneren) Hörzellen eines Gehörmodells oder an einer Mehrzahl von Nervenfasern des Gehörmodells. Eine Überführung des Aktivitätsmusters gemäß den Figuren 3a, 3b in ein Muster gemäß der Fig. 3d kann beispielsweise durch ein zeitliches, paralleles Abtasten der Signale 392, 394, 396, 398 und durch ein Weiterschieben von Abtastwerten der genannten Signale durch eine Schiebeeinrichtung erfolgen.

Somit sei darauf hingewiesen, dass die Aktivitätsmuster gemäß den Figuren 3a, 3b Verfahren der Mustererkennung offen stehen.

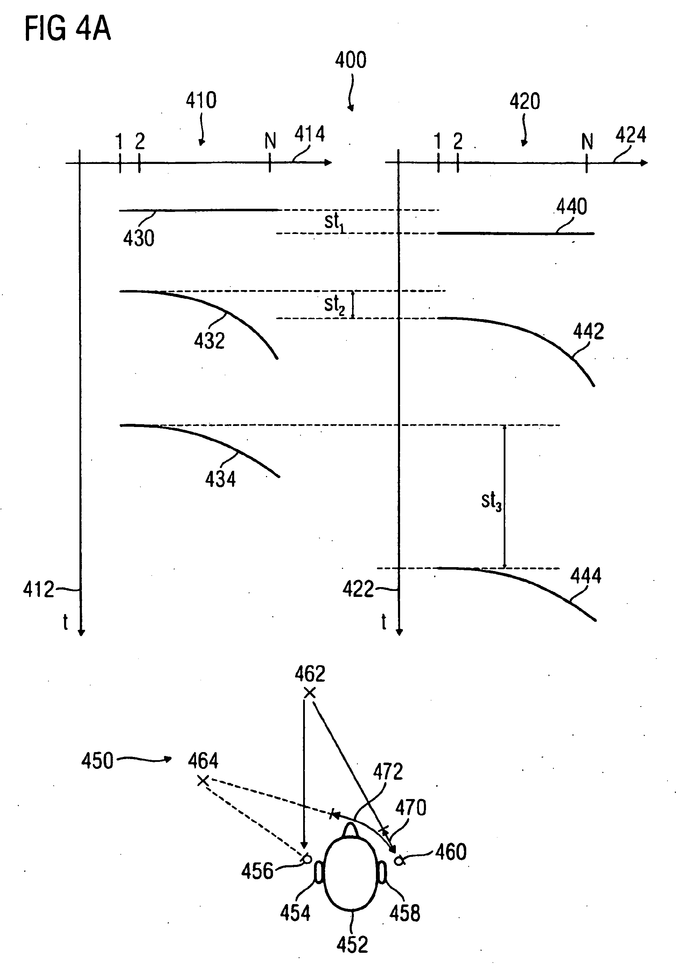

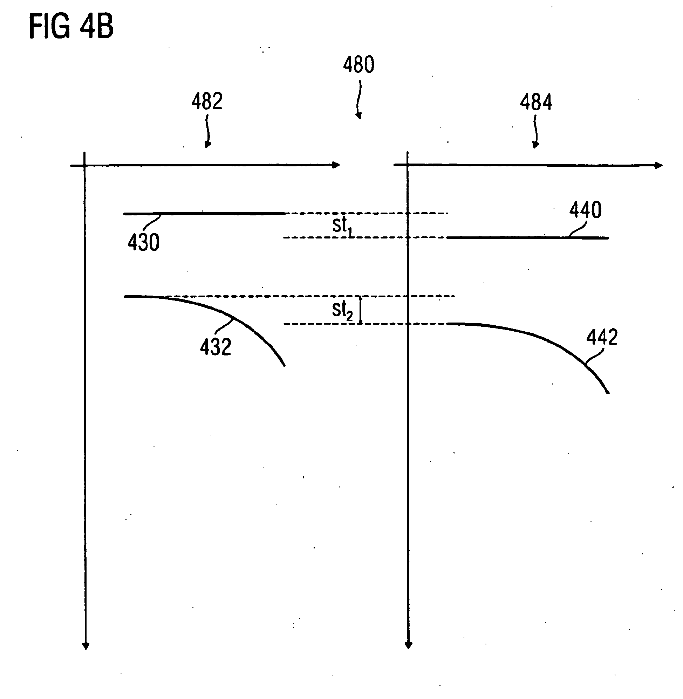

Im Folgenden wird anhand der Figuren 4a und 4b ein Beispiel für eine Verarbeitung von zwei Aktivitätsmustern beschrieben. Die graphische Darstellung der Fig. 4a ist in ihrer Gesamtheit mit 400 bezeichnet. Eine erste graphische Darstellung 410 beschreibt Trajektorien in einem Aktivitätsmuster an oder in einer Mehrzahl von inneren Hörzellen eines Hörmodells oder an einer Mehrzahl von Nervenfasern eines Gehörmodells. Eine erste Achse 412 beschreibt dabei die Zeit, während hingegen eine zweite Achse 414 eine räumliche Position von inneren Hörzellen (auf deren Aktivität das Aktivitätsmuster bzw. die Trajektorien basieren) entlang einer Cochlea beschreibt. Alternativ dazu zeigt die zweite Achse 414 einen Index einer Nervenfaser, wobei angenommen wird, dass die Nervenfasern mit inneren Hörzellen gekoppelt sind, und dass der Index der Nervenfaser eine Position der inneren Hörzelle, mit der die Nervenfaser gekoppelt ist, entlang der Cochlea in einer monotonen Weise beschreibt.

Es sei im Übrigen darauf hingewiesen, dass die graphische Darstellung 400 aus Gründen der Übersichtlichkeit keine einzelnen Aktivitätsereignisse mehr zeigt, sondern dass eine Mehrzahl von zusammengehörigen (zu einem einzigen Schallereignis gehörigen) Aktivitätsereignissen durch eine die Aktivitätsereignisse in der zweidimensionalen Darstellung verbindende Trajektorie beschrieben werden.

Die erste graphische Darstellung 410 beschreibt im Übrigen ein Aktivitätsmuster an einem Gehörmodell eines ersten Ohres, wobei davon ausgegangen wird, dass das Gehörmodell des ersten Ohres mit einem ersten Audiosignal beaufschlagt wird. Die zweite graphische Darstellung 420 zeigt eine entsprechende Darstellung eines Aktivitätsmusters in Form von zugehörigen Trajektorien an einem Gehörmodell eines zweiten Ohres, wobei davon ausgegangen wird, dass das Gehörmodell des zweiten Ohres mit einem zweiten Audiosignal beaufschlagt wird.

Die zweite graphische Darstellung 420 weist im Übrigen analog zu der ersten graphischen Darstellung 410 eine Abszisse 422 auf, an der die Zeit angetragen ist. Eine Ordinate 424 beschreibt eine räumliche Lage von inneren Hörzellen entlang der Cochlea bzw. einen Index der Nervenfasern, wie dies oben erläutert wurde.

Die erste graphische Darstellung 410 zeigt eine erste Trajektorie 430, die geradlinig ist oder nur eine leichte, erste Krümmung aufweist. Eine zweite Trajektorie 432 weist eine stärkere Krümmung auf als die erste Trajektorie 430, und tritt zeitlich nach der Trajektorie 430 auf. Eine dritte Trajektorie 434 weist im Übrigen beispielsweise eine dritte Krümmung auf, die sich von der ersten Krümmung der ersten Trajektorie 430 und der zweiten Krümmung der zweiten Trajektorie 432 unterscheidet. Die dritte Trajektorie 434 tritt im Übrigen zeitlich nach der ersten Trajektorie 430 und der zweiten Trajektorie 432 auf. Das in der graphischen Darstellung 420 dargestellte zweite Aktivitätsmuster weist eine vierte Trajektorie 440 auf. Es wird hier angenommen, dass die erste Trajektorie 430 und die vierte Trajektorie 440 auf dem gleichen Schallereignis einer Nutz-Schallquelle basieren, und dass somit die erste Trajektorie 430 und die vierte Trajektorie 440 gleiche Krümmungen oder Krümmungen, die sich um nicht mehr als eine vorgegebene zulässige Abweichung unterscheiden, aufweisen. Im Übrigen sei darauf hingewiesen, dass die vierte Trajektorie 440 zeitlich um eine Zeitverzögerung $\Delta t_1$ gegenüber der ersten Trajektorie 430 verzögert auftritt.

Die graphische Darstellung 420 zeigt ferner eine fünfte Trajektorie 442, die in dem zweiten Aktivitätsmuster enthalten ist. Es wird hierbei davon ausgegangen, dass die zweite Trajektorie 432 und die fünfte Trajektorie 442 auf dem gleichen Schallereignis der Nutz-Schallquelle basieren. Daher weisen die zweite Trajektorie 432 und die fünfte Trajektorie 442 eine gleiche Krümmung auf, oder die Krümmungen der zweiten Trajektorie 432 und der fünften Trajektorie 442 unterscheiden sich um weniger als eine vorgegebene maximal zulässige Abweichung. Ferner sei darauf hingewiesen, dass die fünfte Trajektorie 442 um die Verzögerungszeit $\Delta t_2$ gegenüber der zweiten Trajektorie 432 verzögert auftritt. Da die Trajektorien 430, 432, 440, 442 alle auf Schallereignissen der gleichen Nutzsignalquelle basieren, kann davon ausgegangen werden, dass die Verzögerungszeit $\Delta t_2$ um nicht mehr als eine vorgegebene maximal zulässige Abweichung von der Verzögerungszeit $\Delta t_1$ abweicht.

Die zweite graphische Darstellung 420 zeigt ferner eine sechste Trajektorie 444, die in dem zweiten Aktivitätsmuster enthalten ist. Es wird hierbei davon ausgegangen, dass die dritte Trajektorie 434 und die sechste Trajektorie 444 beide auf einem gleichen Schallereignis einer Stör-Schallquelle basieren. Aus diesem Grund kann angenommen, dass die dritte Trajektorie 434 und die sechste Trajektorie 444 beispielsweise eine gleiche Krümmung aufweisen, oder dass sich die Krümmungen der dritten Trajektorie 434 und der sechsten Trajektorie 444 um nicht mehr als eine vorgegebenen maximal zulässige Abweichung unterscheiden. Es wird ferner davon ausgegangen, dass sich die Stör-Schallquelle an einem anderen Ort befindet als die Nutz-Schallquelle. Eine graphische Darstellung 450 zeigt diesen Sachverhalt beispielhaft.

Die graphische Darstellung 450 zeigt eine Draufsicht auf einen menschlichen Kopf 452. In der Nähe eines ersten Ohres 454 (beispielsweise eines linken Ohres) ist ein erstes Mikrofon 456 angeordnet. In der Nähe eines zweiten Ohres 458 (beispielsweise eines rechten Ohres) ist ein zweites Mikrofon 460 angeordnet. Die graphische Darstellung 450 zeigt ferner die Nutz-Schallquelle 462 sowie die Stör-Schallquelle 464. Es sei ferner darauf hingewiesen, dass davon ausgegangen wird, dass die erste graphische Darstellung 410 das erste Aktivitätsmuster beschreibt, das aus dem von dem ersten Mikrofon 456 empfangenen Audiosignal unter Verwendung eines Gehörmodells des menschlichen Ohres erzeugt wird, und dass ferner die zweite graphische Darstellung 420 das zweite Aktivitätsmuster zeigt, das basierend auf dem von dem zweiten Mikrofon 460 gelieferten Mikrofonsignal durch Anwendung eines Gehörmodells (z.B. des menschlichen Gehörs) auf das von dem zweiten Mikrofon 460 gelieferte Audiosignal erzeugt wird.

Aus der in der Draufsicht 450 gezeigten Anordnung der Mikrofone 456, 460 sowie der die Nutz-Schallquelle 462 und der Stör-Schallquelle 464 ist ersichtlich, dass beispielsweise ein Laufzeitunterschied zwischen der Nutz-Schallquelle 462 und dem ersten Mikrofon 456 und zwischen der Nutz-Schallquelle 462 und dem zweiten Mikrofon 460 besteht. In anderen Worten, ein Schallereignis der Nutz-Schallquelle 462 trifft bei dem ersten Mikrofon 456 eher ein als bei dem zweiten Mikrofon 460. Der entsprechende Wegunterschied ist im Übrigen mit 470 bezeichnet, und resultiert in der Zeitverschiebung $\Delta t_1$.

In anderen Worten, aufgrund des Wegunterschieds 470 wird die vierte Trajektorie 440 in dem zweiten Aktivitätsmuster um die Verzögerungszeit $\Delta t_1$ später erzeugt als die erste Trajektorie 430 in dem ersten Aktivitätsmuster. Wird davon ausgegangen, dass sich die Nutz-Schallquelle 462 nicht bewegt, so kann ferner angenommen werden, dass die zweite Verzögerungszeit $\Delta t_2$ gleich der ersten Verzögerungszeit $\Delta t_1$ ist.

Ferner wird darauf hingewiesen, dass eine Wegstrecke zwischen der Stör-Schallquelle 464 und dem ersten Mikrofon 456 um einen Wegunterschied 472 kürzer ist als eine Wegstrecke zwischen der Stör-Schallquelle 464 und dem zweiten Mikrofon 460. Aus diesem Grund tritt die sechste Trajektorie 444, die ein Schall-Ereignis der Stör-Schallquelle 464 beschreibt, um die Verzögerungszeit $\Delta t_3$ später auf als die dritte Trajektorie 434, die das gleiche Schallereignis der Stör-Schallquelle 464 beschreibt. Die Verzögerungszeit $\Delta t_3$ ist dabei durch den Wegunterschied 472 bestimmt.

In dem gezeigten Beispiel ist der Wegunterschied 472 größer als der Wegunterschied 470. Somit ist die Verzögerungszeit $\Delta t_3$ größer als die Verzögerungszeit $\Delta t_1$ bzw. größer als die Verzögerungszeit $\Delta t_2$. In anderen Worten, die Verzögerungszeiten $\Delta t_1$, $\Delta t_2$, $\Delta t_3$ zwischen Trajektorien 430, 440; 432, 442; 434, 444 in dem ersten Aktivitätsmuster und in dem zweiten Aktivitätsmuster, die zu gleichen Schallereignissen gehören, ist abhängig von einer Lage der jeweiligen Schallquelle relativ zu den beiden Mikrofonen 456, 460 (und abhängig von einem Winkel, unter dem die entsprechenden Schallquellen sich bezogen auf eine Verbindungslinie der beiden Mikrofone 456, 460 befinden).

Wie schon oben beschrieben ist die erfindungsgemäße Vorrichtung ausgelegt, um zunächst in dem ersten Aktivitätsmuster eine erste Trajektorie und in dem zweiten Aktivitätsmuster eine zweite Trajektorie zu identifizieren, die auf dem gleichen Schallereignis basieren. Dies kann beispielsweise dadurch erfolgen, dass Trajektorien identifiziert werden, die eine gleiche Krümmung und/oder eine gleiche Länge aufweisen, und die ferner optional zusätzlich innerhalb eines vorgegebenen maximalen Zeitintervalls auftreten. Durch die Vorgabe eines maximalen Zeitintervalls wird dabei berücksichtigt, dass eine zeitliche Verschiebung zwischen zwei zu einem gleichen Schallereignis gehörigen Trajektorien in dem ersten Aktivitätsmuster und in dem zweiten Aktivitätsmuster durch eine Laufzeitdifferenz zwischen dem ersten Mikrofon 456 und dem zweiten Mikrofon 460 nach obenhin beschränkt ist.

Basierend auf der genannten Verarbeitungsvorschrift erkennt der Identifizierer 120 beispielsweise, dass die erste Trajektorie 430 in dem ersten Aktivitätsmuster und die vierte Trajektorie 440 in dem zweiten Aktivitätsmuster eine gleiche Krümmung aufweisen, und ordnet daher die beiden Trajektorien 430, 440 dem gleichen Schallereignis zu. Der Identifizierer 120 erkennt ferner, dass die zweite Trajektorie 432 und die fünfte Trajektorie 442 auf dem gleichen Schallereignis basieren, da die beiden Trajektorien eine gleiche Krümmung und/oder eine gleiche Länge aufweisen. Ferner erkennt der Identifizierer 120, dass die dritte Trajektorie 434 und die sechste Trajektorie 444 auf dem gleichen Schallereignis basieren.

Der Bestimmer 120 bestimmt ferner die Zeitverzögerung $\Delta t_1$ zwischen dem Auftreten der ersten Trajektorie 430 und der vierten Trajektorie 440, also zwischen den zusammengehörigen Trajektorien, die auf dem gleichen Schallereignis basieren. Ferner bestimmt der Bestimmer im Übrigen die zeitliche Verzögerung $\Delta t_2$ zwischen dem Auftreten der zweiten Trajektorie 432 und dem Auftreten der fünften Trajektorie 442. Außerdem bestimmt der Identifizierer 120 die zeitliche Verzögerung $\Delta t_3$ zwischen dem Auftreten der dritten Trajektorie 434 und der sechsten Trajektorie 444.

Der Bestimmer 130 bestimmt basierend auf den Informationen über die Trajektorien 430, 432, 434, 440, 442, 444, welche der Trajektorien 430, 433, 434, 440, 442, 444 einem Schallereignis einer Nutz-Schallquelle zugeordnet sind, und welche Trajektorien einem Schallereignis der Stör-Schallquelle zugeordnet sind.

In dem einfachsten Fall umfasst der Bestimmer 130 eine Einrichtung zum Erkennen, ob eine zeitliche Verschiebung zwischen zwei zusammengehörigen Trajektorien 430, 440; 432, 442; 434, 444 innerhalb eines vorgegebenen zulässigen Bereichs. Es wird hierbei angenommen, dass der Bestimmer 130 einen gespeicherten minimalen Verzögerungswert $\Delta t_{min}$ sowie einen gespeicherten maximalen Verzögerungswert $\Delta t_{max}$ umfasst, wobei der minimal zulässige Verzögerungswert $\Delta t_{min}$ und der zulässige maximale Verzögerungswert $\Delta t_{max}$ einen zulässigen Bereich als ein Intervall $[\Delta t_{min;}\ \Delta t_{max}]$ definieren. Es sei hierbei angenommen, dass gilt:

$$\Delta t_{min} \leq \Delta t_1 \leq \Delta t_{max} \text{ und } \Delta t_{min} \leq \Delta t_2 \leq \Delta t_{max}.$$

Ferner gilt beispielsweise:

$$\Delta t_{max} \leq \Delta t_3.$$

Somit erkennt der Bestimmer 130, dass die Zeitverzögerung $\Delta t_1$ zwischen der ersten Trajektorie 430 und der vierten Trajektorie 440 in dem zulässigen Bereich liegt, und dass ferner die Zeitverzögerung $\Delta t_2$ zwischen der zweiten Trajektorie 432 und der fünften Trajektorie 442 in dem zulässigen Bereich liegt. Daher signalisiert der Bestimmer 130 durch das Signal 136, dass es sich bei den Trajektorien 430, 432, 440, 442 um Trajektorien handelt, die ein Schallereignis der Nutz-Schallquelle zugeordnet sind. Der Bestimmer 130 erkennt ferner, dass die Zeitverzögerung $\Delta t_3$ außerhalb des zulässigen Bereichs liegt, und signalisiert somit beispielsweise über das Signal 136, dass die Trajektorien 434, 444 einem Schallereignis der Stör-Schallquelle zugeordnet sind.

Das Filter 140 erzeugt basierend auf der von dem Bestimmer 130 gelieferten Information 136 ein gefiltertes Aktivitätsmuster. Ein Beispiel für das gefilterte Aktivitätsmuster ist in Fig. 4b gezeigt. Die graphische Darstellung der Fig. 4b in ihrer Gesamtheit ist mit 480 bezeichnet. Die graphische Darstellung 480 zeigt in einer ersten graphischen Darstellung 482 ein erstes gefiltertes Aktivitätsmuster, das auf dem ersten Aktivitätsmuster gemäß der graphischen Darstellung 410 basiert. Das erste gefilterte Aktivitätsmuster umfasst die erste Trajektorie 430 sowie die zweite Trajektorie 432 entsprechend dem ersten Aktivitätsmuster. Allerdings umfasst das erste gefilterte Aktivitätsmuster nicht die dritte Trajektorie 430, die auf einem Schallereignis der Störsignalquelle basiert. In anderen Worten, bei der Erzeugung des ersten gefilterten Aktivitätsmusters entfernt beispielsweise das Filter 140 die dem Schallereignis der Stör-Schallquelle zugeordnete Trajektorie 434. Alternativ dazu übernimmt das Filter 140 lediglich die Trajektorie 430 und 432, die auf Nutz-Schallereignissen der Nutz-Schallquelle basieren, in das erste gefilterte Aktivitätsmuster.

Eine zweite graphische Darstellung 484 zeigt ein zweites gefiltertes Aktivitätsmuster. Das zweite gefilterte Aktivitätsmuster 484 enthält entsprechend dem zweiten Aktivitätsmuster die vierte Trajektorie 440 und die fünfte Trajektorie 442, nicht aber die sechste Trajektorie 444. Das zweite gefilterte Aktivitätsmuster enthält somit diejenigen Trajektorien des ersten Aktivitätsmusters, die Nutz-Schallereignissen von der Nutz-Schallquelle zugeordnet sind, nicht aber Trajektorien, die Stör-Schallereignissen von der Stör-Schallquelle zugeordnet sind. Analog dazu umfasst das zweite gefilterte Aktivitätsmuster die Schallereignisse des zweiten Aktivitätsmusters, die Nutz-Schallereignissen der Nutz-Schallquelle zugeordnet sind, nicht aber Trajektorien, die Stör-Schallereignissen der Stör-Schallquelle zugeordnet sind.

Im Übrigen sei darauf hingewiesen, dass das erste gefilterte Aktivitätsmuster eine Mehrzahl von Aktivitätsereignissen umfasst, die die jeweiligen Trajektorien bilden. In anderen Worten, eine jede Trajektorie repräsentiert eine Mehrzahl von einzelnen Aktivitätsereignissen.

Somit ist insgesamt festzuhalten, dass das erste gefilterte Aktivitätsmuster den Informationsinhalt der Nutz-Schallquelle beschreibt, die an dem Ort des ersten Mikrofons 456 wahrnehmbar ist. Der Informationsinhalt der Stör-Schallquelle 464 ist in dem ersten gefilterten Aktivitätsmuster hingegen nicht oder nur in abgeschwächter Form enthalten. Analog dazu enthält das zweite gefilterte Aktivitätsmuster einen Informationsinhalt der Nutz-Schallquelle 464, die an dem Ort des zweiten Mikrofons 460 wahrnehmbar ist, nicht oder nur in abgeschwächter Form hingegen einen Informationsinhalt der Stör-Schallquelle 464.

Im folgenden wird eine Erweiterung des vorher beschriebenen Konzepts beschrieben, die verwendet werden kann, um einen verbesserten Höreindruck zu erzielen bzw. um eine verbesserte Entfernung von Störsignalen von einer StörSignalquelle zu erreichen. Gemäß einem Ausführungsbeispiel der vorliegenden Erfindung werden Nervenaktivitätsmuster getrennt für innere Hörzellen mit verschiedenen Ansprechempfindlichkeiten berechnet.

In anderen Worten, eine erweiterte Identifizierungseinrichtung empfängt von einem Ohrmodell eines ersten Ohres ein erstes Aktivitätsmuster, das Aktivitätsereignisse in oder an inneren Hörzellen, die eine erste Ansprechempfindlichkeit aufweisen, beschreibt, ein zweites Aktivitätsmuster, das Aktivitätsereignisse in oder an inneren Hörzellen, die eine zweite Ansprechempfindlichkeit aufweisen, beschreibt, und ein drittes Aktivitätsmuster, das Aktivitätsereignisse in oder an inneren Hörzellen, die eine dritte Ansprechempfindlichkeit aufweisen, beschreibt. Die erste Ansprechempfindlichkeit ist bevorzugt größer als die zweite Ansprechempfindlichkeit, und die zweite Ansprechempfindlichkeit ist bevorzugt größer als die dritte Ansprechempfindlichkeit. Im übrigen sei darauf hingewiesen, dass das erste Aktivitätsmuster, das zweite Aktivitätsmuster und das dritte Aktivitätsmuster Aktivitätsereignisse an verschiedenen Typen von inneren Hörzelle mit verschiedenen Ansprechempfindlichkeiten beschreiben, die sich aufgrund einer Anregung der verschiedenen Hörzellen basierend auf dem gleichen Audiosignal ergeben. Beispielsweise stammen die drei Aktivitätsmuster von innneren Hörzellen mit einer niedrigen spontanen Emissionsrate (LSR), mit einer mittleren spontanen Emissionsrate (MSR) oder mit einer hohen spontanen Emissionsrate (HSR).

Die erweiterte Identifizierungseinrichtung empfängt ferner von einem Gehörmodell eines zweiten Ohres ein viertes Aktivitätsmuster, das Aktivitätsereignisse in oder an inneren Hörzellen, die eine vierte Ansprechempfindlichkeit aufweisen, beschreibt, ein fünftes Aktivitätsmuster, das Aktivitätsereignisse in oder an inneren Hörzellen, die eine fünfte Ansprechempfindlichkeit aufweisen, beschreibt, und ein sechstes Aktivitätsmuster, das Aktivitätsereignisse in oder an inneren Hörzellen, die eine sechste Ansprechempfindlichkeit aufweisen, beschreibt. Die vierte Ansprechempfindlichkeit ist bevorzugt größer als die fünfte Ansprechempfindlichkeit, und die fünfte Ansprechempfindlichkeit ist bevorzugt größer als die sechste Ansprechempfindlichkeit. Im übrigen sei darauf hingewiesen, dass das vierte Aktivitätsmuster, das fünfte Aktivitätsmuster und das sechste Aktivitätsmuster Aktivitätsereignisse an verschiedenen Typen von inneren Hörzellen mit verschiedenen Ansprechempfindlichkeiten beschreiben, die sich aufgrund einer Anregung der verschiedenen Hörzellen basierend auf dem gleichen Audiosignal ergeben.

Die Gehörmodelle des ersten Ohres und des zweiten Ohres können im übrigen optional Teil der erfindungsgemäßen Vorrichtung sein.

In anderen Worten, das erste Aktivitätsmuster beschreibt beispielsweise Aktivitätsereignisse die sich an inneren Hörzellen einer hohen Empfindlichkeit ergeben, wenn die Hörzellen der hohen Empfindlichkeit mit einem ersten Audiosignal angeregt werden. Das zweite Aktivitätsmuster beschreibt Aktivitätsereignisse, die sich in oder an Hörzellen einer mittleren Empfindlichkeit ergeben, wenn die Hörzellen durch das erste Audiosignal angeregt werden. Das dritte Aktivitätsmuster beschreibt ferner ein Aktivitätsmuster bzw. Aktivitätsereignisse, die sich in oder an inneren Hörzellen mit einer niedrigen Empfindlichkeit ergeben, wenn die inneren Hörzelle (bzw. ein Ohrmodell, das die entsprechenden inneren Hörzellen umfasst) durch das erste Audiosignal angeregt wird.

Das vierte Aktivitätsmuster beschreibt Aktivitätsereignisse, die sich an inneren Hörzellen einer hohen Empfindlichkeit ergeben, wenn die entsprechenden inneren Hörzellen (bzw. das zugrunde liegende Gehörmodell) durch ein zweites Audiosignal angeregt werden. Das fünfte Aktivitätsmuster beschreibt Aktivitätsereignisse in oder an inneren Hörzellen einer mittleren Empfindlichkeit, wenn die inneren Hörzellen durch das zweite Audiosignal angeregt werden, und das sechste Aktivitätsmuster beschreibt Aktivitätsereignisse in oder an inneren Hörzellen einer geringen Empfindlichkeit, wenn die entsprechenden inneren Hörzellen durch das zweite Audiosignal angerecht werden.

In anderen Worten, das erste, zweite und dritte Aktivitätsmuster beschreiben Aktivitätsereignisse an inneren Hörzellen verschiedener Empfindlichkeiten eines Gehörmodells eines ersten Ohres, das durch das erste Audiosignal angeregt wird. Das vierte, fünfte und sechste Aktivitätsmuster beschreiben Aktivitätsereignisse an inneren Hörzellen verschiedener Empfindlichkeiten eines Gehörmodells eines zweiten Ohres, das durch das zweite Audiosignal angeregt wird.

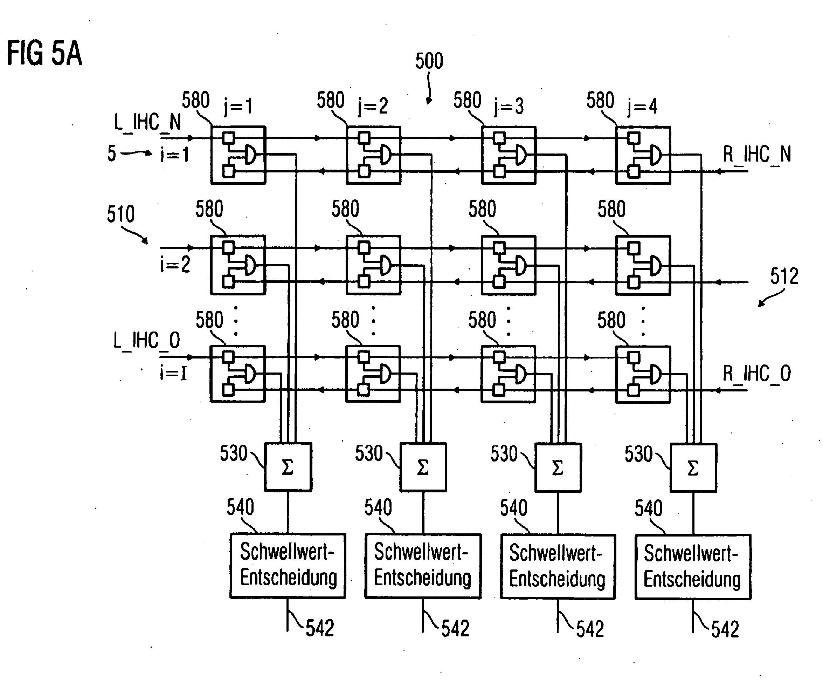

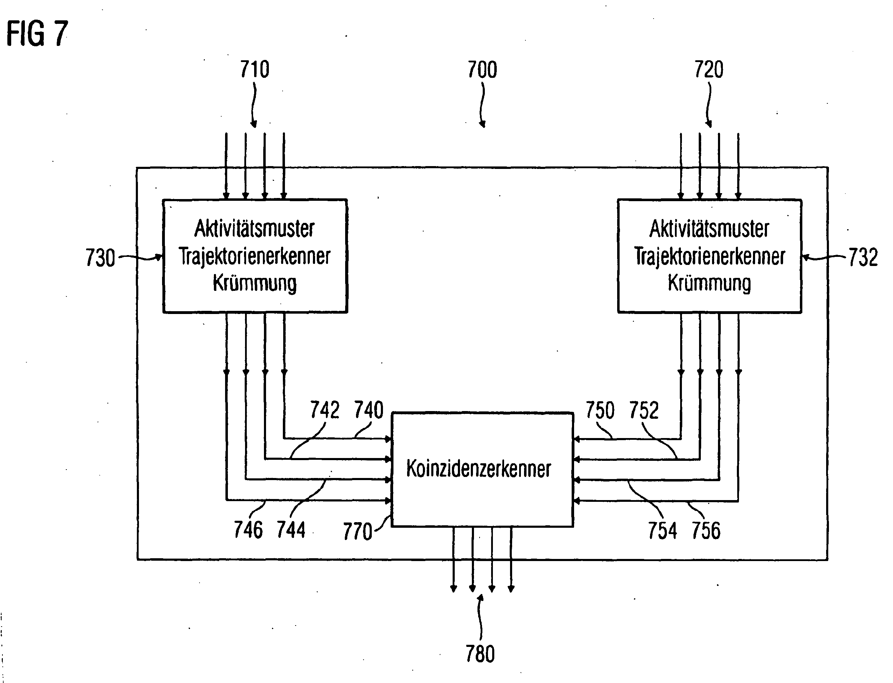

Der Identifizierer ist in diesem Fall ausgelegt, um Aktivitätsmuster an dem Gehörmodell des ersten Ohres und Aktivitätsmuster an dem Gehörmodell des zweiten Ohres, die inneren Hörzellen einer gleichen Empfindlichkeits-Stufe (hohe Empfindlichkeit; mittlere Empfindlichkeit; geringe Empfindlichkeit) zugeordnet sind, miteinander zu verarbeiten. Beispielsweise ist der Identifizierer in diesem Fall ausgelegt, um eine erste Trajektorie in dem ersten Aktivitätsmuster und eine vierte Trajektorie in dem vierten Aktivitätsmuster zu identifizieren, die einem gleichen Schallereignis zugeordnet sind. Zu diesem Zweck werden beispielsweise das erste Aktivitätsmuster und das vierte Aktivitätsmuster einer ersten Erkennungseinrichtung zugeführt, die ausgelegt ist, um in dem ersten Aktivitätsmuster und dem vierten Aktivitätsmuster Trajektorien gleicher Krümmung und/oder gleicher Länge als zusammengehörige Trajektorien zu identifizieren. Bei der ersten Erkennungseinrichtung kann es sich beispielsweise um eine Multikoinzidenzeinheit(beispielsweise gemäß der Fig. 5a) oder um eine Erkennungseinrichtung gemäß der Fig. 7 handeln. Die entsprechende erste Erkennungseinrichtung liefert somit eine Information darüber, zu welchen Zeitpunkten und mit welcher zeitlichen Verschiebung Trajektorien, die einem gleichen Schallereignis zugeordnet sind, in dem ersten Aktivitätsmuster und dem vierten Aktivitätsmuster auftreten.

In ähnlicher Weise empfängt eine zweite Erkennungseinrichtung beispielsweise das zweite Aktivitätsmuster und das fünfte Aktivitätsmuster und bestimmt darin Trajektorien die einem gleichen Schallereignis zugeordnet sind. Die zweite Erkennungseinrichtung liefert somit eine Information darüber, wann und/oder mit welcher zeitlichen Verschiebung gegeneinander in dem zweiten Aktivitätsmuster und dem fünften Aktivitätsmuster Trajektorien enthalten sind, die einem gleichen Schallereignis zugeordnet sind.

Eine dritte Erkennungseinrichtung empfängt ferner das dritte Aktivitätsmuster und das sechste Aktivitätsmuster und identifiziert darin Trajektorien, die gleichen Schallereignissen zugeordnet sind. Die dritte Erkennungseinrichtung liefert daher eine Information über Trajektorien in dem dritten Aktivitätsmuster und dem sechsten Aktivitätsmuster, die gleichen Schallereignissen zugeordnet sind, sowie eine Information über eine zeitliche Verschiebung zwischen Trajektorien, die gleichen Schallereignissen zugeordnet sind.

Durch einen Vergleich der durch die erste Erkennungseinrichtung, die zweite Erkennungseinrichtung und die dritte Erkennungseinrichtung gelieferten Informationen kann im Übrigen festgestellt werden, wie groß eine Lautstärke eines Schallereignisses ist, das durch eine Trajektorie in einem der Aktivitätsmuster beschrieben wird.

Es werden im Folgenden drei Lautstärkegrade unterschieden. So wird angenommen, dass es Schallereignisse großer Lautstärke, Schallereignisse mittlerer Lautstärke und Schallereignisse geringer Lautstärke gibt. Es wird angenommen, dass ein Schallereignis großer Lautstärke eine Trajektorie sowohl in dem ersten Aktivitätsmuster, im zweiten Aktivitätsmuster, in dem dritten Aktivitätsmuster, in dem vierten Aktivitätsmuster, in dem fünften Aktivitätsmuster und in dem sechsten Aktivitätsmuster zur Folge hat. Es wird ferner davon ausgegangen, dass ein Schallereignis mittlerer Lautstärke eine Trajektorie in dem ersten Aktivitätsmuster, in dem zweiten Aktivitätsmuster, in dem vierten Aktivitätsmuster und in dem fünften Aktivitätsmuster zur Folge hat, nicht aber Trajektorien in dem dritten Aktivitätsmuster und in dem sechsten Aktivitätsmuster. Dies liegt darin begründet, dass das dritte Aktivitätsmuster und das sechste Aktivitätsmuster durch Hörzellen geringer Empfindlichkeit gebildet werden, weil davon ausgegangen wird, dass ein Schallereignis mittlerer Lautstärke nicht stark genug ist, um die Hörzellen geringer Empfindlichkeit (deren Reakion durch das dritte Aktivitätsmuster und das sechste Aktivitätsmuster beschrieben wird) anzuregen. Ein Schallereignis geringer Lautstärke erzeugt hingegen lediglich Trajektorien in dem ersten Aktivitätsmuster und in dem vierten Aktivitätsmuster. Es wird angenommen, dass ein Schallereignis geringer Lautstärke nicht ausreicht, um Hörzellen mittlerer Empfindlichkeit sowie geringer Empfindlichkeit anzuregen, so dass ein Schallereignis geringer Lautstärke nicht zu einer Trajektorie in dem zweiten Aktivitätsmuster und in dem fünften Aktivitätsmuster (die durch Hörzellen mittlerer Empfindlichkeit gebildet werden) sowie ferner ebenso wenig in dem dritten Aktivitätsmuster und dem sechsten Aktivitätsmuster (die durch Hörzellen geringer Empfindlichkeit gebildet werden) führt.

Somit ist es möglich, durch einen Vergleich der Trajektorien in dem ersten Aktivitätsmuster, in dem zweiten Aktivitätsmuster und in dem dritten Aktivitätsmuster eine Information über eine Lautstärke eines Schallereignisses zu gewinnen.

In anderen Worten, eine Schallereignis-Lautstärke-Erkennungseinrichtung, die beispielsweise Teil des Identifizierers oder Teil des Bestimmers ist, kann ausgelegt sein, um eine Information und eine Lautstärke eines Schallereignisses, das einer Trajektorie zugrunde liegt, für eine Mehrzahl von Trajektorien zu erzeugen. Die Lautstärke-Erkennungseinrichtung kann beispielsweise ausgelegt sein, um zu erkennen, ob in dem ersten Aktivitätsmuster und in dem zweiten Aktivitätsmuster gleichzeitig (bzw. innerhalb einer vorgegebenen maximalen Zeitintervalls) Trajektorien einer gleichen Krümmung (bzw. Trajektorien verschiedener Krümmung, deren Krümmungen sich um weniger als einen vorgegebenen maximal zulässigen Krümmungsunterschied unterscheiden) vorliegen. Wird dabei festgestellt, dass eine Trajektorie einer betreffenden Krümmung im ersten Aktivitätsmuster auftritt, zu der keine zugehörigen Trajektorien mit entsprechender (zumindest näherungsweise gleicher Krümmung in dem zweiten Aktivitätsmuster vorhanden ist, so kann der Lautstärke-Bestimmer beispielsweise erkennen, dass die in dem ersten Aktivitätsmuster vorhandenen Trajektorien Schallereignissen geringer Lautstärke zugeordnet sind. Ferner kann der Lautstärke-Erkenner alternativ oder zusätzlich ausgelegt sein, um zu erkennen, ob in dem zweiten Aktivitätsmuster und in dem dritten Aktivitätsmuster Trajektorien zumindest näherungsweise gleicher Krümmung zumindest näherungsweise gleichzeitig auftreten. Existiert zu einer Trajektorien in dem zweiten Aktivitätsmuster keine entsprechende näherungsweise gleichzeitig auftretende zugehörige Trajektorie näherungsweise gleicher Krümmung, so kann der Lautstärke-Erkenner beispielsweise feststellen, dass das Schallereignis, zu der die betreffende Krümmung in dem zweiten Aktivitätsmuster gehört, eine mittlere Lautstärke aufweist.

Ferner kann der Lautstärke-Erkenner beispielsweise feststellen, dass ein Schallereignis eine große Lautstärke aufweist, wenn sowohl in dem ersten Aktivitätsmuster als auch in dem zweiten und dem dritten Aktivitätsmuster näherungsweise gleichzeitig Trajektorien näherungsweise gleicher Krümmung auftreten.

Eine entsprechende Verarbeitung kann im Übrigen auch für das vierte Aktivitätsmuster, das fünfte Aktivitätsmuster und das sechste Aktivitätsmuster erfolgen. Tritt in dem vierten Aktivitätsmuster eine Trajektorie auf, zu der keine entsprechende näherungsweise gleichzeitig auftretende Trajektorie näherungsweise gleicher Krümmung in dem fünften Aktivitätsmuster existiert, so kann der Lautstärke-Erkenner feststellen, dass die in dem vierten Aktivitätsmuster auftretende Krümmung einem Schallereignis mit geringer Lautstärke zugeordnet ist. Identifiziert der Lautstärke-Erkenner ferner eine Trajektorie in dem fünften Aktivitätsmuster, zu der keine zugehörige näherungsweise gleichzeitig auftretende Trajektorie näherungsweise gleicher Krümmung in dem sechsten Aktivitätsmuster existiert, so kann der Lautstärke-Erkenner feststellen, dass die in dem fünften Aktivitätsmuster identifizierte Trajektorie einem Schallereignis mittlerer Lautstärke zugeordnet ist. Stellt der Lautstärke-Erkenner ferner fest, dass in dem vierten Aktivitätsmuster, in dem fünften Aktivitätsmuster und in dem sechsten Aktivitätsmuster näherungsweise gleichzeitig Trajektorien näherungsweise gleicher Krümmung auftreten, so kann der Lautstärke-Erkenner feststellen, dass entsprechende Trajektorien einem Schallereignis großer Lautstärke zugeordnet sind.

In anderen Worten, der Lautstärke-Erkenner ist ausgelegt, um den in den untersuchten Aktivitätsmustern auftretenden

Trajektorien (oder zumindest einem Teil der Trajektorien) eine Lautstärke-Information zuzuordnen.

Zu diesem Zweck analysiert der Lautstärke-Erkenner ganz allgemein, ob in den verschiedenen betrachteten Aktivitätsmusters näherungsweise gleichzeitig (d.h. innerhalb eines vorgegebenen maximalen Zeitintervalls) Trajektorien gleicher Krümmung auftreten, und wenn ja, in welchen der Aktivitätsmuster näherungsweise gleichzeitig Trajektorien näherungsweise gleicher Krümmung auftreten. Basierend auf der genannten Information bzw. basierend auf einem Vergleich zwischen Trajektorien, die in verschiedenen Aktivitätsmustern auftreten, die inneren Hörzellen verschiedener Empfindlichkeit zugeordnet sind, bestimmt der Lautstärke-Erkenner somit die den Trajektorien zugeordnete Lautstärke-Information.

Im Übrigen ist das Filter 140 bei einem Ausführungsbeispiel ausgelegt, um die Lautstärke-Information bei der Filterung zu berücksichtigen. In anderen Worten, das Filter 140 kann ausgelegt sein, um die Lautstärke-Information von dem Lautstärke-Erkenner zu empfangen. So kann das Filter 140 beispielsweise ausgelegt sein, um alle Trajektorien auszufiltern bzw. zu entfernen oder zu bedämpfen, deren zugeordnete Lautstärke-Information kleiner als eine vorgegebene Mindest-Lautstärke ist. Alternativ dazu kann das Filter aber auch ausgelegt sein, um das gefilterte Aktivitätsmuster so zu erzeugen, dass in dem gefilterten Aktivitätsmuster nur Trajektorien enthalten sind, die gemäß der von dem Lautstärke-Erkenner gelieferten Information Schallereignissen zugeordnet sind, die eine Lautstärke innerhalb eines vorgegebenen Bereichs aufweisen. Beispielsweise kann das Filter 140 ausgelegt sein, um das gefilterte Aktivitätsmuster so zu erzeugen, dass im Aktivitätsmuster Trajektorien hervorgehoben bzw. verstärkt sind, die zu Schallereignissen mit einer geringen Lautstärke gehören, während beispielsweise Trajektorien, die zu Schallereignissen mit einer größeren Lautstärke gehören, relativ dazu bedämpft oder sogar ganz entfernt werden.

Somit kann beispielsweise erreicht werden, dass in dem gefilterten Aktivitätsmuster nur noch oder zumindest im Wesentlichen Trajektorien enthalten sind, die zu Schallereignissen einer geringen Lautstärke gehören. Somit wird es ermöglicht, dass durch das gefilterte Aktivitätsmuster gerade leise Signalanteile in den ursprünglichen Aktivitätsmustern beschrieben werden, wodurch eine Verständlichkeit der leisen Anteile in den ursprünglichen Aktivitätsmustern verbessert oder erst ermöglicht wird.

Zusammenfassend lässt sich somit festhalten, dass die erfindungsgemäße Vorrichtung gemäß der Fig. 1 bzw. der Fig. 2 dadurch erweitert werden kann, dass die Vorrichtung zwei Sätze von Aktivitätsmustern an Gehörmodellen zweier Ohren empfängt, die jeweils Aktivitätsereignisse an Hörzellen mit einer hohen spontanen Emissionsrate, einer mittleren spontanen Emissionsrate und einer niedrigen spontanen Emissionsrate beschreiben. Eine Identifizierung von zu gleichen Schallereignissen gehörenden Trajektorien erfolgt daraufhin zunächst getrennt für Aktivitätsmuster von Hörzellen verschiedener Emissionsraten. In anderen Worten, ein Aktivitätsmuster des Ohrmodells des ersten Ohres für Hörzellen einer hohen Emissionsrate wird zusammen mit einem Aktivitätsmuster an dem Ohrmodell des zweiten Ohres für Hörzellen einer hohen Emissionsrate verarbeitet, um eine Information über korrespondierende (auf einem gleichen Schallereignis basierende) Trajektorien zu erhalten. In ähnlicher Weise wird eine gemeinsame Verarbeitung für zwei Aktivitätsmuster, die zu Hörzellen einer mittleren Emissionsrate gehören, durchgeführt. Ferner wird eine gemeinsame Verarbeitung für zwei Aktivitätsmuster von Hörzellen einer geringen Emissionsrate durchgeführt. Durch einen Vergleich zwischen den Trajektorien in dem ersten Aktivitätsmuster, in dem zweiten Aktivitätsmuster und in dem dritten Aktivitätsmuster wird ferner eine Lautstärke-Information gewonnen, die anzeigt, ob die entsprechenden Trajektorien Schallereignissen geringer, hoher oder mittlerer Lautstärke zugeordnet sind. Die Lautstärke-Information wird dann bei der Filterung oder bei der Erzeugung der Information, ob Trajektorien einem Nutz-Schallereignis oder einem Stör-Schallereignis zugeordnet sind, berücksichtigt.

Es sei im Übrigen darauf hingewiesen, dass bei einem bevorzugten Ausführungsbeispiel sowohl die Lautstärke-Information als auch die Information über die zeitliche Verschiebung berücksichtigt werden. So kann beispielsweise das Filter (in Verbindung mit dem Bestimmer) ausgelegt sein, um das gefilterte Aktivitätsmuster derart zu erhalten, dass in dem gefilterten Aktivitätsmuster Trajektorien dominieren, deren zugehörige Lautstärke-Information innerhalb eines beispielsweise vorgegebenen Bereichs liegt, und deren zugehörige Zeitverschiebungs-Information innerhalb eines vorgegebenen Bereichs liegt. Somit kann das Filter (in Verbindung mit dem Bestimmer) ausgelegt sein, um beispielsweise das gefilterte Aktivitätsmuster so zu erzeugen, dass das gefilterte Aktivitätsmuster im Wesentlichen ein Audiosignal umfasst, das aus einem räumlich begrenzten Bereich stammt. Eine winkelmäßige Begrenzung des Bereichs besteht dabei durch die Beschränkung der zulässigen zeitlichen Verschiebung zwischen zwei zusammengehörigen Trajektorien. Eine entfernungsmäßige Beschränkung entsteht dabei durch eine Beschränkung der Lautstärke auf einen vorgegebenen Bereich.

In anderen Worten, die Schaltungsanordnung der Fig. 1 bzw. 2 (oder zumindest der Identifizierer 120 daraus) wird bei einem Ausführungsbeispiel dreifach repliziert. Somit entstehen drei parallele Zweige für drei Lautheitsräume HSR, MSR, LSR. Der Aufbau ist dabei strukturell gleich, es werden jedoch einmal Aktivitätsereignisse bzw. Aktivitätsmuster von Hörzellen (bzw. Spiralganglienzellen) mit hoher spontaner Emissionsrate, einmal Aktivitätsmuster von Hörzellen mit mittlerer spontaner Emissionsrate und einmal Aktivitätsmuster von Hörzellen mit geringer spontaner Emissionsrate verarbeitet.

Zusammenfassend lässt sich festhalten: bei einem Ausführungsbeispiel wird die Schaltung (z.B. der Identifizierer, der Bestimmer, die Multikoinzidenzeinheit 500 und/oder die Erkennungseinrichtung 700) dreifach aufgebaut für einen HSR-Raum, einen MSR-Raum und einen LSR-Raum.

Die vorliegende Erfindung umfasst somit die folgenden Aspekte:

a) Binaurale Vesikelfilterung, indem Verzögerungstrajektorien bzw. Delay-Trajektorien durch eine zeitliche Verschiebung gegeneinander gematcht werden;

b) Direkte Vesikelfilterung gegeneinander zum Bestimmen der Koinzidenzpaare;

c) Daraus resultierende Rauschunterdrückung, indem die Vesikel von Rauschquellen rausgerechelt werden;

d) Zeitgenaue Taktung und Synchronisation von linkem und rechtem Cochlea-Implantat, damit Schallquellen bezugsrichtig im Raum geortet werden können.

Gemäß einem weiteren Aspekt schafft die vorliegende Erfindung ein Verfahren und eine Vorrichtung gemäß den Aspekten a), b) und c) in allen drei HSR, MSR, LSR-Räumen (wobei unter einem HSR-Raum eine Mehrzahl von Hörzellen mit einer hohen spontanen Emissionsrate verstanden wird, wobei unter einem MSR-Raum eine Mehrzahl von Hörzellen mit einer mittleren spontanen Emissionsrate verstanden wird, und wobei unter einem LSR-Raum eine Mehrzahl von Hörzellen mit geringen spontanen Emissionsrate verstanden wird). Da alle drei Spiralganglienzellen (bzw. alle drei Typen von Spiralganglienzellen, d.h. HSR-Zellen, MSR-Zellen und LSR-Zellen) unterschiedliche Dynamikschwellen haben, können so leise Signalquellen durch Vergleich, wo die verschiedenen Typen von Zellen (HSR, MSR, LSR) zeitgleich Vesikel auslösen und/oder durch Bildung von Differenzpaaren laute und leise Schallquellen separiert werden.

In anderen Worten, das erfindungsgemäße System (beispielsweise gemäß den Fig. 1 und 2) ist bei einem Ausführungsbeispiel dreifach aufgebaut, wobei separate Signale bzw. Aktivitätsmuster aus Spiralganglienzellen mit hoher spontaner Emissionsrate, mittlerer spontaner Emissionsrate und geringer spontaner Emissionsrate (HSR, MSR, LSR) verwenden werden. Da die Spiralganglienzellen mit unterschiedlicher spontaner Emissionsrate unterschiedliche Ansprechpegelbereiche haben, sieht eine Vesikelausschüttung (bzw. allgemein: sehen Aktivitätsmuster), für laute, mittlere und leisere Töne unterschiedlich aus. Somit können unterschiedlich laute Signalquellen separiert werden.

Fig. 5a zeigt ein Schaltbild einer erfindungsgemäßen MultiKoinzidenzeinheit zur Verwendung in einer erfindungsgemäßen Vorrichtung, gemäß einem Ausführungsbeispiel der vorliegenden Erfindung. Die Schaltungsanordnung gemäß der Fig. 5a ist in ihrer Gesamtheit mit 500 bezeichnet. Die Schaltungsanordnung 500 ist ausgelegt, um eine erste Mehrzahl 510 von parallelen Signalen sowie eine zweite Mehrzahl 512 von parallelen Signalen zu empfangen. Es wird davon ausgegangen, dass die erste Mehrzahl 510 von parallelen Signalen beispielsweise ein Aktivitätsmuster beschreibt. In anderen Worten, Zeitverläufe der Signale der ersten Mehrzahl 510 von parallelen Signalen beschreiben ein zweidimensionales Muster (vergleiche Figuren 3a bis 3d). Das Gleiche gilt für die Signale der zweiten Mehrzahl 512 von parallelen Signalen.

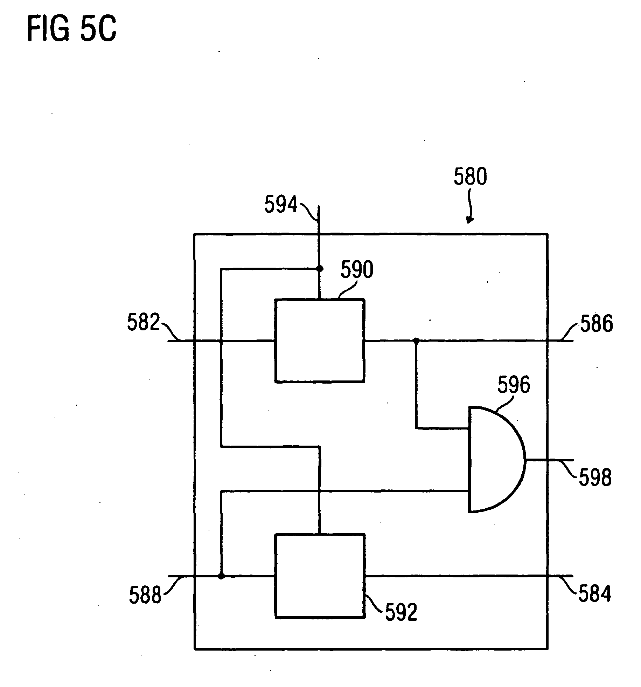

Die Schaltungsanordnung 500 umfasst ein Feld von Koinzidenz-Zellen. Ein Beispiel für eine einzelne Koinzidenz-Zelle ist in der Fig. 5c gezeigt. In anderen Worten, die Fig. 5c zeigt ein Schaltbild einer Koinzidenz-Zelle. Eine Koinzidenz-Zelle 580 weist einen ersten Dateneingang 582 und einen zweiten Dateneingang 584 auf. Die Koinzidenz-Zelle 580 weist ferner einen ersten Datenausgang 586 und einen zweiten Datenausgang 588 auf. Die Koinzidenz-Zelle 580 umfasst eine erste Speichereinrichtung 590 und eine zweite Speichereinrichtung 592. Die erste Speichereinrichtung bzw. Speicherzelle 590 und die zweite Speichereinrichtung bzw. Speicherzelle 592 sind bevorzugt ausgelegt, um ein gemeinsames (oder getrenntes) Taktsignal 594 zu empfangen, und um ansprechend auf das Taktsignal 594 eine Information von dem Eingang zu übernehmen und zu speichern. So ist beispielsweise die erste Speicherzelle 590 ausgelegt, um ansprechend auf das Taktsignal 594 eine Information (beispielsweise einen binären Wert) von dem ersten Eingang 582 zu übernehmen, zu speichern, und an den ersten Ausgang 586 auszugeben. Ferner ist bevorzugt die zweite Speicherzelle 592 ausgelegt, um ansprechend auf das Taktsignal 594 eine Information (z.B. einen binären Wert) von dem zweiten Eingang 584 zu übernehmen, zu speichern, und an dem zweiten Ausgang 588 auszugeben. Die Koinzidenz-Zelle 580 umfasst ferner ein UND-Gatter, das beispielsweise ausgelegt ist, um festzustellen, wenn beide Ausgänge 586, 588 der Koinzidenz-Zelle 580 einen aktiven Zustand aufweisen. Das UND-Gatter ist mit 596 bezeichnet und liefert ein Koinzidenz-Signal 598.

Die Multi-Koinzidenz-Einheit 500 besteht aus einer Mehrzahl von Koinzidenz-Zellen 580, deren Verschaltung im Folgenden beschrieben wird. Es sei darauf hingewiesen, dass die Multi-Koinzidenz-Einheit 500 in Form einer Matrix von Koinzidenz-Zellen 580 aufgebaut ist. Die Koinzidenz-Zellen werden im Folgenden mit zwei Indices i, j bezeichnet, wobei der Index i eine Zeile bezeichnet, und wobei der Index j eine Spalte bezeichnet. Es wird im Übrigen davon ausgegangen, dass die Multi-Koinzidenz-Einheit zumindest I Zeilen und zumindest J Spalten umfasst, wobei I ≥ 3 und J ≥ 3. Die einzelnen Koinzidenz-Zellen sind im Übrigen mit Z(i,j) bezeichnet.

Allgemein gilt damit: ein erster Ausgang der Koinzidenz-Zelle Z(i,j) ist mit einem ersten Eingang einer benachbarten Koinzidenz-Zelle Z(i, j+1) gekoppelt, wobei 1 ≤ j ≤ J-1. Ferner ist der zweite Eingang der Koinzidenz-Zelle Z(i,j) mit dem zweiten Ausgang der Koinzidenz-Zelle Z(i,j+1) gekoppelt, wobei 1 ≤ j ≤ J-1. Ein erster Eingang der Koinzidenz-Zelle Z(i,j=1) umfängt ein i-tes Signal aus einer ersten Mehrzahl 510 von parallelen Eingangssignalen. Ein zweiter Eingang der Koinzidenz-Zelle Z(i,j=J) umfängt ferner ein i-tes Signal der zweiten Mehrzahl 512 von parallelen Signalen.

In anderen Worten, die Koinzidenz-Zellen der i-ten Zeile sind ausgelegt, um das i-te Eingangssignal der ersten Mehrzahl 510 von parallelen Eingangssignalen schrittweise (ansprechend auf das Zeitsignal 594) in einer ersten Richtung (z.B. von links nach rechts) weiterzuleiten, und um das i-te Eingangssignal der zweiten Mehrzahl 512 von parallelen Eingangssignalen in einer zweiten Richtung (z.B. von rechts nach links) schrittweise weiterzuleiten.

Somit liegen an den Ausgängen 586, 588 der Koinzidenzzellen Z(i,j) die weitergeleiteten Signale an. Die Koinzidenzzellen sind dabei ausgelegt, um zu erkennen, wenn das weitergeleitete Signal der ersten Mehrzahl 510 von parallelen Signalen an dem ersten Ausgang 586 der betreffenden Koinzidenzzelle und das weitergeleitete Signal der zweiten Mehrzahl 512 von parallelen Signalen an dem zweiten Ausgang 588 der Koinzidenzzelle gleichzeitig aktiv sind. In diesem Fall gibt die betreffende Koinzidenzzelle ein Koinzidenzsignal an dem Ausgang 598 aus.

Die Multikoinzidenzeinheit 500 umfasst ferner Summierer 530. Dabei ist bevorzugt zumindest ein Summierer 530 einer Spalte der Multikoinzidenzeinheit 500 zugeordnet. Mit anderen Worten, ein j-te Summierer 530 ist ausgelegt, um die Koinzidenzsignale 598 der Koinzidenzzellen 580 der j-ten Spalte zu empfangen und zu summieren. Somit ist der j-te Summierer 530 ausgelegt, um zu bestimmen, an den Ausgängen wie vieler Koinzidenzzellen 580 der j-ten Spalte gleichzeitig oder innerhalb eines vorgegebenen Zeitintervalls Koinzidenzsignale 598 auftreten. Der Summierer 530 kann im Übrigen ausgelegt sein, um ein Rücksetz-Signal synchron oder asynchron zu empfangen. Mit anderen Worten, es können Zeitintervalle definiert sein, innerhalb derer eine Anzahl an Koinzidenzen in der j-ten Spalte der Multikoinzidenzeinheit 500 aufsummiert werden, um beispielsweise eine Trajektorie zu erkennen.

Die Multikoinzidenzeinheit 500 umfasst ferner Schwellwert-Entscheider 540, die ausgelegt sind, um ein zugeordnetes Summensignal von einem zugeordneten Summierer 530 zu empfangen. Die Schwellwert-Entscheider 540 sind damit ausgelegt, um zu erkennen, wenn innerhalb des Zeitintervalls, während dessen eine Summation erfolgt, innerhalb einer einzelnen Spalte j zumindest eine vorgegebene Anzahl an Koinzidenzen in den Koinzidenzzellen 580 der j-ten Spalte aufgetreten sind. In diesem Fall liefern die Schwellwert-Entscheider 540 ein zugehöriges Schwellwert-Signal 542.

Mit anderen Worten, die Koinzidenzeinheit 500 ist ausgelegt, um zu bestimmen, ob innerhalb eines vorgegebenen Zeitintervalls in einer j-ten Spalte zumindest eine vorgegebene Anzahl von Koinzidenzen aufgetreten sind, während zwei Aktivitätsmuster in entgegengesetzter Richtung durch die Multikoinzidenzeinheit 500 geschoben wurden. Unter einer Koinzidenz ist dabei, wie oben definiert, das gleichzeitige Vorliegen von zwei aktiven Signalen in einer Koinzidenzstufe 580 zu verstehen.

Es wird davon ausgegangen, dass durch die Koinzidenzeinheit 500 zwei Aktivitätsmuster in entgegengesetzten Richtungen geschoben werden, die Trajektorien enthalten. So tritt eine Koinzidenz auf, wenn in einer gleichen Koinzidenzzelle sowohl ein Aktivitätsereignis des ersten Aktivitätsmusters als auch ein Aktivitätsereignis des zweiten Aktivitätsmusters anliegen. Vereinfacht gesprochen, eine Koinzidenz tritt auf, wenn eine erste Trajektorie in dem ersten Aktivitätsmuster (das beispielsweise über die erste Mehrzahl 510 von parallelen Eingängen in die Multikoinzidenzeinheit 500 eingegeben wird) sich mit einer zweiten Trajektorie in dem zweiten Aktivitätsmuster (das beispielsweise über die zweite Mehrzahl 512 von parallelen Eingängen in die Multikoinzidenzeinheit 500 eingegeben wird) schneidet. Somit ist die Multikoinzidenzeinheit insgesamt ausgelegt, um festzustellen, an welchen Orten sich Trajektorien des ersten Aktivitätsmusters und Trajektorien des zweiten Aktivitätsmusters im Laufe einer Verschiebung der beiden Aktivitätsmuster schneiden.

Eine Möglichkeit der Auswertung der Ergebnisse der Multikoinzidenzeinheit 500 wird im Folgenden noch beschrieben.

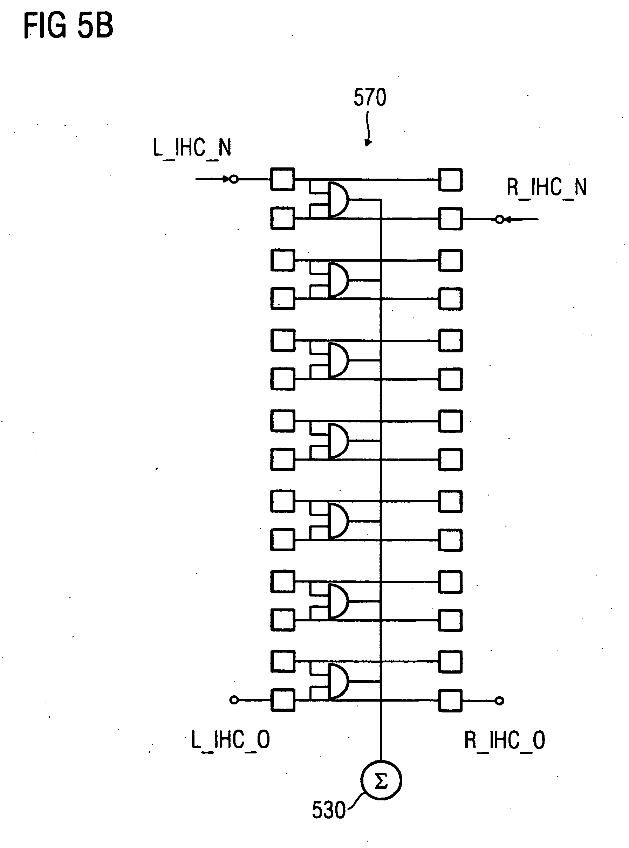

Fig. 5b zeigt im übrigen ein Schaltbild einer Spalte, wie sie beispielsweise in einer Multikoinzidenzeinheit 500 verwendet werden kann. Auf eine detaillierte Beschreibung wird hier verzichtet, da die in Fig. 5b gezeigte Spalte 570 von dem Aufbau her im Wesentlichen mit einer Spalte j von Koinzidenzzellen 580 übereinstimmt (vergl. Fig. 5a und 5c).

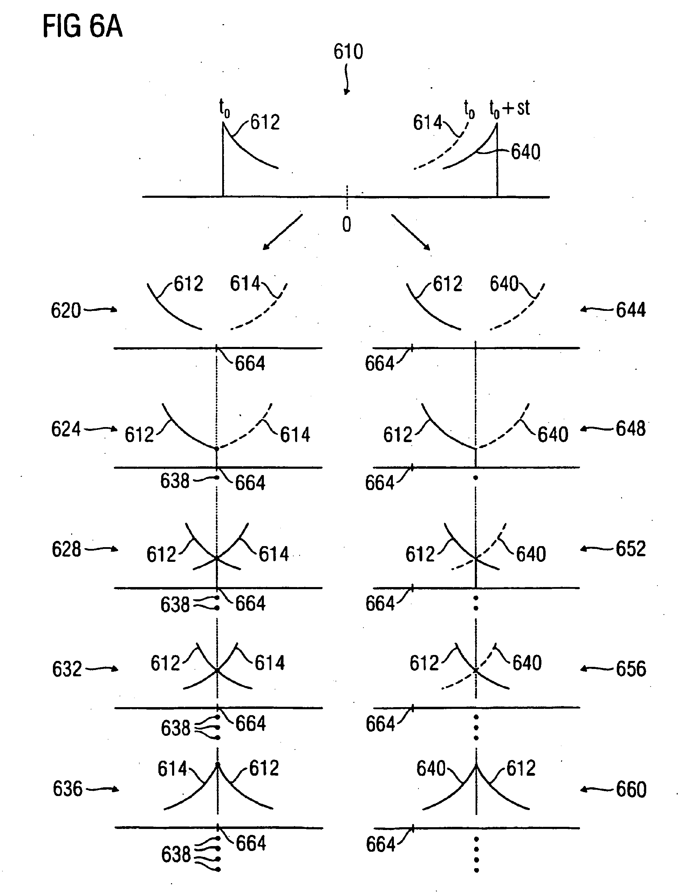

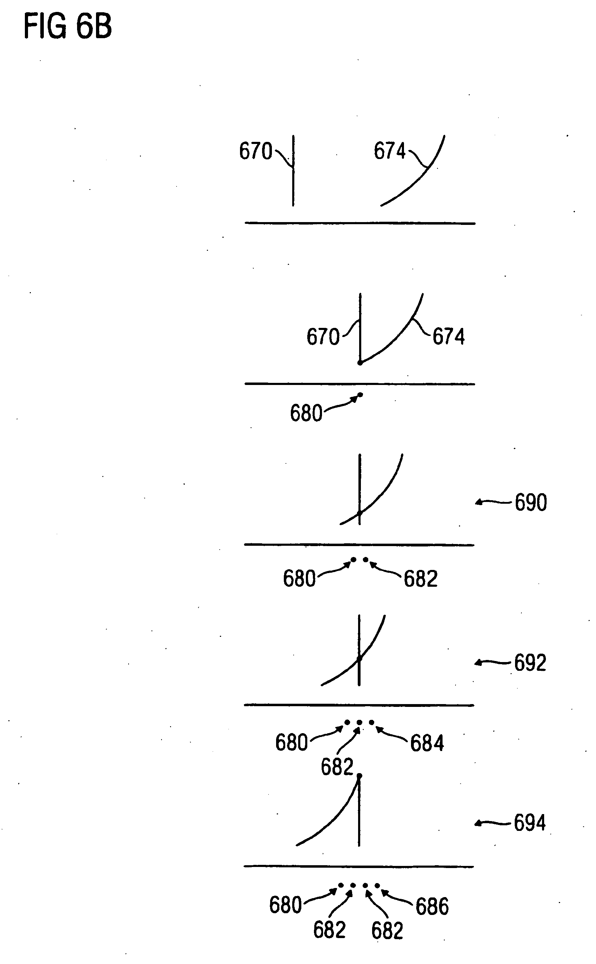

Fig. 6a zeigt eine schematische Darstellung der Vorgänge in der Multikoinzidenzeinheit 500, wenn durch die Multikoinzidenzeinheit 500 zwei Trajektorien eines ersten Aktivitätsmusters und eines zweiten Aktivitätsmusters hindurch geschoben werden. Es sei hier darauf hingewiesen, dass ein Durchschieben einer Trajektorie durch die Multikoinzidenzeinheit 500 in einer bildlichen Beschreibung einer Verschiebung der Trajektorie (beispielsweise um eine Spalte) entspricht. Es sei im Übrigen darauf hingewiesen, dass die in den Fig. 6a, 6b und 6c gezeigten Trajektorien im Rahmen einer Auswertung durch die Multikoinzidenzeinheit 500 durch eine Mehrzahl von parallelen Zeitsignalen repräsentiert werden. Die Fig. 6a zeigt in einer ersten graphischen Darstellung 610 eine erste Trajektorie 612, die beispielsweise in dem ersten Aktivitätsmuster enthalten ist, und von der angenommen wird, dass sie durch eine Mehrzahl von parallelen Signalen der ersten Mehrzahl 510 von parallelen Signalen in die Multikoinzidenzeinheit eingegeben wird. Die graphische Darstellung 610 zeigt ferner eine zweite Trajektorie 614 in dem zweiten Aktivitätsmuster. Es wird davon ausgegangen, dass die zweite Trajektorie 614 in dem zweiten Aktivitätsmuster durch eine Mehrzahl von parallelen Signalen der zweiten Mehrzahl 512 von parallelen binären Signalen in die Multikoinzidenzeinheit 500 eingegeben wird. Es wird ferner davon ausgegangen, dass die beiden Trajektorien 612, 614 eine gleiche Krümmung aufweisen und ferner gleichzeitig auftreten. Die graphischen Darstellungen 620, 624, 628, 632 zeigen eine schrittweise Verschiebung der Trajektorien 612, 614 durch die Stufen der Multikoinzidenzeinheit 500. Da davon ausgegangen wird, dass die Trajektorien 612, 614 eine gleiche Krümmung aufweisen und ferner gleichzeitig auftreten, kommt es im Laufe der Verschiebung der Trajektorien 612, 614 zu einer Koinzidenz jeweils in einer mittleren Spalte der Multikoinzidenzeinheit 500. Mit einem Fortschreiten einer Verschiebung der Trajektorien treten dabei allerdings Koinzidenzen in verschiedenen Zeilen der Multikoinzidenzeinheit 500 auf. Der zu der mittleren Spalte der Multikoinzidenzeinheit 500 gehörige Summierer 530 erhöht daher mit fortschreitender Verschiebung der Trajektorien 612, 614 seinen Zählerstand, wie dies durch dick gezeichnete Punkte 638 in den graphischen Darstellungen 624, 628, 632, 636 gezeigt ist.

Im Folgenden wird davon ausgegangen, dass in die Multikoinzidenzeinheit statt der Trajektorie 614 eine dazu zeitlich verzögerte Trajektorie 640 eingegeben wird. Da die Trajektorie 640 gegenüber der Trajektorie 612 zeitlich verzögert ist, ist die Trajektorie 612 bereits weiter durch die Multikoinzidenzeinheit verschoben als die Trajektorie 640, wenn es zu einer Koinzidenz zwischen der Trajektorie 612 und der Trajektorie 640 kommt. Der entsprechende Sachverhalt ist durch die graphischen Darstellungen 644, 648, 652, 656 und 660 veranschaulicht. Eine mittlere Spalte der Multikoinzidenzeinheit 500 ist im Übrigen mit 664 bezeichnet. In den graphischen Darstellungen 644, 648, 652, 656, 660 ist ersichtlich, dass es bei Vorliegen der gegenüber der Trajektorie 612 zeitlich verzögerten Trajektorie 640 zu einer Koinzidenz zwischen der verschobenen Trajektorie 612 und der verschobenen Trajektorie 640 kommt, wobei ein Ort der Koinzidenz die gegenüber der mittleren Spalte 664 der Multikoinzidenzeinheit 500 verschoben ist.

Mit anderen Worten, wird angenommen, dass eine erste Trajektorie, die der Multikoinzidenzeinheit 500 über die erste Mehrzahl 510 von parallelen Signalen zugeführt wird, und eine zweite Trajektorie 614, 640, die der Multikoinzidenzeinheit 500 über eine zweite Mehrzahl 512 von parallelen Signalen zugeführt wird, eine gleiche Krümmung aufweisen, so entscheidet eine zeitliche Verschiebung zwischen der ersten Trajektorie 612 und der zweiten Trajektorie 614, 640 darüber, in welcher Spalte j der Multikoinzidenzeinheit 500 eine Koinzidenz auftritt. Mit anderen Worten, eine Information über eine zeitliche Verschiebung zwischen einer ersten Trajektorie in dem ersten Aktivitätsmuster (das der Multikoinzidenzeinheit über die erste Mehrzahl 510 von parallelen Signalen zugeführt wird) und einer zweiten Trajektorie in dem zweiten Aktivitätsmuster (das der Multikoinzidenzeinheit 500 über die zweite Mehrzahl 512 von parallelen Signalen zugeführt wird) kann daraus abgeleitet werden, an welcher Spalte der Multikoinzidenzeinheit die Koinzidenz auftritt.

Fig. 6b zeigt eine schematische Darstellung der sich ergebenden Verhältnisse in der Multikoinzidenzeinheit 500, wenn die Multikoinzidenzeinheit 500 mit einer ersten Trajektorie 670, die in dem ersten Aktivitätsmuster enthalten ist und einer zweiten Trajektorie 674, die in dem zweiten Aktivitätsmuster enthalten ist, beaufschlagt wird, wobei davon ausgegangen wird, dass die erste Trajektorie 670 und die zweite Trajektorie 674 eine unterschiedliche Krümmung aufweisen. Bei dem gezeigten Extrembeispiel gemäß der Fig. 6b wird zur Veranschaulichung davon ausgegangen, dass die erste Trajektorie 670 ungekrümmt bzw. geradlinig ist. Werden die erste Trajektorie 670 und die zweite Trajektorie 674 wie beschrieben in entgegengesetzter Richtung durch die Multikoinzidenzeinheit 500 verschoben, so tritt eine erste Koinzidenz in einer Spalte der Multikoinzidenzeinheit 500 auf, die hier mit 680 bezeichnet ist. Wird die Trajektorie 670 weiter in der ersten Richtung verschoben, während hingegen die Trajektorie 674 weiter in der entgegengesetzten Richtung verschoben wird, so tritt eine nächste Koinzidenz nicht in der Spalte 680 sondern typischerweise in einer dazu benachbarten Spalte 682 der Multikoinzidenzeinheit 500 auf (vergl. graphische Darstellung 690). Nach einer nochmaligen Verschiebung der Trajektorien 670, 674 tritt schließlich eine Koinzidenz in einer Spalte 684 auf, die typischerweise der Spalte 682 benachbart ist (vergl. graphische Darstellung 692). Eine weitere Fortsetzung der Verschiebung ist in der graphischen Darstellung 694 gezeigt, wobei sich eine Koinzidenz in einer wiederum weiteren Spalte 686 ergibt.

Mit anderen Worten, die Multikoinzidenzeinheit 500 ist so ausgelegt, dass Koinzidenzen in jeweils der gleichen Spalte auftreten, wenn Trajektorien gleicher Krümmung in entgegengesetzter Richtung durch die Multikoinzidenzeinheit 500 schrittweise verschoben werden. Werden hingegen Trajektorien unterschiedlicher Krümmung schrittweise durch die Multikoinzidenzeinheit 500 verschoben, so treten dabei Koinzidenzen in verschiedenen Spalten auf.

Bei einem Ausführungsbeispiel ist der Schwellwert der Schwellwert-Entscheider 540 so eingestellt, dass die Schwellwert-Entscheider 540 nur dann ansprechen (bzw. ein aktives Ausgangssignal ausgeben), wenn Trajektorien einer gleichen Krümmung (oder mit verschiedenen Krümmungen, die höchstens um eine vorgegebene maximale Abweichung voneinander abweichen) in entgegengesetzter Richtung durch die Multikoinzidenzeinheit 500 geschoben werden.

Mit anderen Worten, die Multikoinzidenzeinheit 500 ist so ausgelegt, dass das Auftreten eines aktiven Ausgangssignals an einem beliebigen der Schwellwert-Entscheider 540 anzeigt, dass in dem ersten Aktivitätsmuster und dem zweiten Aktivitätsmuster zwei Trajektorien zumindest näherungsweise (innerhalb eines vorgegebenen Toleranzbereichs) gleicher Krümmung vorliegen. Aus der Tatsache, welcher der Schwellwert-Entscheider 540 ein aktives Signal an seinem Ausgang abgibt, ergibt sich ferner eine Information über eine zeitliche Verschiebung zwischen den Trajektorien gleicher Krümmung.

Es sei allerdings darauf hingewiesen, dass die Multikoinzidenzeinheit 500 durch jede andere Einrichtung ersetzt werden kann, die in der Lage ist, in einem ersten Aktivitätsmuster und in einem zweiten Aktivitätsmuster Trajektorien (bzw. linienförmige geometrische Gebilde) zu erkennen, die zumindest näherungsweise (d.h. beispielsweise innerhalb eines Toleranzbereichs) gleiche Krümmungen aufweisen, um eine zeitliche Verschiebung zwischen den Trajektorien gleicher oder näherungsweise gleicher Krümmung zu bestimmen. Ferner ist es möglich, neben der Krümmung der Trajektorien in dem ersten Aktivitätsmuster und in dem zweiten Aktivitätsmuster auch eine Länge der Trajektorien in dem ersten Aktivitätsmuster und in dem zweiten Aktivitätsmuster heranzuziehen, um zu entscheiden, ob eine erste Trajektorie in dem ersten Aktivitätsmuster und eine zweite Trajektorie in dem zweiten Aktivitätsmuster einem gleichen Schallereignis zugeordnet sind.

Fig. 7 zeigt ein Blockschaltbild eines erfindungsgemäßen Identifizierers gemäß einem Ausführungsbeispiel der vorliegenden Erfindung. Der Identifizierer gemäß der Fig. 7 ist in seiner Gesamtheit mit 700 bezeichnet. Der Identifizierer 700 ist ausgelegt, um ein erstes Aktivitätsmuster in Form einer ersten Mehrzahl 710 von parallelen Signalen bzw. parallelen Informationen zu empfangen. Ferner ist der Identifizierer 700 ausgelegt, um ein zweites Aktivitätsmuster in Form einer zweiten Mehrzahl 720 von parallelen Signalen zu empfangen. Die Beschreibung der Aktivitätsmuster durch die parallelen Signale 710, 720 kann dabei beispielsweise durch binäre Signale erfolgen, wobei aktive Zustände das Auftreten eines Aktivitätsereignisses kennzeichnen.

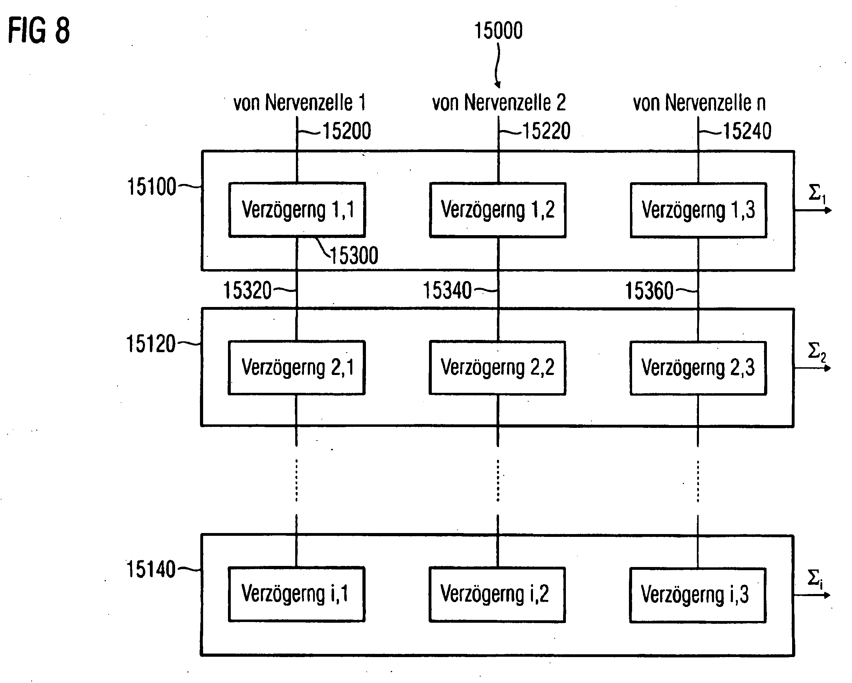

Der Identifizierer 700 umfasst einen ersten Trajektorien-Erkenner 730 und einen zweiten Trajektorien-Erkenner 732. Der erste Trajektorien-Erkennung 730 ist ausgelegt, um das erste Aktivitätsmuster über die erste Mehrzahl 710 von parallelen Signalen zu empfangen und um in dem ersten Aktivitätsmuster Trajektorien zu erkennen. Beispielsweise ist der erste Trajektorien-Erkenner 730 ausgelegt, um in dem ersten Aktivitätsmuster linienförmige Strukturen zu erkennen, die eine bestimmte vorgegebene Krümmung aufweisen. Beispielsweise kann der Trajektorien-Erkenner ausgelegt sein, linienförmige Strukturen mit einer Mehrzahl von verschiedenen vorgegebenen Krümmungen zu erkennen. Ein Beispiel für einen Trajektorien-Erkenner wird im Übrigen später noch anhand der Fig. 8, 9 und 10 beschrieben. In analoger Weise ist der zweite Trajektorien-Erkenner 732 ausgelegt, um das zweite Aktivitätsmuster über die zweite Mehrzahl 720 von parallelen Signalen zu empfangen. Der zweite Trajektorien-Erkenner 732 ist dabei beispielsweise ausgelegt, um Trajektorien verschiedener Krümmung zu erkennen.

Beispielsweise ist der erste Trajektorien-Erkenner 730 ausgelegt, um basierend auf dem ersten Aktivitätsmuster zumindest eine Ausgangsleitung zu aktivieren, so dass die Tatsache, welche der mehreren Ausgangsleitungen aktiviert wird, eine Information über eine Krümmung einer in dem Aktivitätsmuster identifizierten Trajektorie umfasst. Mit anderen Worten, erkennt der Trajektorien-Erkenner 730 eine Trajektorie in dem ersten Aktivitätsmuster, so aktiviert der Trajektorien-Erkenner bevorzugt eine (möglicherweise aber auch mehr als eine) seiner Ausgangsleitungen 740, 742, 744, 746 in Abhängigkeit von der Krümmung der Trajektorie. Ferner ist beispielsweise der zweite Trajektorien-Erkenner 732 ausgelegt, um in Abhängigkeit von einer Krümmung einer in dem zweiten Aktivitätsmuster erkannten Trajektorie zumindest eine (bevorzugt genau eine, möglicherweise aber auch mehrere) seiner Ausgangsleitungen 750, 752, 754, 756 zu aktivieren. Dabei sind der erste Trajektorien-Erkenner 730 und der zweite Trajektorien-Erkenner 732 so ausgelegt, dass korrespondierende Ausgangsleitungen der beiden Trajektorien-Erkenner 730, 732 (innerhalb eines Toleranzbereichs) gleiche Krümmungen von Trajektorien anzeigen. Mit anderen Worten, ein i-tes Ausgangssignal des ersten Trajektorien-Erkenners 730 zeigt ein Vorliegen einer Trajektorie mit einer bestimmten i-ten Krümmung in dem ersten Aktivitätsmuster an, und ein i-tes Ausgangssignal des zweiten Trajektorien-Erkenners 732 zeigt ein Vorliegen einer Trajektorie mit der gleichen i-ten Krümmung in dem zweiten Aktivitätsmuster an.

Der Identifizierer 700 umfasst ferner einen Koinzidenz-Erkenner 770. Der Koinzidenz-Erkenner 770 entspricht von seinem Aufbau her beispielsweise der Multikoinzidenzeinheit 500. Der Koinzidenz-Erkenner 700 empfängt beispielsweise als Eingangssignale an der ersten Mehrzahl 510 von Eingänge, die Ausgangssignale 740, 742, 744, 746 des ersten Trajektorien-Erkenners 730. Der Koinzidenz-Erkenner 770 empfängt ferner bevorzugt an den Eingängen der zweiten Mehrzahl 512 von Eingängen die Ausgangssignale 750, 752, 754, 756 des zweiten Trajektorien-Erkenners 732. Es wird dabei davon ausgegangen, dass der i-ten Zeile der Multikoinzidenzeinheit 500 das i-te Ausgangssignal des ersten Trajektorien-Erkenners 730 und das i-te Ausgangssignal des zweiten Trajektorien-Erkenners 732 zugeführt werden. Erkennen somit der erste Trajektorien-Erkenner 730 und der zweite Trajektorien-Erkenner 732 Trajektorien gleicher Krümmung, so aktivieren der erste Trajektorien-Erkenner 730 und der zweite Trajektorien-Erkenner 732 korrespondierende Ausgangssignale (beispielsweise das jeweilige i-te Ausgangssignal). Somit tritt beispielsweise in der i-ten Zeile der Multikoinzidenzeinheit 500 (die den Koinzidenz-Erkenner 770 bildet) eine Koinzidenz auf. Die Tatsache, in welcher Spalte j der Multikoinzidenzeinheit 500 die Koinzidenz auftritt, stellt wiederum ein Maß dafür da, wie groß eine zeitliche Verschiebung zwischen den Trajektorien gleicher Krümmung ist.

Mit anderen Worten, ein bloßes Auftreten einer Koinzidenz in einer Koinzidenzzelle der Multikoinzidenzeinheit 500 zeigt bei dem Identifizierer 700 bereits das Vorliegen von Trajektorien gleicher Krümmung an. Daher können beispielsweise bei einem Einsatz der Multikoinzidenzeinheit 500 als Koinzidenz-Erkenner 770 die Schwellwert-Entscheider 540 und auch die Summierer 530 optional entfallen. Der Koinzidenz-Erkenner 770 liefert somit eine Mehrzahl von bevorzugt parallelen Ausgangssignalen, die anzeigen, mit welcher zeitlichen Verschiebung Trajektorien gleicher Krümmung auftreten. Die Ausgangssignale des Koinzidenz-Erkenners 770 sind im Übrigen mit 780 bezeichnet.

Somit stellt die Schaltungsanordnung 700 eine weitere Alternative dar, um basierend auf zwei Aktivitätsmustern festzustellen, ob in den beiden Aktivitätsmustern Trajektorien gleicher Krümmung (also Trajektorien, die gleichen Schallereignissen zugeordnet sind) vorhanden sind, und falls ja, welche zeitliche Verschiebung diese Trajektorien aufweisen.

Es sei im Übrigen darauf hingewiesen, dass bei einem bevorzugten Ausführungsbeispiel bei Einsatz der Multikoinzidenzeinheit 500 an der Stelle des Koinzidenz-Erkenners 770 die Summierer 530 durch eine Oder-Verknüpfung ersetzt werden können, so dass die Ausgangssignale der Oder-Verknüpfungen die Ausgangssignale 780 des Koinzidenz-Erkenners 770 bzw. des Identifizierers 700 bilden.

Im Folgenden werden anhand der Fig. 8, 9 und 10 verschiedene Vorrichtungen beschrieben, die beispielsweise als Trajektorienerkenner eingesetzt werden können, und die somit bei bestimmten Ausführungsbeispielen der vorliegenden Erfindung an die Stelle der Trajektorienerkenner 730, 732 treten können.

Figur 8 zeigt ein Blockschaltbild einer Vorrichtung zum erfindungsgemäßen Verarbeiten des Nervenaktivitätsmusters. Die in der Figur 8 gezeigten Vorrichtung ist in ihrer Gesamtheit mit 15000 bezeichnet. Die gezeigte Vorrichtung 15000 weist eine Mehrzahl von Stufen 15100, 15120, 15140 auf, wobei die erste Stufe 15100 parallel Signale 15200, 15220, 15240 von Nervenzellen empfängt. Die Signale 15200, 15220, 15240 beschreiben bevorzugt ein Aktivitätsmuster (z.B. Aktionspotentiale auf Nervenfasern, die mit den entsprechenden Nervenzellen bzw. inneren Haarzellen gekoppelt sind, oder Neurotransmitter-Vesikel-Auftreten an einer Mehrzahl von inneren Hörzellen). Die Signale 15200, 15220, 15240 beschreiben somit beispielsweise das Nervenaktivitätsmuster. Die Signale können aber auch ein anderes Aktivitätsmuster, beispielsweise ein Neurotransmitter-Vesikel-Auftreten in einer Mehrzahl von inneren Haarzellen beschreiben.

In einer ersten Stufe 15100 wird dann beispielsweise das erste Signal bzw. Nervensignal 15200 in einer ersten Verzögerungseinrichtung 15300 einer Verzögerung unterworfen und dann als verzögertes Signal bzw. Nervensignal 15320 an eine zweite Stufe 15120 weitergeleitet. In ähnlicher Weise wird auch das zweite Signal bzw. Nervensignal 15220 in der ersten Stufe 15100 verzögert und als verzögertes Signal Nervensignal an die zweite Stufe 15120 weitergeleitet. In der gleichen Weise werden auch die übrigen Signale bzw. Nervensignale in der ersten Stufe 15100 verarbeitet (also beispielsweise auch das n-te Signal bzw. Nervensignal 15240). Die zweite Stufe 15120 ist parallel zu der ersten Stufe 15100 ausgelegt, ermöglicht also wiederum die verzögerte Weiterleitung der verzögerten Signale bzw. Nervensignale 15320, 15340, 1536, wodurch zweimal verzögerte Signale bzw. Nervensignale entstehen. Eine Vorrichtung zur erfindungsgemäßen Verarbeitung des Aktivitätsmusters umfasst eine Mehrzahl von hintereinander geschalteten Stufen, die gleich wie die erste Stufe 15100 bzw. die zweite Stufe 15120 aufgebaut sind. Die Signale bzw. Nervensignale 15200, 15220, 15240 werden also parallel durch die Mehrzahl von Stufen 15100, 15120, 15140 weitergeleitet, wobei jede Stufe eine einstellbare Verzögerung zu den Signalen bzw. Nervensignalen hinzufügt.

Weiterhin ist jede der Stufen 15100, 15120, 15140 ausgelegt, um eine Summe der an ihr einlaufenden bzw. der aus ihr auslaufenden Signale bzw. Nervensignale (bzw. m-mal verzögerten Nervensignale) zu bilden. Ferner sind die Stufen 15100, 15120, 15140 bevorzugt ausgelegt, um diese Summe mit einem einstellbaren Schwellwert zu vergleichen, um festzustellen, ob zu einem gegebenem Zeitpunkt mindestens eine vorgegebene Anzahl von Signalen bzw. Nervensignalen bzw. verzögerten Signalen bzw. Nervensignalen (also einlaufende Signale bzw. Nervensignale oder auslaufende Signale bzw. Nervensignale) aktiv sind (bzw. ein Aktionspotential aufweisen).

Es wird ferner bevorzugt, dass die Verzögerungen der in den Stufen 15100, 15120, 15140 vorhandenen Verzögerungseinrichtungen unterschiedlich eingestellt sind, sodass beispielsweise ein erstes Signal bzw. Nervensignal 15200 bei einem Durchlaufen der Stufen 15100, 15120, 15140 einer anderen Verzögerung unterliegt als das zweite Signal bzw. Nervensignal 15220. Verzögerungen können beispielsweise so eingestellt sein, dass sich für die Signale bzw. Nervensignale 15200, 15220, 15240 unterschiedliche Gesamtverzögerungen beim Durchlaufen durch die Stufen 15100, 15120, 15140 ergeben (wobei es freilich zulässig ist, dass beispielsweise zwei Signale bzw. Nervensignale in der gleichen Weise verzögert werden). In anderen Worten, die Einrichtung 15000 ist bevorzugt so ausgelegt, das sich nicht für alle Signale bzw. Nervensignale die gleichen Verzögerungen ergeben. Außerdem ist es vorteilhaft, dass bei Vorhandensein von j Stufen 15100, 15120, 15140 mindestens (j-1) Stufen 15100, 15120 so ausgelegt sind, dass die in einer Stufe enthaltenen Verzögerungseinrichtungen für die Mehrzahl von Signalen nicht alle die gleiche Verzögerung aufweisen. Dadurch kann erreicht werden, dass ein in eine erfindungsgemäße Einrichtung 15000 einlaufendes Aktivitätsmuster über der Zeit beim Durchlauf durch die beschriebene Einrichtung zeitlich so verzerrt wird, dass einzelne Signale bzw. Nervensignale gegenüber anderen Signalen bzw. Nervenssignalen zeitlich verschoben werden. Durch die Verzerrung können in einer zeitlichen Darstellung gebogene linienartige Muster, also Trajektorien, in dem Aktivitätsmuster gerade gebogen werden.

Ferner wird darauf hingewiesen, dass durch die Summenbildung innerhalb einer Stufe erkannt werden kann, wenn eine ursprünglich gebogene Trajektorie in dem Aktivitätsmuster zu einer geraden Linie verbogen wurde (wobei eine geradegebogene Linie dadurch beschrieben bzw. erkannt wird, dass eine vorgegebene Anzahl der verzögerten Signale bzw. Nervensignale nahezu gleichzeitig bzw. zeitlich überlappend einen aktiven Zustand oder ein Aktionspotential aufweisen).

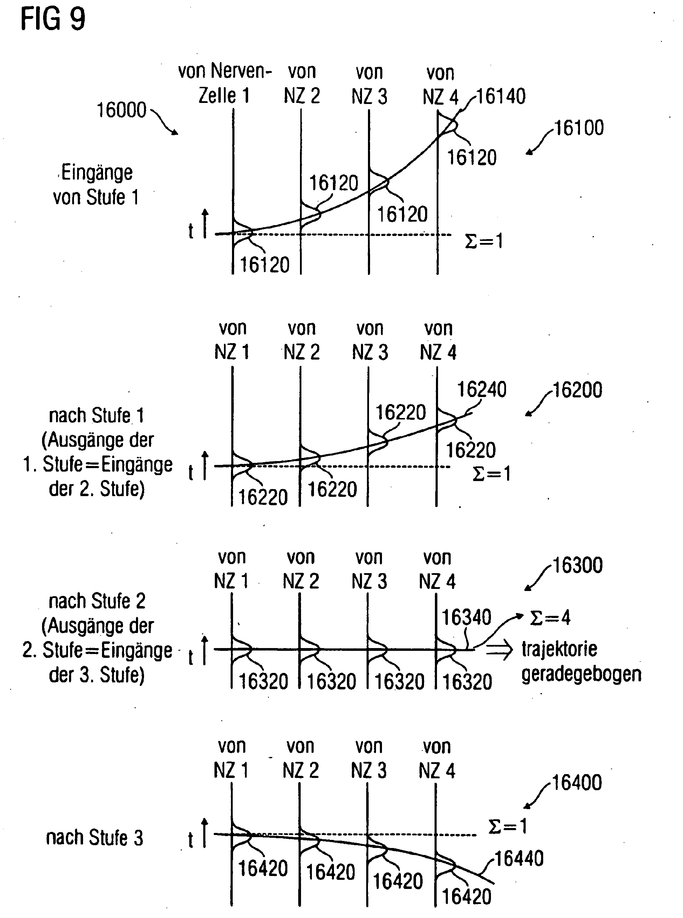

Die Funktionsweise der Einrichtung 1500 soll anhand der Figur 9 veranschaulicht werden. Figur 9 zeigt eine beispielhafte grafische Darstellung der Signale in einer Vorrichtung 15000 zum erfindungsgemäßen Verarbeiten des Aktivitätsmusters. Die grafische Darstellung der Figur 9 ist in ihrer Gesamtheit mit 16000 bezeichnet.

Eine erste graphische Darstellung 16100 beschreibt hierbei ein beispielhaftes Aktivitätsmuster an Eingängen der Vorrichtung 15000. Gezeigt sind beispielhaft die Signale von vier Nervenzellen (bzw. auf vier Nervenfasern) in einem zeitlichen Verlauf. Im übrigen wird darauf hingewiesen, dass die Aktionspotentiale 16120 eine Trajektorie 16140 bilden. Wie gezeigt, weist die Trajektorie 16140 in der zeitlichen Darstellung eine starke Krümmung auf, da die Aktionspotentiale 16120 von den verschiedenen Nervenfasern an den Eingängen der ersten Stufe 15100 einen deutlichen zeitlichen Versatz aufweisen. Somit liegt in der ersten Stufe 15100 zu einem festen Zeitpunkt nur jeweils ein Aktionspotential vor, sodass ein Schwellwert für eine Summe der an der ersten Stufe anliegenden Aktionspotentiale, der beispielsweise zu zwei gesetzt ist, nicht überschritten wird. Folglich liefert die erste Stufe kein Ausgangssignal an einem Schwellwertausgang.

Eine zweite grafische Darstellung 16200 beschreibt die Verhältnisse an einem Ausgang der ersten Stufe 15100. Hierbei wird davon ausgegangen, dass in der ersten Stufe 15100 das von der erste Nervenzelle NZ 1 gelieferte Nervensignal stärker verzögert wird als die von den anderen Stufen gelieferten Nervensignale. Im übrigen wird davon ausgegangen, dass bei dem gegebenem Beispiel das von der vierten Nervenzelle NZ4 gelieferte Nervensignal am wenigsten verzögert wird, während das Nervensignal von der dritten Nervenzelle NZ3 etwas mehr verzögert wird, und wobei die Verzögerung für Nervensignalen von den Nervenzellen NZ2 und NZ1 immer stärker ansteigt. Allgemein gesprochen werden Signale, die zu Nervenzellen gehören, die auf eine niedrigere Frequenz ansprechen, weniger stark verzögert als Nervensignale von Nervenzellen, die höhere Frequenzen detektieren.

Die zweite grafische Darstellung zeigt somit wiederum Aktionspotentiale 16240 als Funktion der Zeit, wobei die Aktionspotentiale 16220 eine Trajektorie 16240 bilden. Wie aus der zweiten grafischen Darstellung 16200 ersichtlich ist, ist die Krümmung der Trajektorie 16240 an den Ausgängen der ersten Stufe geringer als eine (zeitlich-örtliche bzw. zeitlich-frequenzmäßige) Krümmung der Trajektorie 16160 an den Eingängen der ersten Stufe. Dies resultiert aus der unterschiedlichen Verzögerung der zu verschiedenen Nervenzellen gehörigen Nervensignale in den Verzögerungseinrichtungen (z.B. 15300) der ersten Stufe. Dadurch wird also eine gekrümmte Trajektorie gleichsam geradegebogen. Wie aus der zweiten grafischen Darstellung 16200 ersichtlich, weist die zweite Trajektorie 16240 allerdings noch eine Restkrümmung auf, so dass die von verschiedenen Nervenzellen bzw. Nervenfasern stammenden Aktionspotentiale 16220 nicht alle gleichzeitig an den Ausgängen der ersten Stufe 15100 bzw. Eingängen der zweiten Stufe 15120 anliegen.

Auch die zweite Stufe 15120 bewirkt eine weitere Verzögerung, wobei wiederum Signale von Nervenzellen, die für niedrige Frequenzen empfindlich sind, weniger verzögert werden als Signale von Nervenzellen, die für höhere Frequenzen empfindlich sind. Eine dritte grafische Darstellung 1630 zeigt die in der zweiten Stufe 15120 nochmals verzögerten Nervensignale an Ausgängen der zweiten Stufe. Es ist aus der dritten graphischen Darstellung 16300 ersichtlich, dass bei dem vorliegenden Beispiel die Nervensignale an den Ausgängen der zweiten Stufe jeweils so verzögert sind, dass Aktionspotentiale 16320 von mehreren Nervenzellen an den Ausgängen der zweiten Stufe gleichzeitig anliegen. In anderen Worten, eine Trajektorie 16340, die durch die Aktionspotentiale 16320 beschrieben wird, ist zumindest näherungsweise gerade gebogen. Die Aktionspotentiale 16320 treten also gleichzeitig bzw. näherungsweise gleichzeitig (zumindest aber zeitlich überlappend) auf, sodass das gleichzeitige Auftreten durch eine Summation der an den Ausgängen der zweiten Stufe (bzw. Eingängen der dritten Stufe) anliegenden Signale einen deutlichen Peak aufweist, der groß genug ist, um einen vorgegebenen Schwellenwert (z. B. zwei oder drei) zu überschreiten.

In anderen Worten, es kann durch eine geeignete Summiereinrichtung (oder eine andere geeignete Einrichtung) erkannt werden, wann eine gekrümmte Trajektorie gerade gebogen wurde. Die entsprechende Information ermöglicht einen Rückschluss sowohl auf den Anfangszeitpunkt der Trajektorie als auch auf die Form der Trajektorie. Es kann nämlich festgestellt werden, nach Durchlaufen von wie vielen Stufe eine Trajektorie gerade gebogen wurde. Dadurch kann bei Kenntnis der Verzögerungen für die einzelnen Nervensignale in den Stufen der Einrichtung 15000 auch auf eine ursprüngliche Form der Trajektorie zurückgeschlossen werden. Ferner ist die Durchlaufzeit für die Stufen bevorzugterweise bekannt, sodass auch der Zeitpunkt, zu dem eine Trajektorie in die Einrichtung 15000 eingelaufen ist, bestimmt werden kann. Somit kann sowohl eine charakteristische Zeitinformationen der Trajektorien als auch Informationen über Form bzw. Krümmung der Trajektorien ermittelt werden, um zu bestimmen, welche Aktivitätsereignisse zu einer Trajektorie gehören und/oder welche Aktivitätsereignisse nicht zu einer Trajektorie gehören.

Im übrigen wird noch darauf hingewiesen, dass eine vierte grafische Darstellung 16400 zur Verbesserung des Verständnisses noch Ausgangssignale an Ausgängen einer dritten Stufe zeigt. Aktionspotentiale 16420 beschreiben eine Trajektorie 16440, die allerdings durch ein weiteres Verbiegen der Trajektorie wieder gekrümmt ist.

Es wird darauf hingewiesen, dass die Verzögerungen in den Stufen 15100, 15120, 15160 auf verschiedenem Weg erzielt werden können. Die Verzögerungseinrichtungen (z.B. 15300) können beispielsweise getaktet sein, und/oder es kann sich um kontinuierlich oder diskret einstellbare Verzögerungseinrichtungen handeln. Im übrigen ist es auch möglich, dass eine oder mehrere Verzögerungseinrichtungen in einer vorgegebenen Stufe für eine oder mehrere Nervensignale deaktiviert sind, sodass einige Nervensignale durch eine Stufe mit geringst möglicher Verzögerung weitergeleitet werden. Im übrigen ist festzuhalten, dass die Einrichtung 15000 insgesamt als eine analoge oder digitale Schaltung implementiert sein kann.

Ferner sei darauf hingewiesen, dass oben eine Auswertung eines Nervenaktivitätsmusters beschrieben wurde. Die Vorrichtung 15000 kann allerdings für ein Auffinden von Trajektorien in beliebigen Aktivitätsmustern herangezogen werden.

Ferner wird darauf hingewiesen, dass die Signale 15200, 15220, 15240 beispielsweise den Signalen 710 bzw. 720 entsprechen. Die Schwellwert-bewerteten Summensignale $\Sigma_1$, $\Sigma_2$, $\Sigma_i$ (die durch Vergleich der Summensignale mit einem Schwellwert entstehen) entsprechen im übrigen den Signalen 740, 742, 744, 746 bzw. 750, 752, 754, 756.

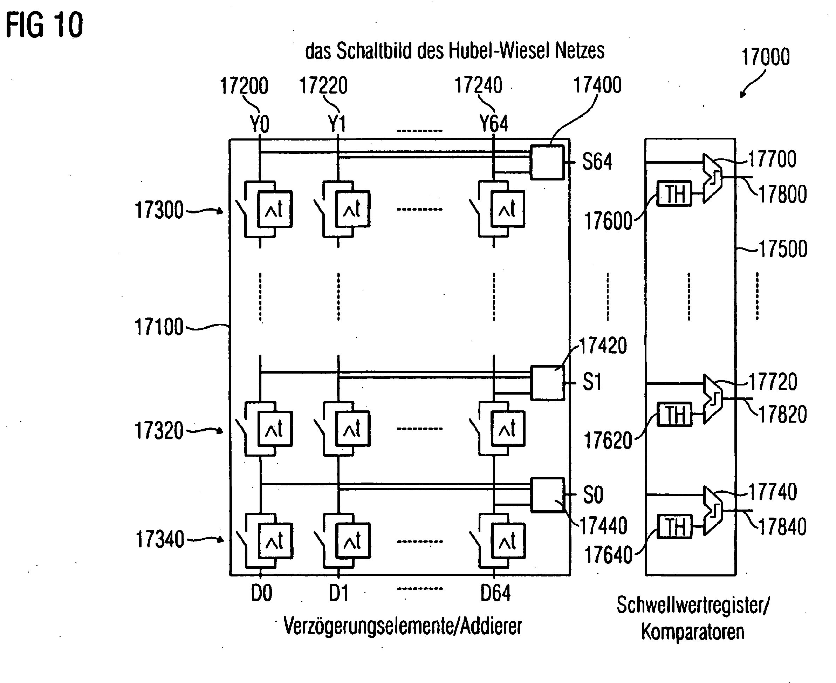

Figur 10 zeigt ein Schaltbild eines beispielhaften Hubel-Wiesel-Netzes zur erfindungsgemäßen Berechnung einer Analysedarstellung eines Audiosignals gemäß dem zweiten Ausführungsbeispiel der vorliegenden Erfindung. Das Schaltbild der Figur 17 ist in seiner Gesamtheit mit 17000 bezeichnet. Ein erster Schaltungsblock 17100 empfängt Eingangssignale 17200, 17220, 17240, die beispielsweise ein Nervenaktivitätsmuster, ein Anregungsmuster einer Basilarmembran oder ein Neurotransmitter-Vesikel-Auftreten repräsentieren können. Die Eingangssignale 17200, 17220, 17240 werden dann durch eine Mehrzahl von Stufen 17300, 17320, 17340 geleitet. Ein Eingangssignal 17200 durchläuft somit eine Mehrzahl von Stufen 17300, 17320, 17340, wobei ein Eingangssignal 17200 in einer Stufe 17300, 17320, 17340 entweder eine Verzögerungseinrichtung durchläuft oder direkt zu einer darauffolgenden Stufe weitergeleitet wird. In anderen Worten, die Verzögerungseinrichtungen können auch überbrückt werden.

In anderen Worten, jede Stufe umfasst für jedes Signal eine schaltbare Verzögerungseinrichtung, wobei die Verzögerungseinrichtung in einen Signalpfad, der von einem Eingangssignal durchlaufen wird, eingeschaltet werden kann oder überbrückt werden kann. Signale an den Eingängen jeder Stufe werden abgegriffen und Summierern 17400, 17420, 17440 zugeführt, wobei jeweils die an den Eingängen einer Stufe anliegenden Signale aufsummiert werden. Der erste Schaltungsblock 17100 bildet somit ein Gitter von Verzögerungselementen und Addieren, die in der gezeigten Weise verschaltet sind.

Das Hubel-Wiesel-Netz 17000 weist ferner eine Schwellwerteinrichtung 17500 auf, wobei jeweils ein Wert aus einem Schwellwertregister 17600, 17620, 17640 sowie ein Ausgang eines Summierers 17400, 17430, 17440 einem Komparator 17700, 17720, 17720 zugeführt wird. Ausgangsignale 17800, 17820, 17840 der Komparatoren 17700, 17720, 17740 liefern dabei eine Aussage darüber, ob an den Eingängen einer vorgegebenen Stufe 17300, 17320, 17340 eine Anzahl von Signalen gleichzeitig aktiv sind, wobei eine Mindestanzahl, bei der ein aktives Ausgangssignal 17800, 17820, 17840 ausgegeben wird, durch die Schwellwertregister 17600, 17620, 17640 festgelegt ist. In anderen Worten, durch die Komparatoren 17700, 17720, 17740 kann in Verbindung mit den Summierern 17400, 17420, 17440 und den Schwellwertregistern 17600, 17620, 17640 festgestellt werden, wenn (bzw. nach Durchlaufen wie vieler der Stufen 17300, 17320, 17340) eine Trajektorie, die über die Eingänge 17200, 17220, 17240 des ersten Blocks 17100 eingelesen wurde, gerade gebogen ist.

Die Verzögerungen der einzelnen Stufen 17300, 17320, 17340 können dabei geeignet vorgegeben werden, um eine Erkennung einer möglichst großen Anzahl von Trajektorien (bzw. Trajektorien-Formen) zu ermöglichen.

Die Eingangssignale 17200, 17220, 17240 entsprechen beispielsweise den Signalen 710, 720 gemäß Fig. 7, während die Ausgangssignale 17800, 17820, 17840 beispielsweise den Signlen 740, 724, 744, 746 bzw. den Signalen 750, 752, 754, 756 gemäß Fig. 7 entsprechen.

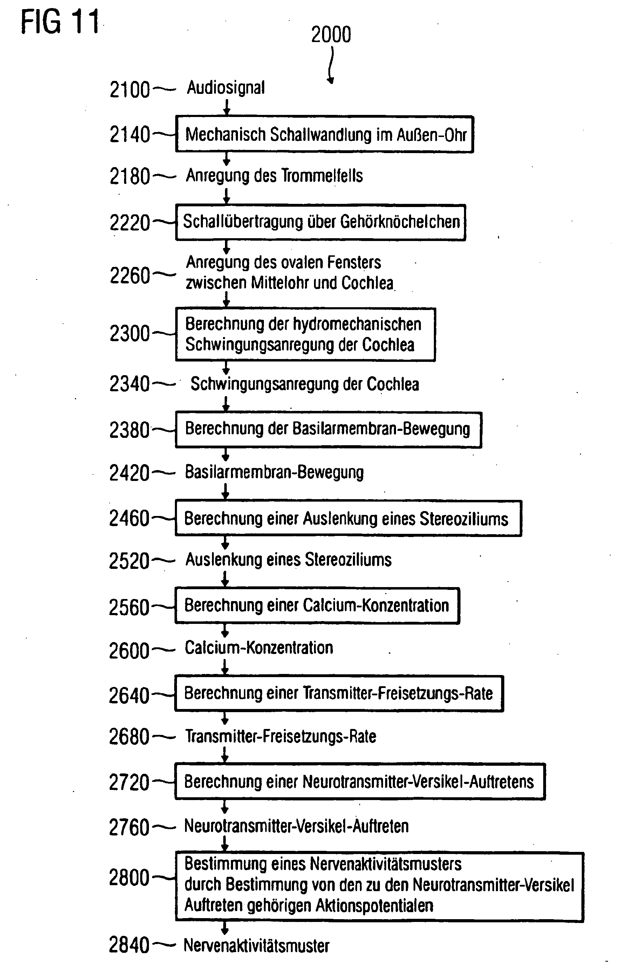

Im Folgenden wird anhand der Fig. 11 beschrieben, wie ein Aktivitätsmuster für die Verwendung im Rahmen der vorliegenden Erfindung basierend auf einem Gehörmodell eines menschlichen Ohres berechnet werden kann. Es sei darauf hingewiesen, dass ein Aktivitätsmuster eine Beschreibung für eine Aktivität in oder an einer Mehrzahl von Hörzellen eines Gehörmodells oder an einer Mehrzahl von Hörnerven eines Gehörmodells darstellt. Im Folgenden wird beschrieben, welche Zwischengrößen beispielsweise bei der Auswertung eines Gehörmodells eines Ohres berechnete werden können. Jede dieser Zwischengrößen eignet sich beispielsweise für eine Bestimmung eines Aktivitätsmusters, wobei ein Aktivitätsereignis dann gegeben ist, wenn die betreffende Zwischengröße von einem Ruhewert, der sich ohne Vorliegen eines Audiosignals ergibt, um mehr als einen vergebenen Wert abweicht. In anderen Worten, ein Aktivitätsereignis liegt vor, wenn eine der im Folgenden genannten Zwischengrößen oder Endgrößen einen vorgegebenen Schwellwert überschreitet oder unterschreitet. Es hat sich im Übrigen gezeigt, dass sich für die Bildung des Aktivitätsmusters besonders gut die Transmitter-Freisetzungsrate 2680, das Neurotransmitter-Vesikel-Auftreten 2760 sowie das Nervenaktivitätsmuster 2840 eignen.

Details der Berechnung der gegebenen Größen unter Verwendung eines fortschrittlichen Gehörmodells werden im Folgenden angegeben. Allerdings sei darauf hingewiesen, dass sich für die Berechnung des Aktivitätsmusters bzw. der Aktivitätsmuster auch andere Gehörmodelle eignen, bei denen eine oder mehrere der Zwischengrößen in einer anderen Weise berechnet werden, oder bei denen die Berechnung einer oder mehrerer Zwischengrößen entfällt.

Die Fig. 11 zeigt zu diesem Zweck eine schematische Darstellung des Ablaufs bei einer Simulation eines menschlichen Gehörs sowie der bei der Simulation auftretenden Zwischen- und Endergebnisse. Die schematische Darstellung der Fig. 1 ist in ihrer Gesamtheit mit 2000 bezeichnet. In anderen Worten, die Fig. 11 beschreibt ein Gehörmodell eines menschlichen Ohrs zum Einsatz in Verbindung mit der vorliegenden Erfindung.

Die schematische Darstellung 2000 der Fig. 11 beschreibt somit ein Simulationsmodell eines menschlichen Gehörs. Ein Audiosignal 2100 dient als Eingangssignal für das Simulationsmodell 2000. Basierend auf dem Audiosignal 2100 wird in einem ersten Schritt 2140 eine mechanische Schallwandlung in einem Außenohr ausgewertet, wodurch eine Anregung 2180 eines Trommelfells ermittelt wird. In einem zweiten Schritt 2220 wird eine Schallübertragung über Gehörknöchel berechnet bzw. simuliert, wodurch aus der Anregung 2180 des Trommelfells eine Anregung 2260 eines ovalen Fensters zwischen einem Mittelohr und einer Cochlea ermittelt wird. In einem dritten Schritt 2300 wird eine hydromechanische Schwingungsanregung 2340 der Cochlea berechnet bzw. simuliert. In einem vierten Schritt 2380 wird aus der Schwingungsanregung 2340 der Cochlea eine Basilarmembranbewegung 2420 ermittelt. In einem fünften Schritt 2460 wird von der Basilarmembranbewegung 2420 auf eine Auslenkung 2520 eines Stereoziliums gefolgert. Basierend auf der Auslenkung 2520 des Stereoziliums wird sodann in einem sechsten Schritt 2560 eine Calciumkonzentration 2600 in einer Haarzelle berechnet. Die Calciumkonzentration 2600 wird dann herangezogen, um in einem siebten Schritt 2640 eine Transmitter-Freisetzungs-Rate 2680 von Transmitter-Substanzen zu berechnen. Basierend auf der Transmitter-Freisetzungs-Rate 2680 wird in einem achten Schritt 2720 ein Neurotransmitter-Vesikel-Auftreten 2760 hergeleitet, das ein Auftreten von Neurotransmitter-Vesikeln beschreibt. Schließlich wird in einem neunten Schritt 2800 ein Nervenaktivitätsmuster 2840 von dem Neurotransmitter-Vesikel-Auftreten 2760 abgeleitet. Das Nervenaktivitätsmuster 2840 beschreibt dabei näherungsweise eine bei einem gesunden Gehör auftretende Aktivität auf Nervenzellen eines (menschlichen oder tierischen) Hörnervs. Das Nervenaktivitätsmuster 2840 ist somit gut geeignet, um eine Aussage über eine Stimulation von Hörnerven durch ein Cochlea-Implantat zu liefern.

Es wird darauf hingewiesen, dass im Rahmen des Simulationsmodells 2000 mehrere der Schritte 2140, 2220, 2300, 2380, 2460, 2560, 2640, 2720, 2800 zusammengefasst werden können, ohne ein entsprechendes Zwischenergebnis zu berechnen. In anderen Worten, es können mehrere Schritte in einem vereinfachten Schritt ohne Berechnung der in der graphischen Darstellung 2000 gezeigten Zwischenschritte abgearbeitet werden.

Sowohl die Auslenkung 2520 eines Stereoziliums, die Calciumkonzentration 2600 in einer inneren Haarzelle, die Transmitter-Freisetzungs-Rate 2680 in einer inneren Haarzelle, ein Neurotransmitter-Vesikel-Auftreten 2760 in einer inneren Haarzelle oder ein an der inneren Haarzelle anliegendes Nervenaktivitätsmuster 2840 (bzw. der zugehörige zeitliche Verlauf) können jeweils ein Aktivitätsmuster bzw. einen Teil eines Aktivitätsmusters an einer inneren Haarzelle darstellen.

Ebenso können beispielsweise die Konzentrationen 2600 von Calcium-Ionen in einer Mehrzahl von inneren Haarzellen ein Aktivitätsmuster bilden. Ein Aktivitätsereignis ist bei Betrachtung der Calciumkonzentration beispielsweise ein signifikanter Anstieg der Calciumkonzentration über einen bestimmten Schwellwert oder ein Abfall der Calciumkonzentration unter einem bestimmten Schwellwert.

Im Übrigen kann das Aktivitätsmuster beispielsweise auch eine Abweichung der gegenwärtigen Calciumkonzentrationen von Gleichgewichts-Calciumkonzentrationen beschreiben. Ein Aktivitätsereignis wird in diesem Fall durch eine signifikante Abweichung der jeweiligen Calcium-Konzentrationen nach oben bzw. nach unten beschrieben.

Ferner kann auch der zeitliche Verlauf einer Transmitter-Freisetzungs-Rate 2680 oder einer Transmitter-Freisetzungs-Wahrscheinlichkeit in einer Mehrzahl von inneren Haarzellen als Aktivitätsmuster über der Zeit verwendet werden. Das Aktivitätsmuster wird dabei durch die Transmitter-Freisetzungs-Rate oder Transmitter-Freisetzungs-Wahrscheinlichkeit in der i-ten inneren Haarzelle gebildet.

Im Übrigen wird darauf hingewiesen, dass bei der Betrachtung der Transmitter-Freisetzungs-Rate 2680 bzw. Transmitter-Freisetzungs-Wahrscheinlichkeit beispielsweise das Vorliegen einer Transmitter-Freisetzungs-Rate größer Null bzw. eine Transmitter-Freisetzungs-Wahrscheinlichkeit größer Null als ein Aktivitätsereignis verstanden werden kann.

Ferner kann auch ein Neurotransmitter-Vesikel-Auftreten 2760 in einer Mehrzahl von inneren Haarzellen ein Aktivitätsmuster bilden. In anderen Worten, ein Aktivitätsmuster beschreibt in dem genanten Fall, wie viele Neurotransmitter-Vesikel beispielsweise während eines bestimmten Zeitraums bzw. Zeitintervalls freigesetzt vorliegen. Das Neurotransmitter-Vesikel-Auftreten 2760 kann allerdings auch beschreiben, wie viele Neurotransmitter-Vesikel während eines Zeitraums bzw. eines Zeitintervalls neu freigesetzt werden. Der entsprechende zeitliche Verlauf kann ferner beispielsweise beschreiben, wie viele Neurotransmitter-Vesikel in der präsynaptischen inneren Haarzelle in einer Zeiteinheit freigesetzt werden oder in einem Zeitintervall oder zu einem Zeitpunkt insgesamt in freier Form vorhanden sind. Ferner kann das Neurotransmitter-Vesikel-Auftreten 2760 aber auch beschreiben, wie viele Neurotransmitter-Vesikel beispielsweise pro Zeiteinheit aus der präsynaptischen inneren Haarzelle in den synaptischen Spalt diffundieren, der die präsynaptische innere Haarzelle mit einer Nervenfaser koppelt.

In anderen Worten, unter einem Neurotransmitter-Vesikel-Auftreten ist beispielsweise eine Anzahl von tatsächlich vorhandenen oder pro Zeiteinheit freigesetzten Neurotransmitter-Vesikeln zu verstehen, wobei es für die vorliegende Erfindung nicht relevant ist, wo genau innerhalb einer Haarzelle oder Synapse die entsprechende Anzahl an Neurotransmitter-Vesikeln oder die entsprechende Freisetzungs-rate von Neurotransmitter-Vesikeln bestimmt wird. Es ist nämlich davon auszugehen, dass Neurotransmitter-Vesikel, die im präsynaptischen Teil der inneren Haarzelle freigesetzt werden, mit einer gewissen Zeitkonstante in den synaptischen Spalt diffundieren.

Im Übrigen wird darauf hingewiesen, dass ein Neurotransmitter-Vesikel-Auftreten sowohl qualitativ als auch quantitativ beschrieben werden kann, um ein Aktivitätsmuster im Sinne der vorliegenden Erfindung zu beschreiben. In anderen Worten, ein zu einem Neurotransmitter-Vesikel-Auftreten 2760 gehöriger zeitlicher Verlauf kann beispielsweise beschreiben, wie viele Neurotransmitter-Vesikel freigesetzt werden oder in freier Form vorhanden sind. Der Verlauf kann auch nur eine qualitative Information darüber geben, ob Neurotransmitter-Vesikel in einem Zeitintervall freigesetzt werden bzw. in freier Form vorhanden sind.

Bei der Betrachtung eines Neurotransmitter-Vesikel-Auftretens 2760 in bzw. an einer Mehrzahl von inneren Haarzellen eines Gehörmodells als das Aktivitätsmuster können als Aktivitätsereignisse beispielsweise die Freisetzung einer Anzahl an Neurotransmitter-Vesikeln in einer Zeiteinheit, die größer als ein bestimmter Schwellwert ist, angesehen werden. Beispielsweise kann angenommen werden, dass immer dann ein Aktivitätsereignis auftritt, wenn überhaupt ein Neurotransmitter-Vesikel innerhalb eines Zeitintervalls freigesetzt wird. Ferner kann angenommen werden, dass ein Aktivitätsereignis vorliegt, wenn zumindest ein (oder allgemeiner: mehr als eine vorgegebene minimale Anzahl) freies Neurotransmitter-Vesikel in einer inneren Haarzelle vorliegt.

In anderen Worten, ein Aktivitätsereignis ist ein Ereignis, das in einer einzigen inneren Haarzelle auftritt. Ein Aktivitätsereignis macht sich typischerweise in einem lokalen Minimum oder Maximum des Aktivitätsmusters oder in einer Über- bzw. Unterschreitung eines Schwellwerts bemerkbar.

Ferner kann als Aktivitätsmuster auch ein Nervenaktivitätsmuster an einer Mehrzahl von inneren Haarzellen eines Gehörmodells verwendet werden. Das Nervenaktivitätsmuster beschreibt dabei die Aktivität bzw. den zeitlichen Verlauf der Aktivität auf mehreren verschiedenen Nervenfasern, die mit mehreren verschiedenen inneren Haarzellen gekoppelt sind. Einem i-ten zeitlichen Verlauf des Aktivitätsmusters ist beispielsweise ein zeitlicher Verlauf eines Potentials bzw. einer Spannung an einer mit einer i-ten inneren Haarzelle gekoppelten Nervenfaser zugeordnet. Auf den einzelnen Nervenfasern treten dabei Aktionspotentiale auf, deren Auftreten durch das Aktivitätsmuster beschrieben wird. Unter einem Aktivitätsereignis ist in diesem Falle das Auftreten eines Aktionspotentials auf einer von n betrachteten Nervenfasern zu verstehen.

Zusammenfassend lässt sich also festhalten, dass in einem Verfahren gemäß dem Flussdiagramm 2000 verschiedene Größen 2520, 2600, 2680, 2760 für eine Mehrzahl von verschiedenen inneren Haarzellen berechnet werden können, wobei ein Aktivitätsmuster durch eine Zusammenfassung der gleichen Größe für eine Mehrzahl von unterschiedlichen Haarzellen gebildet wird.

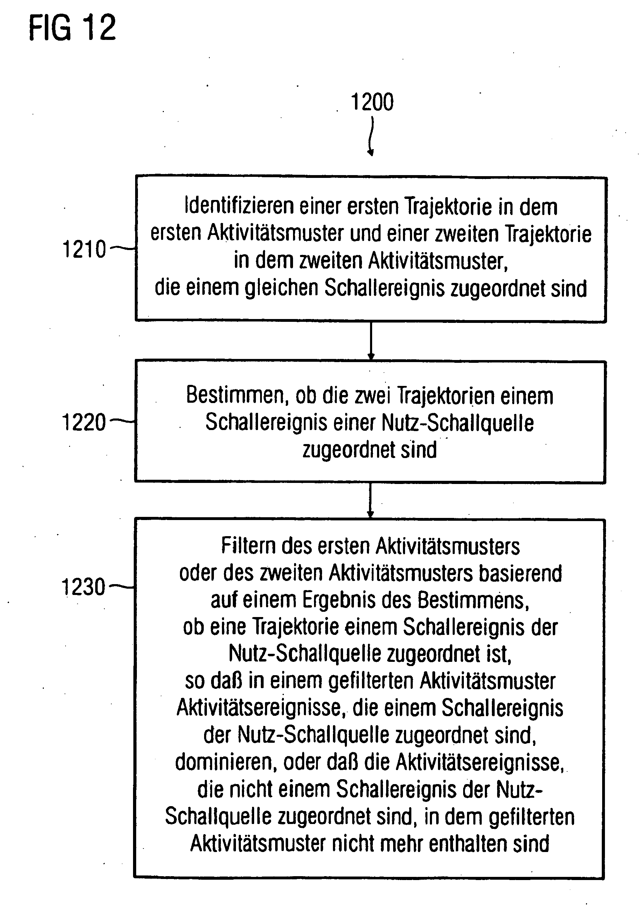

Fig. 12 zeigt im Übrigen ein Flussdiagram eines erfindungsgemäßen Verfahrens zum Erzeugen eines gefilterten Aktivitätsmusters basierend auf einem ersten Aktivitätsmuster an einem Gehörmodell eines ersten Ohres und eines zweiten Aktivitätsmusters an einem Gehörmodell eines zweiten Ohres. Das Verfahren gemäß der Fig. 12 ist in seiner Gesamtheit mit 1200 bezeichnet.

Das Verfahren 1200 umfasst in einem ersten Schritt 1210 ein Identifizieren einer ersten Trajektorie in dem ersten Aktivitätsmuster und einer zweiten Trajektorie in dem zweiten Aktivitätsmuster, die einem gleichen Schallereignis zugeordnet sind.

Das Verfahren 1200 umfasst in einem zweiten Schritt 1220 ein Bestimmen, ob die zwei Trajektorien einem Schallereignis einer Nutz-Schallquelle zugeordnet sind.

Das Verfahren 1200 umfasst ferner in einem dritten Schritt 1230 ein Filtern des ersten Aktivitätsmusters oder des zweiten Aktivitätsmusters basierend auf einem Ergebnis des Bestimmens, ob eine Trajektorie einem Schallereignis der Nutz-Schallquelle zugeordnet ist, so dass in einem gefilterten Aktivitätsmuster Aktivitätsereignisse, die einem Schallereignis der Nutz-Schallquelle zugeordnet sind, dominieren, oder dass Aktivitätsereignisse, die nicht einem Schallereignis der Nutz-Schallquelle zugeordnet sind, in dem gefilterten Aktivitätsmuster nicht mehr enthalten sind.

Es sei im Übrigen darauf hingewiesen, dass das Verfahren 1200 um all diejenigen Schritte ergänzt bzw. erweitert werden kann, die durch die oben beschriebenen erfindungsgemäßen Vorrichtungen ausgeführt werden.

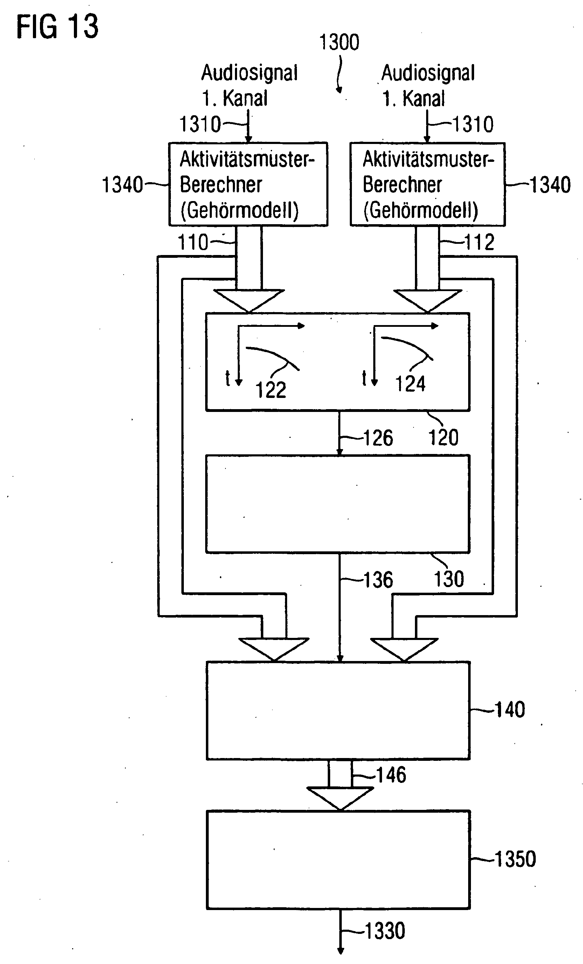

Fig. 13 zeigt ein Blockschaltbild eines erfindungsgemäßen Quellentrenners gemäß einem Ausführungsbeispiel der vorliegenden Erfindung. Der Quellentrenner der Fig. 13 ist in seiner Gesamtheit mit 1300 bezeichnet. Der Quellentrenner 1300 ist ausgelegt, um einen ersten Kanal 1310 eines zumindest zweikanaligen Audiosignals zu empfangen. Der Quellentrenner 1300 ist ferner ausgelegt, um einen zweiten Kanal 1320 des zumindest zweikanaligen Audiosignals zu empfangen. Der Quellentrenner 1300 ist ferner ausgelegt, um ein bereinigtes Audiosignal 1330 basierend auf dem Audiosignal mit zumindest zwei Kanälen zu liefern.

Der Quellentrenner 1300 umfasst einen ersten Aktivitätsmusterberechner 1340, der ausgelegt ist, um ein erstes Aktivitätsmuster 110 basierend auf dem ersten Kanal 1310 des Audiosignals zu berechnen. Der Aktivitätsmusterberechner 1340 umfasst bzw. verwendet dabei beispielsweise ein Gehörmodell eines Ohres. Der Quellentrenner 1300 umfasst ferner einen zweiten Aktivitätsmusterberechner 1342, der ausgelegt ist, um ein zweites Aktivitätsmuster 112 basierend auf dem zweiten Kanal 1320 des Audiosignals zu berechnen. Zu diesem Zweck ist der Aktivitätsmusterberechner 1342 beispielsweise ausgelegt, um ein Gehörmodell eines Ohres auf den zweiten Kanal 1320 des Audiosignals anzuwenden, um das zweite Aktivitätsmuster 112 zu erhalten.

Der Quellentrenner 1300 umfasst im übrigen einen Identifizierer 120, einen Bestimmer 130 und ein Filter 140, wie sie bereits anhand der Fig. 1 beschrieben wurden. Die Einrichtung des Quellentrenners 1300, die mit Einrichtungen der Vorrichtung 100 übereinstimmen, sind mit gleichen Bezugszeichen wie in den Fig. 1 und 2 bezeichnet und werden hier nicht noch einmal erläutert. Vielmehr wird auf die Beschreibung im Hinblick auf die Fig. 1 und 2 verwiesen.

Der Quellentrenner 1300 umfasst zusätzlich zu den Einrichtungen der Vorrichtung 100, 200 einen Synthesizer 1350, der ausgelegt ist, um das gefilterte Aktivitätsmuster 146 zu erhalten, und um basierend auf dem gefilterten Aktivitätsmuster 146 das bereinigte Audiosignal 1330 zu erzeugen. Zu diesem Zweck ist der Synthesizer ausgelegt, um das bereinigte Aktivitätsmuster 146 in eine Zeitdarstellung, eine Frequenzdarstellung oder eine Subbanddarstellung zu transformieren. In anderen Worten, der Synthesizer 1350 ist bevorzugt ausgelegt, um die bei der Bestimmung des Aktivitätsmusters anhand des Gehörmodells durchgeführten Berechnungen zumindest teilweise umzukehren. In anderen Worten, der Synthesizer 1350 ist beispielsweise ausgelegt, um basierend auf einem Aktivitätsmuster, das ein Neurotransmitter-Vesikel-Auftreten darstellt, das bereinigte Audiosignal als ein Zeitbereichs-Audiosignal zu rekonstruieren. Alternativ dazu kann der Synthesizer 1350 auch ausgelegt sein, um beispielsweise ein Nervenaktivitätsmuster in ein Zeitbereichs-Audiosignal zu transformieren. Anstelle der Zeitbereichs-Darstellung des Audiosignals kann im Übrigen auch eine Frequenzbereichs-Darstellung, also beispielsweise eine Darstellung von Energien oder komplexen Amplitudenwerten in einer Mehrzahl von spektralen Bereichen, oder als eine Darstellung in Form einer Mehrzahl von komplexen Zeigern in einer Mehrzahl von Frequenzbändern, verwendet werden.

Der Quellentrenner 1300 ermöglicht damit unter Verwendung eines mehrkanaligen (zumindest zweikanaligen) Audiosignals eine Quellentrennung, wobei die Quellentrennung auf der Basis einer Erkennung von zusammengehörigen Trajektorien in zwei Aktivitätsmustern, die die zwei Kanäle des Audiosignals darstellen, erfolgt.

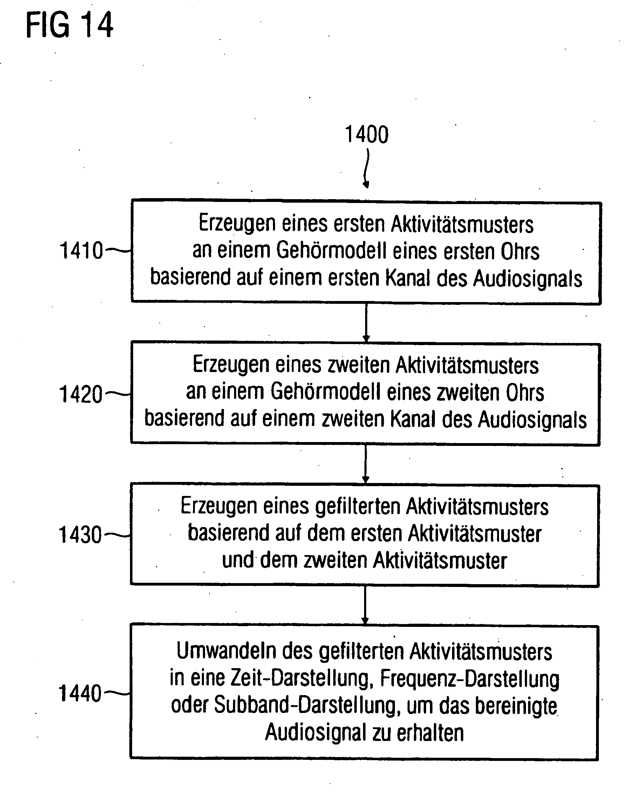

Fig. 14 zeigt ein Flussdiagramm eines erfindungsgemäßen Verfahrens zum Erzeugen eines bereinigte Audiosignals basierend auf einem Audiosignal mit zumindest zwei Kanälen. Das Verfahren gemäß der Fig. 14 ist in seiner Gesamtheit mit 1400 bezeichnet.

Das Verfahren 1400 umfasst in einem ersten Schritt 1410 ein Erzeugen eines ersten Aktivitätsmusters an einem Gehörmodell eines ersten Ohres basierend auf einem ersten Kanal des Audiosignals.

Das Verfahren 1400 umfasst ferner in einem zweiten Schritt 1420 ein Erzeugen eines zweiten Aktivitätsmusters an einem Gehörmodell eines zweiten Ohres basierend auf einem zweiten Kanal des Audiosignals.

Das Verfahren 1400 umfasst ferner in einem dritten Schritt 1430 ein Erzeugen eines gefilterten Aktivitätsmusters basierend auf dem ersten Aktivitätsmuster und dem zweiten Aktivitätsmuster, wie dies bereits oben anhand der Fig. 1 und 2 beschrieben wurde.

Das Verfahren 1400 umfasst ferner in einem vierten Schritt 1440 ein Umwandeln des gefilterten Aktivitätsmusters in eine Zeit-Darstellung, Frequenz-Darstellung oder Subband-Darstellung, um das bereinigte Audiosignal zu erhalten.

Es sei im Übrigen darauf hingewiesen, dass das Verfahren 1400 um all diejenigen Schritte erweitert werden kann, die durch die im Rahmen der vorliegenden Anmeldung beschriebenen erfindungsgemäßen Vorrichtungen ausgeführt werden. Im Übrigen sei darauf hingewiesen, dass das Erzeugen des gefilterten Aktivitätsmusters basierend auf dem ersten Aktivitätsmuster und dem zweiten Aktivitätsmuster beispielsweise unter Verwendung des Verfahrens 1200 gemäß Fig. 12 erfolgen kann.

Fig. 15 zeigt im Übrigen einen Auszug aus einem Blockschaltbild einer erfindungsgemäßen Vorrichtung zur Berechnung eines gefilterten Aktivitätsmusters basierend auf zwei Audiosignalen. Die Vorrichtung gemäß der Fig. 15 ist in ihrer Gesamtheit mit 1500 bezeichnet. Die Vorrichtung 1500 ist ausgelegt, um ein erstes Audiosignal 1510, beispielsweise von einem ersten Mikrofon, das beispielsweise in einer Umgebung eines ersten Ohres (beispielsweise eines Menschen) angeordnet ist, zu empfangen. Die Vorrichtung 1500 ist ferner ausgelegt, um ein zweites Audiosignal 1512, beispielsweise von einem zweiten Mikrofon, das beispielsweise in einer Umgebung eines zweiten (beispielsweise menschlichen Ohres) angeordnet ist, zu empfangen. Die Vorrichtung 1500 ist also beispielsweise ausgelegt, um das erste Audiosignal 1510 von einem an einer linken Seite eines menschlichen Kopfes angeordneten Mikrofon zu empfangen, und um das zweite Audiosignal 1512 von einem an einer rechten Seite eines menschlichen Kopfes angeordneten Mikrofon zu empfangen. Die Vorrichtung 1500 umfasst ferner einen ersten Aktivitätsmusterberechner 1520, der ausgelegt ist, um beispielsweise unter Verwendung eines Gehörmodells ein erstes Aktivitätsmuster 1522 basierend auf dem ersten Audiosignal 1510 zu berechnen. Die Vorrichtung 1500 umfasst ferner einen zweiten Aktivitätsmusterberechner 1530, der ausgelegt ist, um unter Verwendung eines Gehörmodells ein zweites Aktivitätsmuster 1532 basierend auf dem zweiten Audiosignal 1512 zu berechnen. Die Vorrichtung 1500 umfasst ferner ein erstes Hubel-Wiesel-Netzwerk 1540, das ausgelegt ist, um das erste Aktivitätsmuster 1522 zu empfangen, und um basierend darauf eine Mehrzahl von parallelen Signalen 1542 zu erzeugen, die ausgelegt sind, um das Vorliegen von Trajektorien verschiedener Krümmung in dem Aktivitätsmuster 1522 anzuzeigen. In anderen Worten, die Mehrzahl von parallelen Signalen 1542 ist ausgelegt, um anzuzeigen, wenn eine Trajektorie mit einer einem Signal zugeordneten Krümmung aus einer Mehrzahl von Trajektorien mit verschiedenen Krümmungen vorliegt. Die parallelen Leitungen 1542 entsprechen dabei in ihrer Funktionsweise den Signalen 740, 742, 744, 746 der Vorrichtung 700, und das erste Hubel-Wiesel-Netzwerk 1540 entspricht dem ersten Trajektorien-Erkenner 730. Die Vorrichtung 1500 umfasst ferner ein zweites Hubel-Wiesel-Netzwerk 1550, das ausgelegt ist, um basierend auf dem zweiten Aktivitätsmuster 1532 eine Mehrzahl von parallelen Signalen 1552 zu erzeugen. Die parallelen Signale der Mehrzahl 1542 von parallelen Signalen signalisieren dabei das Vorliegen einer Trajektorie mit einer bestimmten Krümmung in dem zweiten Aktivitätsmuster 1532. Im Hinblick auf die parallelen Leitungen der Mehrzahl 1552 von parallelen Leitungen gilt im Übrigen das im Hinblick auf die Mehrzahl von parallelen Leitungen 1542 das Gesagte. Weiterhin entsprechen die parallelen Leitungen 1552 den Leitungen 750, 752, 754, 756 der Vorrichtung 700, und das zweite Hubel-Wiesel-Netzwerk 1550 entspricht dem zweiten Trajektorien-Erkenner 732 der Vorrichtung 700.

Die Vorrichtung 1500 umfasst ferner eine Multikoinzidenzeinheit 1560, die ausgelegt ist, um erste Eingangssignale über die erste Mehrzahl 1542 von parallelen Leitungen zu empfangen, und um zweite Eingangssignale über die zweite Mehrzahl 1552 von parallelen Leitungen zu empfangen. Es sei im Übrigen darauf hingewiesen, dass die Multikoinzidenzeinheit 1560 im Wesentlichen dem Koinzidenz-Erkenner 770 der Vorrichtung 700 entspricht. Es sei darauf hingewiesen, dass es sich bei der Multikoinzidenzeinheit 1560 beispielsweise um eine Multikoinzidenzeinheit handeln kann, wie sie anhand der Fig. 5 bereits beschrieben wurde.

Die Multikoinzidenzeinheit 1560 ist somit ausgelegt, um in Verbindung mit den Hubel-Wiesel-Netzwerken 1540, 1550 festzustellen, ob in den Aktivitätsmustern 1522, 1532 Trajektorien einer gleichen Krümmung enthalten sind, und um ferner eine zeitliche Verschiebung zwischen den Trajektorien mit der gleichen Krümmung zu bestimmen. Die entsprechende Information kann dann verwendet werden, um das erste Aktivitätsmuster 1522 und/oder das zweite Aktivitätsmuster 1532 zu filtern, um ein gefiltertes Aktivitätsmuster zu erhalten, wie dies bereits oben ausgeführt wurde.

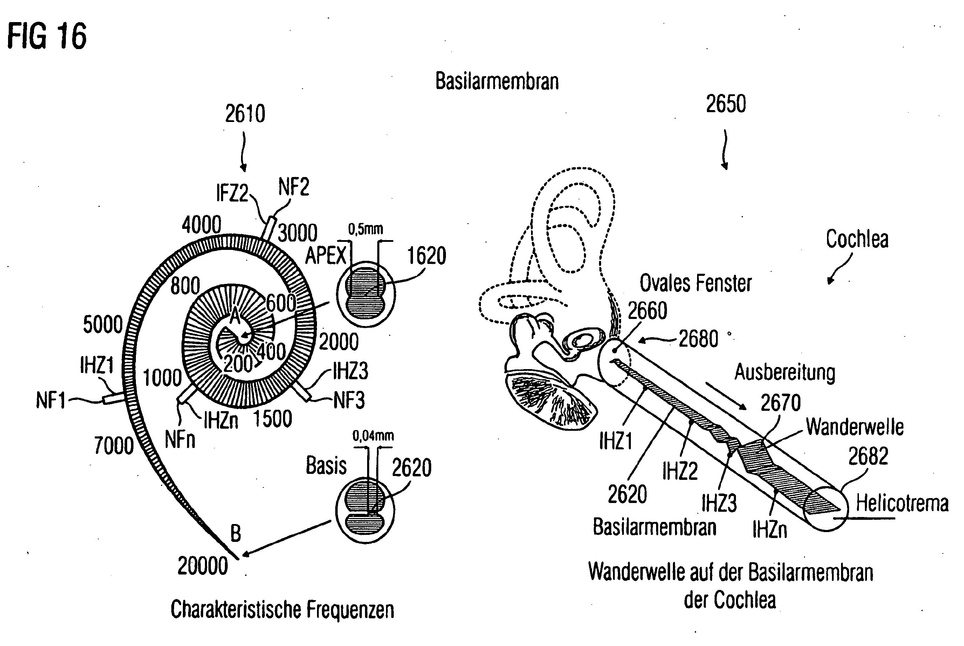

Fig. 16 zeigt im Übrigen zur Erläuterung, wie die inneren Hörrzellen bzw. inneren Haarzellen IHZ1, IHZ2, IHZ3, IHZ4 entlang einer Basilarmembran angeordnet sein können.

Fig. 16 zeigt zu diesem Zweck eine graphische Darstellung einer Geometrie der Basilarmembran und eine Reaktion der Basilarmembran auf eine Anregung.

Eine erste graphische Darstellung 2610 zeigt, dass eine Breite einer Basilarmembran 2620 von der Basis der Cochlea zu dem Ende (Apex) der Cochlea hin um etwa den Faktor 10 zunimmt. Ferner zeigt die graphische Darstellung 2610, verschiedene charakteristische Frequenzen (in Hertz), hinsichtlich derer an verschiedenen Orten der Cochlea eine maximale Empfindlichkeit besteht. In der Nähe der Basis der Cochlea werden Frequenzen in der Größenordnung von 20.000 Hertz am stärksten wahrgenommen. Von der Basis der Cochlea aus betrachtet, nimmt die Frequenz, für die sich eine maximale Empfindlichkeit ergibt, kontinuierlich ab. Die graphische Darstellung 610 zeigt ferner vier beispielhafte innere Haarzellen IHZ1, IHZ2, IHZ3, IHZn, die entlang der Cochlea angeordnet sind, und die mit zugehörigen Nervenfasern NF1, NF2, NF3, NFn gekoppelt sind. Unter der Annahme einer sinusförmigen Anregung der Cochlea spricht somit beispielsweise die erste innere Haarzelle IHZ1 am stärksten bei einer Anregung mit einer Frequenz von etwa 6.000 Hz an. Die zweite innere Haarzelle IHZ2 weist hingegeben beispielsweise eine maximale Empfindlichkeit bei einer Anregung mit einer Frequenz von etwa 3.300 Hz auf. Analog weisen die übrigen inneren Haarzellen IHZ3, IHZn andere Frequenzen maximaler Empfindlichkeit auf.

Eine zweite graphische Darstellung 2650 beschreibt ferner eine Einkopplung einer akustischen Welle in die Cochlea über ein ovales Fenster 2660. Die Einkopplung über das ovale Fenster 2660 erzeugt eine Wanderwelle 2670 in der Cochlea, die von einer Basis 2680 der Cochlea zu einem Apex 2682 der Cochlea läuft und dabei die Basilarmembran 2620 auslenkt. Die Nervenzellen, die näher bei der Basis 2680 die Cochlea gelegen sind, werden früher angeregt, als Nervenzellen, die weiter von der Basis 2680 der Cochlea entfernt sind. Mit anderen Worten, der Ort der Wanderwelle 2670 als Funktion der Zeit kann als Trajektorie der Wanderwelle 2670 angesehen werden. Die Trajektorie kann freilich auch auf diskrete Nervenzellen abgebildet werden, so dass eine Trajektorie ebenso beschreibt, in welcher zeitlichen Folge mehrere räumlich getrennte Nervenzellen durch eine Wanderwelle angeregt werden.

In dem gezeigten Beispiel wird beispielsweise die innere Haarzelle IHZ1 früher von der Wanderwelle 2670 angeregt als die anderen inneren Haarzellen IHZ2, IHZ3, IHZn. Die erste innere Haarzelle IHZ1 liegt nämlich näher an dem ovalen Fenster 2660, wobei sich die Wanderwelle 2670 von dem ovalen Fenster (also von der Basis 2680 der Cochlea) zu dem Apex 2682 der Cochlea ausbreitet. Somit werden nacheinander die erste innere Haarzelle IHZ1, die zweite innere Haarzelle IHZ2, die dritte innere Haarzelle IHZ3 und die n-te innere Haarzelle IHZn angeregt. In anderen Worten, eine einzige Wanderwelle erzeugt an den gezeigten inneren Haarzellen Aktivitätsereignisse in einer zeitlichen Folge, wobei die zeitlichen Abstände durch die Ausbreitungsgeschwindigkeit der Wanderwelle 2670 und die Lage der entsprechenden inneren Haarzellen IHZ1, IHZ2, IHZ3, IHZn bestimmt werden. Es ist allerdings festzuhalten, dass die Aktivitätsereignisse an den inneren Haarzellen (bei Betrachtung in einer zweidimensionalen Darstellung in Abhängigkeit von der Zeit und dem Index i der inneren Haarzellen IHZi eine Trajektorie bilden, die beispielsweise einer zeitlichen Trajektorie eines Orts maximaler Auslenkung der Wanderwelle 2670 entspricht.

Ferner können die erfindungsgemäßen Verfahren, abhängig von den Gegebenheiten, in Hardware oder in Software implementiert werden. Die Implementation kann auf einem digitalen Speichermedium, beispielsweise einer Diskette, CD, DVD, ROM, PROM, EPROM, EEPROM oder einem Flash-Speichermedium, mit elektronisch auslesbaren Steuersignalen erfolgen, die so mit einem programmierbaren Computersystem zusammenwirken können, dass das entsprechende Verfahren ausgeführt wird. Allgemein besteht die Erfindung also auch in einem Computer-Programm-Produkt mit auf einem maschi-nenlesbaren Träger gespeicherten Programm-Code zur Durchführung des erfindungsgemäßen Verfahrens, wenn das Computer-Programm-Produkt auf einem Rechner abläuft. In anderen Worten ausgedrückt, kann die Erfindung somit als ein Computer-Programm mit einem Programm-Code zur Durchführung des Verfahrens realisiert werden, wenn das Computer-Programm auf einem Computer abläuft.

Zusammenfassend lässt sich also festhalten, dass im Rahmen der vorliegenden Erfindung Verzögerungstrajektorien bzw. Delay-Trajektorien (die sich beispielsweise aufgrund einer Ausbreitung einer Wanderwelle auf einer Cochlea eines Innenohres bei Anregung der Cochlea durch ein Schallereignis ergeben, und die sich in Aktivitätsmustern wiederspiegeln, die aufgrund des Gehörmodells bestimmt sind) in einer Multikoinzidenzeinheit bzw. Multikoinzidenz-Unit (z.B. in einer Multikoinzidenzeinheit 500 gemäß Fig. 5a) gegeneinander verlaufen. Die Multikoinzidenzeinheit umfasst zu diesem Zweck n antiparallele Verzögerungsstrecken der Länge m, wobei n eine Anzahl an inneren Haarzellen (oder Nervenfasern) beschreibt, die bei einer Berechnung des Aktivitätsmusters berücksichtigt werden, und wobei m eine Anzahl an Körben bzw. Bins beschreibt und damit eine maximale Verzögerungszeit und/oder eine maximale Zeitauflösung definiert.

Im Rahmen der vorliegenden Erfindung sind zwei Verfahren von besonderem Vorteil. So können beispielsweise Aktivitätsmuster bzw. Aktivitätsereignisse (z.B. Vesikel) direkt in der Multikoinzidenzeinheit (z.B. der Multikoinzidenzeinheit 500 gemäß Fig. 5a) gegeneinander laufen. Daraufhin erfolgt eine Aufsummation (z.B. von Multikoinzidenzsignalen von Koinzidenzausgängen von Koinzidenzzellen 580) in Integratorzellen (bzw. in Summierern 530). Jede Verzögerungstrajektorie hat bzw. umfasst beispielsweise n Aktivitätsereignisse bzw. Vesikel. Folglich ist es vorteilhaft, dass jeder Integrator (bzw. Summierer 530) mit einer Schwelle versehen ist, die etwas unterhalb von m liegt (z.B. mindestens 50 % von n beträgt). Die Schwelle ist im Übrigen in dem Blockschaltbild gemäß der Fig. 5a durch Schwellwertentscheider 540 repräsentiert, wobei ferner gilt: n = I. Die Integratoren (bzw. die Summierer 530) sind ferner innerhalb einer Integrationszeit von etwa 10 bis 100 ms zurückzusetzen (um eine zuverlässige Detektion von einzelnen Trajektorien zu ermöglichen, und eine Vermischung con Koinzidenzereignissen von verschiedenen Trajektorien zu vermeiden).

Gemäß einem weiteren bevorzugten Konzept ist es möglich, der Multikoinzidenzeinheit 500 Hubel-Wiesel-Netzwerke bzw. Hough-Transformator-Netzwerke vorzuschalten, wie dies beispielsweise in Fig. 7 gezeigt ist. Dadurch werden die Verzögerungstrajektorien (in den Aktivitätsmustern) zu einer geraden Signalwellenfront gebogen. Erkannte Verzögerungstrajektorien bzw. Delay-Trajektorien werden dann in der Multikoinzidenzeinheit (z.B. in dem Koinzidenz-Erkenner 700) in Übereinstimmung bracht bzw. gematcht. In anderen Worten, in dem Koinzidenz-Erkenner 700 wird erkannt, wenn Trajektorien bzw. Verzögerungstrajektorien in den Aktivitätsmustern vorhanden sind, und es wird ferner eine zeitliche Verschiebung zwischen den Verzögerungstrajektorien mit gleicher Krümmung bestimmt.

Die vorliegende Erfindung schafft somit eine Vorrichtung zur binauralen Filterung von Aktivitätsmustern bzw. zur binauralen Vesikelfilterung. Verzögerungstrajektorien (in den Aktivitätsmustern) werden durch eine zeitliche Verschiebung gegeneinander gematcht bzw. in Übereinstimmung bracht. Details diesbezüglich sind beispielsweise der US 6, 442, 510 B1 zu entnehmen.

Ferner erfolgt eine direkte Vesikelfilterung gegeneinander, um Koinzidenzpaare zu bestimmen. Weiterhin erfolgt daraus resultierend eine Rauschunterdrückung bzw. Rauschquellenunterdrückung oder Störquellenunterdrückung, indem Vesikel von Rauschquellen (aus den ursprünglichen Aktivitätsmustern bzw. aus zumindest einem der ursprünglichen Aktivitätsmuster) herausgerechelt werden.

Ferner ermöglicht die vorliegende Erfindung eine zeitgenaue Taktung und Synchronisation eines linken und eines rechten Cochlea-Implantats, damit Schallquellen bezugsrichtig im Raum geortet werden können.

Durch die vorliegende Erfindung wird somit eine binaurale Rauschquellenfilterung ermöglicht, die es ermöglicht, einen Cocktail-Party-Effekt zu mildern.

Zusammenfassend lässt sich im Übrigen festhalten, dass es die vorliegende Erfindung ermöglicht, eine Winkelbestimmung von Schallquellen durchzuführen, indem beispielsweise eine zeitliche Verschiebung von Trajektorien, die zu dem gleichen Schallereignis gehören, bestimmt wird. Ferner ist es möglich, Aktivitätsereignisse bzw. Vesikel (im Rahmen der Filterung durch das Filter) schallquellenabhängig auszuwählen bzw. zu selektieren. In anderen Worten, in Abhängigkeit davon, von welcher Schallquelle Aktivitätsereignisse bzw. Vesikel stammen, werden die Aktivitätsereignisse bzw. Vesikel in das gefilterte Aktivitätsmuster übernommen bzw. die gefilterten Aktivitätsmuster bedämpft oder unterdrückt. Dies führt zu einer Milderung eines Cocktail-Party-Effekts (beispielsweise für einen Träger eines Cochlea-Implantats, das unter Verwendung des gefilterten Aktivitätsmusters angesteuert wird), da bei einem Ausführungsbeispiel der vorliegenden Erfindung nur noch Schallquellen aus einer bestimmten Richtung (beispielsweise in Bezug auf den Patienten mit dem Cochlea-Implantat) wiedergegeben werden.

Es sei im Übrigen darauf hingewiesen, dass das erfindungsgemäße Verfahren besondere Vorteile mit sich bringt, wenn durch das gefilterte Aktivitätsmuster (bzw. noch besser, durch zwei gefilterte Aktivitätsmuster) ein Cochlea-Implantat angesteuert wird. Bei einem Träger eines Cochlea-Implantats kann nämlich in manchen Fällen durch das Gehirn eine binaurale Schallquellenortung nicht mehr geleistet werden. Dies ist beispielsweise der Fall, weil die Elektroden der Implantate in beiden Ohren an unterschiedlichen Stellen längs der Basilarmembran liegen. Somit ist keine gleiche Zuordnung zu inneren Hörzellen bzw. inneren Haarzellen mehr möglich.

Ein vorteilhafter Einsatz des erfindungsgemäßen Konzepts kann ferner erfolgen, wenn von einem gegenüberliegenden Ohr (bzw. von einem Ausgang der Multikoinzidenzeinheit) afferent äußere Haarzellen angesteuert werden. Mit dieser vorberechneten Information kann ein Cochlea-Implantat gebaut werden, das intakte äußere Haarzellen in der gleichen Weise reizt, wodurch eine neue Generation von Cochlea-Implantaten entsteht.

In anderen Worten, die Ausgabe bzw. die Ausgangssignale der Multikoinzidenzeinheit ist eine Eingangsgröße für einen efferenten Rückkopplungsregelkreis zur Steuerung der äußeren motorischen Haarzellen, um selektiv einen Dynamikpegelbereich zu justieren.

Gemäß einem bevorzugten Ausführungsbeispiel der vorliegenden Erfindung wird ein Ohrmodell paarig aufgebaut. Signale von inneren Hörzellen bzw. inneren Haarzellen der linken und der rechten Seite werden paarweise in antiparallelen Verzögerungsstrecken zur Koinzidenz gebracht (Schaltungsvariante 1).

Gemäß einem Aspekt der vorliegenden Erfindung kommt hinzu, dass das System dreifach aufgebaut wird, mit den separaten Signalen aus HSR, MSR und LSR Spiralganglienzellen. Da diese einen unterschiedlichen Ansprechpegelbereich haben, sieht die Vesikelausschüttung für laute, mittlere und leisere Töne unterschiedlich aus. Somit können unterschiedlich laute Signalquellen separiert werden.

Das erfindungsgemäße Konzept dient dazu, einen Cocktail-Party-Effekt zu mindern, indem die Schallquellen durch die erfindungsgemäße Vorrichtung bzw. das erfindungsgemäße Verfahren geortet werden. Daraufhin werden Vesikel, die zu einer Rauschquelle (auch als Stör-Schallquelle bezeichnet) gehören (bzw. die zu einer Stör-Schallquelle gehören) selektiv gefiltert (bzw. ausgefiltert bzw. entfernt) (z.B. bei der Erzeugung des gefilterten Aktivitätsmusters).

Gemäß einem weiteren Aspekt der vorliegenden Erfindung ist im übrigen der Multikoinzidenzeinheit ein Hubel-Wiesel-Netzwerk vorgeschaltet.

Gemäß einem weiteren Aspekt der vorliegenden Erfindung werden Ausgänge der Multikoinzidenzeinheit einem Rückkopplungskreis zugeführt, der am Ausgang äußere Haarzellen innerviert durch efferente Leitungsbahnen. Wird von dem Regelkreis aus den Eingangskenngrößen ein selektives Ansteuersignal berechnet, wird ein Aktionspotential an der berechneten efferenten Zelle (bzw. an den berechneten efferenten Zellen) ausgelöst und somit die äußere Haarzelle zur Kontraktion angeregt. Es erfolgt somit eine Reizleitung von efferenten Neuronen (bzw. zu efferenten Neuronen, oder unter Verwendung efferenter Neuronen) an äußere Haarzellen.

Die vorliegende Erfindung schafft somit ein Konzept, das zur Erzeugung eines gefilterten Aktivitätsmusters verwendet werden kann, wobei das gefilterte Aktivitätsmuster wiederum vorteilhaft für eine Ansteuerung eines Cochlea-Implantats einsetzbar ist, so dass bei einem Träger des Cochlea-Implantats ein herkömmlicherweise auftretender Cocktail-Party-Effekt verringert wird.

## Patentansprüche

1. Vorrichtung (100; 200) zum Erzeugen eines gefilterten Aktivitätsmusters (146) basierend auf einem ersten Aktivitätsmuster (110) an einem Gehörmodell eines ersten Ohres, und einem zweiten Aktivitätsmuster (112) an einem Gehörmodell eines zweiten Ohres, mit folgenden Merkmalen:

einem Identifizierer (120) zum Identifizieren einer ersten Trajektorie (430, 432, 434) in dem ersten Aktivitätsmuster und einer zweiten Trajektorie (440, 442, 444) in dem zweiten Aktivitätsmuster, die einem gleichen Schallereignis zugeordnet sind;
einem Bestimmer (130) zum Bestimmen, ob die zwei Trajektorien (430, 440; 432, 442; 434, 444) einem Schallereignis einer Nutz-Schallquelle (462) zugeordnet sind; und
einem Filter (140) zum Filtern des ersten Aktivitätsmusters oder des zweiten Aktivitätsmusters basierend auf einem Ergebnis (136) des Bestimmens, ob eine Trajektorie (430, 432, 434, 440, 442, 444) einem Schallereignis der Nutz-Schallquelle zugeordnet ist, so dass in einem gefilterten Aktivitätsmuster Aktivitätsereignisse, die einem Schallereignis der Nutz-Schallquelle zugeordnet sind, dominieren, oder dass die Aktivitätsereignisse, die nicht einem Schallereignis der Nutz-Schallquelle zugeordnet sind, in dem gefilterten Aktivitätsmuster nicht mehr vorhanden sind;
wobei das erste Aktivitätsmuster (110) auf einem durch das Gehörmodell des ersten Ohres verarbeiteten ersten Audiosignal basiert, und wobei das zweite Aktivitätsmuster (112) auf einem durch das Gehörmodell des zweiten Ohrs verarbeiteten zweiten Audiosignal basiert, wobei das erste Aktivitätsmuster und das zweite Aktivitätsmuster zwei Audiosignale von verschiedenen Audiosignalquellen oder Audiosignale von zwei Kanälen eines mehrkanaligen Audiosignals beschreiben,
wobei eine Trajektorie in dem Aktivitätsmuster zusammengehörige Aktivitätsereignisse in dem Aktivitätsmuster beschreibt, die einer Wanderwelle auf einer Basilarmembran des Ohrmodells zugeordnet sind;

wobei der Identifizierer (120) ausgelegt ist, um eine erste gekrümmte Trajektorie (430, 432, 434) in dem ersten Aktivitätsmuster und eine zweite gekrümmte Trajektorie (440,442, 444) in dem zweiten Aktivitätsmuster als zu einem gleichen Schallereignis gehörige Trajektorien zu identifizieren, wenn die erste Trajektorie und die zweite Trajektorie innerhalb eines vorgegebenen Toleranzbereichs eine gleiche Krümmung aufweisen, und wenn die erste Trajektorie und die zweite Trajektorie innerhalb eines vorgegebenen maximalen Zeitraums auftreten;

wobei der Identifizierer (120) ausgelegt ist, um eine zeitliche Verschiebung ($\Delta t_1$, $\Delta t_2$, $\Delta t_3$) zwischen den zwei identifizierten Trajektorien, die dem gleichen Schallereignis zugeordnet sind, zu bestimmen; und

wobei der Bestimmer (130) ausgelegt ist, um anhand der zeitlichen Verschiebung festzustellen, ob die zwei identifizierten Trajektorien (430, 440; 432, 442; 434, 444), die dem gleichen Schallereignis zugeordnet sind, einem Schallereignis einer Nutz-Schallquelle (462) zugeordnet sind.

2. Vorrichtung (100; 200) gemäß Anspruch 1, bei dem das erste Ohr ein linkes Ohr ist, und bei dem das zweite Ohr ein rechtes Ohr ist, oder umgekehrt,

wobei das erste Aktivitätsmuster (110) auf einem durch das Gehörmodell des ersten Ohres verarbeiteten ersten Audiosignal basiert, und wobei das zweite Aktivitätsmuster (112) auf einem durch das Gehörmodell des zweiten Ohrs verarbeiteten zweiten Audiosignal basiert,

wobei das erste Audiosignal ein in einer Umgebung des ersten Ohres wahrnehmbares Audiosignal beschreibt, und wobei das zweite Audiosignal ein in einer Umgebung des zweiten Ohres wahrnehmbares Audiosignal beschreibt, und wobei das erste Aktivitätsmuster (110) ein Nervenaktivitätsmuster über der Zeit an einer Mehrzahl von Nervenfasern (NF1, NF2, NF3, NF4) des Gehörmodells des ersten Ohres, das eine Reaktion auf der Mehrzahl von Nervenfasern bei Vorliegen eines ersten Audiosignals an dem ersten Ohr beschreibt, ist, bei dem das zweite Aktivitätsmuster (112) ein Nervenaktivitätsmuster über der Zeit an einer Mehrzahl von Nervenfasern des Gehörmodells des zweiten Ohres, das eine Reaktion auf der Mehrzahl von Nervenfasern bei Vorliegen eines zweiten Audiosignals an dem zweiten Ohr beschreibt, ist, und bei dem das gefilterte Aktivitätsmuster ein gefiltertes Nervenaktivitätsmuster ist.

3. Vorrichtung (100; 200) gemäß einem der Ansprüche 1 oder 2, bei dem das erste Aktivitätsmuster ein Neurotransmitter-Vesikel-Auftreten an inneren Hörzellen (IHZ1, IHZ2, IHZ3, IHZ4) des Gehörmodells des ersten Ohres, das eine Reaktion bei Vorliegen eines ersten Audiosignals an dem ersten Ohr beschreibt, ist,

bei dem das zweite Aktivitätsmuster ein Neurotransmitter-Vesikel-Auftreten an inneren Hörzellen des Gehörmodells des zweiten Ohres, das eine Reaktion auf ein Vorliegen eines zweiten Audiosignals an dem zweiten Ohr beschreibt, ist, und

bei dem das gefilterte Aktivitätsmuster ein gefiltertes Neurotransmitter-Vesikel-Auftreten ist.

4. Vorrichtung (100; 200) gemäß einem der Ansprüche 1 bis 3, bei der der Identifizierer (120) ausgelegt ist, um eine zeitliche Verschiebung ($\Delta t_1$, $\Delta t_2$, $\Delta t_3$) zwischen den zwei identifizierten Trajektorien, die dem gleichen Schallereignis zugeordnet sind, zu bestimmen, und bei der der Bestimmer (130) ausgelegt ist, um anzuzeigen, dass die zwei identifizierten Trajektorien einem Schallereignis der Nutz-Schallquelle (462) zugeordnet sind, wenn eine zeitliche Verschiebung zwischen den zwei identifizierten Trajektorien innerhalb eines fest vorgegebenen oder einstellbaren Bereichs liegt, und um anderenfalls anzuzeigen, dass die zwei identifizierten Trajektorien nicht einem Schallereignis der Nutz-Schallquelle zugeordnet sind.

5. Vorrichtung (100; 200) gemäß einem der Ansprüche 1 bis 4, bei dem das erste Aktivitätsmuster (110) ein zweidimensionales Muster ist, das durch zeitliche Verläufe (302, 310, 315, 320; 342, 360, 365, 370) von Signalen einer ersten Mehrzahl von Signalen (392, 394, 396, 398; 510; 710) beschrieben wird, wobei die Signale der ersten Mehrzahl von Signalen verschiedenen Hörnerven oder inneren Hörzellen des Gehörmodells des ersten Ohres zugeordnet sind, und wobei die zeitlichen Verläufe der Signale der ersten Mehrzahl von Signalen Verläufe einer charakteristischen Größe an den Hörnerven oder an den inneren Hörzellen oder in den inneren Hörzellen des Gehörmodells des ersten Ohres beschreiben;

bei dem das zweite Aktivitätsmuster (112) ein zweidimensionales Muster ist, das durch zeitliche Verläufe (302, 310, 315, 320; 342, 360, 365, 370) von Signalen einer zweiten Mehrzahl von Signalen (392, 394, 396, 398; 512; 720) beschrieben wird, wobei die Signale der zweiten Mehrzahl von Signalen verschiedenen Hörnerven oder inneren Hörzellen des Gehörmodells des zweiten Ohres zugeordnet sind, und wobei die zeitlichen Verläufe der Signale der zweiten Mehrzahl von Signalen Verläufe einer charakteristischen Größe an den Hörnerven oder an den inneren Hörzellen oder in den inneren Hörzellen des Gehörmodells des zweiten Ohres beschreiben, und

wobei der Identifizierer (120) ausgelegt ist, um Trajektorien zumindest näherungsweise gleicher Krümmung in dem ersten Aktivitätsmuster (110) und in dem zweiten Aktivitätsmuster(112) zu erkennen,

wobei der Identifizierer ausgelegt ist, um das erste Aktivitätsmuster und das zweite Aktivitätsmuster im Hinblick auf eine Zeitrichtung gegeneinander zu verschieben, um Orte zu erkennen, an denen das erste Aktivitätsmuster und das zweite Aktivitätsmuster für verschiedene Verschiebungs-Zustände Koinzidenzen aufweisen, und um basierend auf den für die verschiedenen Verschiebungs-Zustände erkannten Orten der Koinzidenzen zu entscheiden, ob eine Trajektorie in dem ersten Aktivitätsmuster und eine Trajektorie in dem zweiten Aktivitätsmuster eine gleiche Krümmung aufweisen, und, falls ja, um basierend auf den erkannten Orten der Koinzidenzen eine zeitliche Verschiebung der Trajektorien in dem ersten Aktivitätsmuster und der Trajektorien in dem zweiten Aktivitätsmuster zu erkennen.

6. Vorrichtung (100; 200) gemäß Anspruch 5, bei der der Identifizierer (120) einen Aktivitätsmuster-Schieber (500) umfasst, der ausgelegt ist, um das erste Aktivitätsmuster (110) in Form einer Mehrzahl von parallelen Zeitsignalen (510) parallel zu empfangen und getaktet oder zeitkontinuierlich in einer ersten Richtung zu verschieben, und der ferner ausgelegt ist, um das zweite Aktivitätsmuster (112) in Form einer Mehrzahl von parallelen Zeitsignalen (512) parallel zu empfangen und getaktet oder zeitkontinuierlich in einer zweiten Richtung, die der ersten Richtung entgegengesetzt ist, zu verschieben,

wobei der Aktivitätsmuster-Schieber eine erste Mehrzahl von parallelen Zweigen umfasst, die ausgelegt sind, um das erste Aktivitätsmuster zu verschieben, und wobei der Aktivitätsmuster-Schieber ferner eine zweite Mehrzahl von parallelen Zweigen umfasst, die ausgelegt sind, um das zweite Aktivitätsmuster zu verschieben,

wobei eine Zuordnung zwischen einem Zweig der ersten Mehrzahl von parallelen Zweigen und einem Zweig der zweiten Mehrzahl von parallelen Zweigen besteht,

wobei eine Zuordnung zwischen einzelnen Positionen entlang eines ersten betrachteten Zweigs der ersten Mehrzahl von parallelen Zweigen und Positionen entlang eines zugehörigen zweiten betrachteten Zweigs der zweiten Mehrzahl von parallelen Zweigen besteht, derart, dass ein Aktivitätsereignis, das dem ersten betrachteten Zweig zugeführt wird, die einander zugeordneten Positionen im Verlauf der Zeit in einer ersten Reihenfolge durchläuft, und dass ein Aktivitätsereignis, das dem zweiten betrachteten Zweig zugeführt wird, die einander zugeordneten Positionen im Verlauf der Zeit in einer zweiten Reihenfolge, die der ersten Reihenfolge entgegengesetzt ist, durchläuft,

wobei der Identifizierer ferner eine Koinzidenz-Erkennungseinrichtung aufweist, die ausgelegt ist, um für eine Mehrzahl von einander zugeordneten parallelen Zweigen und für eine Mehrzahl von einander zugeordneten Positionen innerhalb der einander zugeordneten parallelen Zweige zu erkennen, wenn an zwei einander zugeordneten Positionen gleichzeitig zwei Aktivitätsereignisse vorliegen, und

wobei der Identifizierer ferner eine Auswerteeinrichtung umfasst, die ausgelegt ist, um basierend auf der von der Koinzidenz-Erfassungseinrichtung gelieferten Information zu erkennen, wenn zwei Trajektorien, die einem gleichen Schallereignis zugeordnet sind, in dem ersten Aktivitätsmuster und in dem zweiten Aktivitätsmuster enthalten sind, und

wobei die Auswerteeinrichtung ausgelegt ist, um für eine Mehrzahl von Positionen in Verschiebungsrichtung der Aktivitätsmuster getrennt zu bestimmen, wie viele Koinzidenzen von Aktivitätsereignissen in dem ersten Aktivitätsmuster (110) und Aktivitätsereignissen in dem zweiten Aktivitätsmuster (112) für eine Mehrzahl von einander zugeordneten parallelen Zweigen in einem vorgegebenen Zeitraum insgesamt aufgetreten sind, um basierend darauf eine erste Trajektorie in dem ersten Aktivitätsmuster und eine zweite Trajektorie in dem zweiten Aktivitätsmuster zu identifizieren, die einem gleichen Schallereignis zugeordnet sind, und um ferner eine zeitliche Verschiebung zwischen den zwei Trajektorien, die dem gleichen Schallereignis zugeordnet sind, zu bestimmen.

7. Vorrichtung (100; 200) gemäß Anspruch 6, bei der der Identifizierer (120) ausgelegt ist, um basierend auf einer Information, wie viele Koinzidenzen von Aktivitätsereignissen in dem ersten Aktivitätsmuster und in dem zweiten Aktivitätsmuster für eine Mehrzahl von einander zugeordneten parallelen Zweigen an einer betrachteten Position entlang der Verschiebungsrichtung der Aktivitätsmuster in einem vorgegebenen Zeitraum insgesamt aufgetreten sind, zu erkennen, dass eine erste Trajektorie und eine zweite Trajektorie, die einem gleichen Schallereignis zugeordnet sind, in dem ersten Aktivitätsmuster und dem zweiten Aktivitätsmuster vorliegen, wenn die numerische Information ein Auftreten von zumindest einer vorgegebenen MindestAnzahl von Koinzidenzen anzeigt, und um basierend auf der betrachteten Position entlang der Verschiebungsrichtung der Aktivitätsmuster die zeitliche Verschiebung zwischen den zwei Trajektorien, die dem gleichen Schallereignis zugeordnet sind, zu bestimmen.

8. Vorrichtung (100; 200) gemäß Anspruch 5, bei dem der Identifizierer (120) einen Trajektorien-Erkenner (730, 732; 15000; 17000) umfasst, und ferner ausgelegt ist, um das erste Aktivitätsmuster (110) und das zweite Aktivitätsmuster (112) dem Trajektorien-Erkenner zuzuführen,

wobei der Trajektorien-Erkenner ausgelegt ist, um basierend auf dem ersten Aktivitätsmuster eine Information (740, 742, 744, 746) über eine in dem ersten Aktivitätsmuster erkannte Trajektorie zu liefern, wobei die Information über die in dem ersten Aktivitätsmuster erkannte Trajektorie eine zeitliche Lage und eine Krümmung der erkannten Trajektorie beschreibt;

wobei der Trajektorien-Erkenner ferner ausgelegt ist, um basierend auf dem zweiten Aktivitätsmuster eine Information (750, 752, 754, 756) über eine in dem zweiten Aktivitätsmuster erkannte Trajektorie zu liefern, wobei die Information über die in dem zweiten Aktivitätsmuster erkannte Trajektorie eine zeitliche Lage und eine Krümmung der erkannten Trajektorie beschreibt;

wobei der Identifizierer ausgelegt ist, um basierend auf der Information über die in dem ersten Aktivitätsmuster erkannte Trajektorie und der Information über die in dem zweiten Aktivitätsmuster erkannte Trajektorie zu bestimmen, ob die erste erkannte Trajektorie und die zweite erkannte Trajektorie einem gleichen Schallereignis zugeordnet sind; und

wobei der Identifizierer ferner ausgelegt ist, um basierend auf der Information über die in dem ersten Aktivitätsmuster erkannte Trajektorie und über die in dem zweiten Aktivitätsmuster erkannte Trajektorie eine zeitliche Verschiebung der ersten erkannten Trajektorie und der zweiten erkannten Trajektorie zu bestimmen.

9. Vorrichtung (100; 200) gemäß einem der Ansprüche 5 bis 8, bei der der Identifizierer (120) eine Schiebeeinrichtung (500) aufweist, die ausgelegt ist, um das erste Aktivitätsmuster (110) und das zweite Aktivitätsmuster (112) parallel in gegenläufiger Richtung durch ein Feld von Schieberegisterzellen (580) zu verschieben,

wobei das Feld von Schieberegisterzellen eine Mehrzahl von Reihen und eine Mehrzahl von Spalten aufweist;

wobei das Feld von Schieberegisterzellen ausgelegt ist, um das erste Aktivitätsmuster als eine Mehrzahl (510) von parallelen Zeilensignalen als Eingangssignal einer ersten Spalte zu empfangen, und um das zweite Aktivitätsmuster als eine Mehrzahl von parallelen Zeilensignalen als Eingangssignal einer letzten Spalte zu empfangen;

wobei eine Schieberegisterzelle in einer betrachteten Zeile und in einer betrachteten Spalte ausgelegt ist, um einen Informationswert des ersten Aktivitätsmusters, der einen aktiven Zustand und einen inaktiven Zustand annehmen kann, und ein Informationswert des zweiten Aktivitätsmusters, der einen aktiven Zustand und einen inaktiven Zustand annehmen kann, zu speichern, um den zu dem ersten Aktivitätsmuster gehörigen Informationswert an eine benachbarte Schieberegisterzelle der betrachteten Zeile und einer ersten benachbarten Spalte weiterzugeben, und um den zu dem zweiten Aktivitätsmuster gehörigen Informationswert an eine benachbarte Schieberegisterzelle der betrachteten Zeile und einer zweiten benachbarten Spalte weiterzugeben;

wobei eine Schieberegisterzelle ferner ausgelegt ist, um ein Koinzidenz-Ereignis zu erkennen, wenn in der Schieberegisterzelle vorliegende Informationswerte, die zu dem ersten Aktivitätsmuster und dem zweiten Aktivitätsmuster gehören, gleichzeitig einen aktiven Zustand anzeigen; und

wobei der Identifizierer ferner ausgelegt ist, um für die Mehrzahl von Spalten separat zu ermitteln, wie viele Koinzidenz-Ereignisse innerhalb eines vorgegebenen Zeitintervalls in einer betrachteten Spalte aufgetreten sind, und um basierend darauf zu bestimmen, ob, und falls ja, mit welcher zeitlichen Verschiebung, in den Aktivitätsmustern Trajektorien aufgetreten sind, die einem gleichen Schallereignis zugeordnet sind, und

wobei der erste Identifizierer (120) eine Mehrzahl von Zählern, Addierern oder Integrierern umfasst, die Spalten des Feldes zugeordnet sind, und die ausgelegt sind, um zu ermitteln, wie viele Koinzidenz-Ereignisse in dem betrachteten Zeitintervall in den Spalten aufgetreten sind.

10. Vorrichtung (100; 200) gemäß einem der Ansprüche 8 bis 9, bei dem der Trajektorien-Erkenner eine Einrichtung zur Mustererkennung umfasst, die ausgelegt ist, um in einer zweidimensionalen Darstellung, die durch das Aktivitätsmuster über der Zeit gebildet wird, ein gerades oder gekrümmtes linienförmiges Muster als eine Trajektorie zu erkennen, die zeitliche Lage der Trajektorie zu ermitteln und eine zu der Trajektorie gehörige Zeitinformation zu liefern,

wobei die Einrichtung zur Mustererkennung ausgelegt ist, um eine zweidimensionale Darstellung des Aktivitätsmusters über der Zeit schrittweise zu verzerren, um eine verzerrte zweidimensionale Darstellung des Aktivitätsmusters über der Zeit zu erhalten, um zu erkennen, wenn in der verzerrten zweidimensionalen Darstellung des Aktivitätsmusters über der Zeit eine näherungsweise gerade Linie enthalten ist, um die näherungsweise gerade Linie als eine Trajektorie zu erkennen, um die zeitliche Lage der Trajektorie zu ermitteln, und um die zu der Trajektorie gehörige Zeitinformation zu liefern,

wobei die Einrichtung zur Mustererkennung ausgelegt ist, um die zweidimensionale Darstellung des Nervenaktivitätsmusters über der Zeit schrittweise derart zu verzerren, dass eine gekrümmte Trajektorie in dem Nervenaktivitätsmuster durch das schrittweise Verzerren schrittweise gerade gebogen wird, wobei eine Anzahl von Verzerrungsschritten, die für ein Geradebiegen der gekrümmten Trajektorie benötigt werden, von einer Krümmung der gekrümmten Trajektorie abhängig sind, und wobei eine Anzahl der Verzerrungsschritte, die für das Geradebiegen der gekrümmten Trajektorie benötigt werden, eine Aussage über eine ursprüngliche Form der Trajektorie umfasst,

wobei die Einrichtung zur Mustererkennung eine Kurvenerkennungseinrichtung umfasst, die ausgelegt ist, um das Nervenaktivitätsmuster in Form einer Mehrzahl von Signalen parallele zu empfangen, um die Signale unterschiedlich schnell parallel durch eine Mehrzahl von hintereinander geschalteten Stufen weiterzuleiten, wobei mindestens eine vorbestimmte Stufe eine Schwellerkennungseinrichtung aufweist, die ausgelegt ist, um zu erkennen, wenn mindestens eine vorgegebene Anzahl an Signalen in der vorbestimmten Stufe gleichzeitig aktiv sind, und

wobei mindestens eine Stufe ausgelegt ist, um mehrere Signale bei einer Weiterleitung durch die Stufe unterschiedlich stark zu verzögern.

11. Vorrichtung gemäß einem der Ansprüche 1 bis 10, wobei die Vorrichtung ausgelegt ist, um als das erste Aktivitätsmuster ein Aktivitätsmuster in oder an inneren Hörzellen einer ersten Ansprechempfindlichkeit an dem Gehörmodell des ersten Ohres zu empfangen, um als das zweite Aktivitätsmuster ein Aktivitätsmuster in oder an inneren Hörzellen der ersten Ansprechempfindlichkeit an dem Gehörmodell des zweiten Ohres zu empfangen, um ein drittes Aktivitätsmuster in oder an inneren Hörzellen einer zweiten Ansprechempfindlichkeit an dem Gehörmodell des ersten Ohres zu empfangen, und um ein viertes Aktivitätsmuster in oder an inneren Hörzellen der zweiten Ansprechempfindlichkeit an dem Gehörmodell des zweiten Ohres zu empfangen, wobei der Identifizierer (120) ausgelegt ist, um eine dritte Trajektorie in dem dritten Aktivitätsmuster und eine vierte Trajektorie in dem vierten Aktivitätsmuster, die einem gleichen Schallereignis zugeordnet sind, zu identifizieren; wobei der Bestimmer ausgelegt ist, um zu bestimmen, ob die erste Trajektorie, die zweite Trajektorie, die dritte Trajektorie und die vierte Trajektorie einem Schallereignis einer Nutz-Schallquelle (462) zugeordnet sind, wobei der Bestimmer ausgelegt ist, um basierend auf einem Ergebnis eines Vergleichs, ob in dem in dem ersten Aktivitätsmuster und in dem dritten Aktivitätsmuster innerhalb eines vorgegebenen zeitlichen Toleranzbereichs Trajektorien mit innerhalb eines vorgegebenen Krümmungs-Toleranzbereichs gleicher Krümmung vorliegen, eine Lautstärkeinformation für die Trajektorien und damit für die den Trajektorien zugeordneten Aktivitätsereignisse zu ermitteln, und um die Information darüber, ob die identifizierten Trajektorien einem Nutz-Schallereignis zugeordnet sind, basierend auf der Lautstärkeinformation zu ermitteln.

12. Quellentrenner (1300) zum Erzeugen eines bereinigten Audiosignals (1330) basierend auf einem Audiosignal mit zumindest zwei Kanälen, mit folgenden Merkmalen:

einem Aktivitätsmuster-Erzeuger (1340, 1342) zum Erzeugen eines ersten Aktivitätsmusters (110) an einem Gehörmodell eines ersten Ohres basierend auf einem ersten Kanal (1310) des Audiosignals, und zum Erzeugen eines zweiten Aktivitätsmusters (112) an einem Gehörmodell eines zweiten Ohres basierend auf einem zweiten Kanal (1320) des Audiosignals; einer Vorrichtung (100; 200) zum Erzeugen eines gefilterten Aktivitätsmusters (146) basierend auf dem ersten Aktivitätsmuster (110) und dem zweiten Aktivitätsmuster (112), gemäß einem der Ansprüche 1 bis 11; einem Synthesizer (1350) zum Wandeln des gefilterten Aktivitätsmusters (146) in eine Zeit-Darstellung, eine Frequenz-Darstellung oder eine Subband-Darstellung, um das bereinigte Audiosignal (1330) zu erhalten.

13. Verfahren (1200) zum Erzeugen eines gefilterten Aktivitätsmusters (146) basierend auf einem ersten Aktivitätsmuster (110) an einem Gehörmodell eines ersten Ohres und einem zweiten Aktivitätsmuster (112) an einem Gehörmodell eines zweiten Ohres, mit folgenden Schritten:

Identifizieren (1210) einer ersten Trajektorie (430, 432, 434) in dem ersten Aktivitätsmuster (110), und einer zweiten Trajektorie (440, 442, 444) in dem zweiten Aktivitätsmuster (112), die gleichen Schallereignissen zugeordnet sind; Bestimmen einer zeitlichen Verschiebung zwischen den zwei identifizierten Trajektorien, die dem gleichen Schallereignis zugeordnet sind; Bestimmen (1220), ob die zwei Trajektorien (430, 440; 432, 442; 434, 444) einem Schallereignis einer Nutz-Schallquelle (462) zugeordnet sind; und Filtern (1230) des ersten Aktivitätsmusters oder des zweiten Aktivitätsmusters basierend auf einem Ergebnis (136) des Bestimmens (1220), ob eine Trajektorie einem Schallereignis der Nutz-Schallquelle (462) zugeordnet ist, so dass in einem gefilterten Aktivitätsmuster Aktivitätsereignisse, die einem Schallereignis der Nutz-Schallquelle zugeordnet sind, gegenüber Aktivitätsereignissen, die nicht einem Schallereignis der Nutz-Schallquelle zugeordnet sind, dominieren, oder dass die Aktivitätsereignisse, die nicht einem Schallereignis der Nutz-Schallquelle zugeordnet sind, in dem gefilterten Aktivitätsmuster nicht mehr vorhanden sind; wobei das erste Aktivitätsmuster (110) auf einem durch das Gehörmodell des ersten Ohres verarbeiteten ersten Audiosignal basiert, und wobei das zweite Aktivitätsmuster (112) auf einem durch das Gehörmodell des zweiten Ohrs verarbeiteten zweiten Audiosignal basiert; wobei das erste Aktivitätsmuster und das zweite Aktivitätsmuster zwei Audiosignale von verschiedenen Audiosignalquellen oder Audiosignale von zwei Kanälen eines mehrkanaligen Audiosignals beschreiben; wobei eine Trajektorie in dem Aktivitätsmuster zusammengehörige Aktivitätsereignisse in dem Aktivitätsmuster beschreibt, die einer Wanderwelle auf einer Basilarmembran des Ohrmodells zugeordnet sind;

wobei das Identifizieren einer ersten Trajektorie und einer zweiten Trajektorie ein Identifizieren einer ersten gekrümmten Trajektorie in dem ersten Aktivitätsmuster und einer zweiten gekrümmten Trajektorie in dem zweiten Aktivitätsmuster als zu einem gleichen Schallereignis gehörige Trajektorien umfasst, wenn die erste Trajektorie und die zweite Trajektorie innerhalb eines vorgegebenen Toleranzbereichs eine gleiche Krümmung aufweisen; und

wobei das Bestimmen, ob die zwei identifizierten Trajektorien (430, 440; 432, 442; 434, 444) einem Schallereignis einer Nutz-Schallquelle (462) zugeordnet sind, anhand der zeitlichen Verschiebung erfolgt.

**14.** Verfahren (1400) zum Erzeugen eines bereinigten Audiosignals (1330) basierend auf einem Audiosignal mit zumindest zwei Kanälen, mit folgenden Merkmalen:

Erzeugen (1410) eines ersten Aktivitätsmusters (110) an einem Gehörmodell eines ersten Ohres basierend auf einem ersten Kanal (1310) des Audiosignals, und Erzeugen (1420) eines zweiten Aktivitätsmusters (112) an einem Gehörmodell eines zweiten Ohres basierend auf einem zweiten Kanal (1320) des Audiosignals;
Erzeugen eines gefilterten Aktivitätsmusters (146) basierend auf dem ersten Aktivitätsmuster (110) und dem zweiten Aktivitätsmuster (112), gemäß Anspruch 13; und
Wandeln (1440) des gefilterten Aktivitätsmusters in eine Zeit-Darstellung, Frequenz-Darstellung oder Subband-Darstellung, um das bereinigte Audiosignal (1330) zu erhalten.

**15.** Computerprogramm zum Durchführen eines Verfahrens gemäß Anspruch 13 oder 14, wenn das Computerprogramm auf einem Computer abläuft.

## Claims

**1.** Apparatus (100; 200) for generating a filtered activity pattern (146) on the basis of a first activity pattern (110) at an auditory model of a first ear, and of a second activity pattern (112) at an auditory model of a second ear, the apparatus comprising:

an identifier (120) for identifying a first trajectory (430, 432, 434) within the first activity pattern, and a second trajectory (440, 442, 444) within the second activity pattern, which are associated with a same sound event;
a determiner (130) for determining whether the two trajectories (430, 440; 432; 442; 434; 444) are associated with a sound event of a useful sound source (462); and
a filter (140) for filtering the first activity pattern or the second activity pattern on the basis of a result (136) of determining whether a trajectory (430, 432, 434, 440, 442, 444) is associated with a sound event of the useful sound source, so that activity events which are associated with a sound event of the useful sound source will dominate within a filtered activity pattern, or that the activity events which are not associated with a sound event of the useful sound source no longer exist within the filtered activity pattern;
wherein the first activity pattern (110) is based on a first audio signal processed by the auditory model of the first ear, and wherein the second activity pattern (112) is based on a second audio signal processed by the auditory model of the second ear,
wherein the first activity pattern and the second activity pattern describe two audio signals of different audio signal sources or audio signals of two channels of a multichannel audio signal,
wherein a trajectory within the activity pattern describes activity events within the activity pattern which belong together and are associated with a traveling wave on a basilar membrane of the ear model;
wherein the identifier (120) is designed to identify a first curved trajectory (430, 432, 434) within the first activity pattern and a second curved trajectory (440, 442, 444) within the second activity pattern as trajectories belonging to a same sound event if the first trajectory and the second trajectory comprise a same curvature within a predefined tolerance range and if the first trajectory and the second trajectory occur within a predefined maximum period of time;
wherein the identifier (120) is designed to determine a time shift ($\Delta t1$, $\Delta t2$, $\Delta t3$) between the two identified trajectories associated with the same sound event; and
wherein the determiner (130) is designed to ascertain by means of the time shift whether the two identified trajectories (430, 440; 432, 442; 434, 444) associated with the same sound event are associated with a sound event of a useful sound source (462).

**2.** Apparatus (100; 200) as claimed in claim 1, wherein the first ear is a left-hand ear, and wherein the second ear is a right-hand ear, or vice-versa,

wherein the first activity pattern (110) is based on a first audio signal processed by the auditory model of the first ear, and wherein the second activity pattern (112) is based on a second audio signal processed by the auditory model of the second ear,

wherein the first audio signal describes an audio signal perceivable in the surroundings of the first ear, and wherein the second audio signal describes an audio signal perceivable in the surroundings of the second ear, and wherein the first activity pattern (110) is a nerve activity pattern over time at a plurality of nerve fibers (NF1, NF2, NF3, NF4) of the auditory model of the first ear which describes a reaction at the plurality of nerve fibers when a first audio signal is present at the first ear, wherein the second activity pattern (112) is a nerve activity pattern over time at a plurality of nerve fibers of the auditory model of the second ear which describes a reaction at the plurality of nerve fibers when a second audio signal is present at the second ear, and

where within the filtered activity pattern is a filtered nerve activity pattern.

3. Apparatus (100; 200) as claimed in one of claims 1 or 2, wherein the first activity pattern is a neurotransmitter vesicle occurrence at internal auditory cells (IHZ1, IHZ2, IHZ3, IHZ4) of the auditory model of the first ear which describes a reaction when a first audio signal exists at the first ear,

wherein the second activity pattern is a neurotransmitter vesicle occurrence at internal auditory cells of the auditory model of the second ear which describes a reaction to an existence of a second audio signal at the second ear, and where within the filtered activity pattern is a filtered neurotransmitter vesicle occurrence.

4. Apparatus (100; 200) as claimed in any of claims 1 to 3, wherein the identifier (120) is designed to determine a time shift ($\Delta t1$, $\Delta t2$, $\Delta t3$) between the two identified trajectories associated with the same sound event, and wherein the determiner (130) is designed to indicate that the two identified trajectories are associated with a sound event of the useful sound source (462) if a time shift between the two identified trajectories is within a fixedly predefined or adjustable range, and otherwise to indicate that the two identified trajectories are not associated with a sound event of the useful sound source.

5. Apparatus (100; 200) as claimed in any of claims 1 to 4, wherein the first activity pattern (110) is a two-dimensional pattern described by time waveforms (302, 310, 315, 320; 342, 360, 365, 370) of signals of a first plurality of signals (392, 394, 396, 398; 510; 710), the signals of the first plurality of signals being associated with different auditory nerves or internal auditory cells of the auditory model of the first ear, and the time waveforms of the signals of the first plurality of signals describing waveforms of a characteristic size at the auditory nerves or at the internal auditory cells or within the internal auditory cells of the auditory model of the first ear;

wherein the second activity pattern (112) is a two-dimensional pattern described by time waveforms (302, 310, 315, 320; 342, 360, 365, 370) of signals of a second plurality of signals (392, 394, 396, 398; 512; 720), the signals of the second plurality of signals being associated with different auditory nerves or internal auditory cells of the auditory model of the second ear, and the time waveforms of the signals of the second plurality of signals describing waveforms of a characteristic size at the auditory nerves or at the internal auditory cells or within the internal auditory cells of the auditory model of the second ear, and

wherein the identifier (120) is designed to detect trajectories of at least approximately identical curvatures within the first activity pattern (110) and within the second activity pattern (112),

wherein the identifier is designed to mutually shift the first activity pattern and the second activity pattern with regard to a time direction to detect locations where the first activity pattern and the second activity pattern have coincidences for different shifting states, and to decide, on the basis of the locations of the coincidences which are detected for the various shifting states, whether a trajectory within the first activity pattern and a trajectory within the second activity pattern have a same curvature, and if this is so, to detect, on the basis of the detected locations of the coincidences, a time shift of the trajectories within the first activity pattern and of the trajectories within the second activity pattern.

6. Apparatus (100; 200) as claimed in claim 5, wherein the identifier (120) comprises an activity pattern shifter (500) which is adapted to receive the first activity pattern (110) in the form of a plurality of parallel time signals (510) in parallel and to shift it in a first direction in a clocked or time-continuous manner, and which further is designed to receive the second activity pattern (112) in the form of a plurality of parallel time signals (512) in parallel and to shift it in a second direction, which is opposite the first direction, in a clocked or time-continuous manner,

wherein the activity pattern shifter comprises a first plurality of parallel branches designed to shift the first activity pattern, and wherein the activity pattern shifter further comprises a second plurality of parallel branches designed to shift the second activity pattern,

wherein there is an association between a branch of the first plurality of parallel branches and a branch of the second plurality of parallel branches,

wherein an association between individual positions along a first branch contemplated among the first plurality of parallel branches, and positions along an associated second branch contemplated among the second plurality of parallel branches exists such that an activity event supplied to the first branch contemplated passes the mutually associated positions in the course of time in a first sequence, and that an activity event supplied to the second branch contemplated passes the mutually associated positions in the course of time in a second sequence, which is opposed to the first sequence,

wherein the identifier further comprises a coincidence detection means designed to detect, for a plurality of mutually associated parallel branches and for a plurality of mutually associated positions within the mutually associated parallel branches, when two activity events are present at the same time at two mutually associated positions, and

wherein the identifier further comprises an evaluation means designed to detect, on the basis of the information provided by the coincidence detection means, if two trajectories which are associated with a same sound event are contained within the first activity pattern and within the second activity pattern, and

wherein the evaluation means is designed to separately determine, for a plurality of positions in the shifting direction of the activity patterns, how many coincidences of activity events within the first activity pattern (110) and of activity events within the second activity pattern (112) have occurred in total for a plurality of mutually associated parallel branches within a predefined period of time, to identify, on the basis thereof, a first trajectory within the first activity pattern and a second trajectory within the second activity pattern which are associated with a same sound event, and to further determine a time shift between the two trajectories associated with the same sound event.

7. Apparatus (100; 200) as claimed in claim 6, wherein the identifier (120) is designed to detect, on the basis of information about how many coincidences of activity events within the first activity pattern and within the second activity pattern have occurred in total for a plurality of mutually associated parallel branches at a contemplated position along the shifting direction of the activity patterns within a predefined period of time, that a first trajectory and a second trajectory, which are associated with a same sound event, are present within the first activity pattern and the second activity pattern when the numerical information indicates an occurrence of at least a predefined minimum number of coincidences, and to determine, on the basis of the contemplated position along the shifting direction of the activity patterns, the time shift between the two trajectories associated with the same sound event.

8. Apparatus (100; 200) as claimed in claim 5, wherein the identifier (120) comprises a trajectory detector (730, 732; 15000; 17000) and is further designed to supply the first activity pattern (110) and the second activity pattern (112) to the trajectory detector,

wherein the trajectory detector is designed to provide, on the basis of the first activity pattern, information (740, 742, 744, 746) on a trajectory detected within the first activity pattern, the information on the trajectory detected within the first activity pattern describing a temporal location and a curvature of the trajectory detected;

wherein the trajectory detector is further designed to provide, on the basis of the second activity pattern, information (750, 752, 754, 756) on a trajectory detected within the second activity pattern, the information on the trajectory detected within the second activity pattern describing a temporal location and a curvature of the trajectory detected;

wherein the identifier is designed to determine, on the basis of the information on the trajectory detected within the first activity pattern and of the information on the trajectory detected within the second activity pattern, whether the first trajectory detected and the second trajectory detected are associated with a same sound event; and

wherein the identifier is further designed to determine, on the basis of the information on the trajectory detected within the first activity pattern and on the trajectory detected within the second activity pattern, a time shift of the first trajectory detected and of the second trajectory detected.

9. Apparatus (100; 200) as claimed in any of claims 5 to 8, wherein the identifier (120) comprises a shifting means (500) designed to shift the first activity pattern (110) and the second activity pattern (112) in parallel through an array of shift register cells (580) in opposite directions,

wherein the array of shift register cells comprises a plurality of rows and a plurality of columns;

wherein the array of shift register cells is designed to receive the first activity pattern as a plurality (510) of parallel row signals as an input signal of a first column, and to receive the second activity pattern as a plurality of parallel row signals as an input signal of a last column;

wherein a shift register cell in a row contemplated and in a column contemplated is designed to store an information value of the first activity pattern which may adopt an active state and an inactive state, and an information value of the second activity pattern which may adopt an active state and an inactive state, so as to pass on the information value belonging to the first activity pattern to an adjacent shift register cell of the row contemplated and of a first adjacent column, and to pass on the information value belonging to the second activity pattern to an adjacent shift register cell of the row contemplated and of a second adjacent column;

wherein a shift register cell is further designed to detect a coincidence event when information values which are present within the shift register cell and belong to the first activity pattern and to the second activity pattern indicate an active state at the same time; and

wherein the identifier is further designed to separately determine, for the plurality of columns, how many coincidence events have occurred in a column contemplated within a predefined time interval, and to determine, on the basis thereof, whether, and if so, with which time shift, trajectories associated with a same sound event have occurred within the activity patterns, and

wherein the first identifier (120) comprises a plurality of counters, adders or integrators which are associated with columns of the array and are designed to determine how many coincidence events have occurred in the columns within the time interval contemplated.

10. Apparatus (100; 200) as claimed in any of claims 8 to 9, wherein the trajectory detector comprises a means for pattern detection which is designed to detect, in a two-dimensional representation formed by the activity pattern over time, a straight-line or curved linear pattern as a trajectory, to determine the temporal location of the trajectory, and to provide time information belonging to the trajectory,

wherein the means for pattern detection is designed to distort a two-dimensional representation of the activity pattern over time in a step-by-step fashion so as to obtain a distorted two-dimensional representation of the activity pattern over time, to detect when an approximately straight line is contained within the distorted two-dimensional representation of the activity pattern over time, to detect the approximately straight line as a trajectory, to determine the temporal location of the trajectory, and to provide the time information belonging to the trajectory,

wherein the means for pattern detection is designed to distort the two-dimensional representation of the nerve activity pattern over time in a step-by-step fashion such that a curved trajectory within the nerve activity pattern is straightened in a step-by-step fashion by the step-by-step distortion, a number of distortion steps as are required for straightening the curved trajectory being dependent on a curvature of the curved trajectory, and a number of the distortion steps required for straightening the curved trajectory comprising a statement on an original shape of the trajectory,

wherein the means for pattern detection comprises a curve detection means adapted to receive the nerve activity pattern in the form of a plurality of signals in parallel so as to pass the signals on in parallel at different speeds through a plurality of cascaded stages, at least one predetermined stage comprising a threshold detection means designed to detect when at least a predefined number of signals are active at the same time in the predetermined stage, and

wherein at least one stage is designed to delay several signals to various extents while they are being passed on through the stage.

11. Apparatus as claimed in any of claims 1 to 10, wherein the apparatus is designed to receive, as the first activity pattern, an activity pattern in or on internal auditory cells of a first responsiveness at the auditory model of the first ear, to receive, as the second activity pattern, an activity pattern in or on internal auditory cells of the first responsiveness at the auditory model of the second ear,

to receive a third activity pattern in or on internal auditory cells of a second responsiveness at the auditory model of the first ear, and to receive a fourth activity pattern in or on internal auditory cells of the second responsiveness at the auditory model of the second ear,

wherein the identifier (120) is designed to identify a third trajectory within the third activity pattern and a fourth trajectory within the fourth activity pattern which are associated with a same sound event;

wherein the determiner is designed to determine whether the first trajectory, the second trajectory, the third trajectory, and the fourth trajectory are associated with a sound event of a useful sound source (462),

wherein the determiner is designed to determine, on the basis of a result of a comparison as to whether trajectories having the same curvature within a predefined curvature tolerance range are present within the first activity pattern and within the third activity pattern within a predefined temporal tolerance range, loudness information for the trajectories and, therefore, for the activity events associated with the trajectories,

and to determine the information on whether the identified trajectories are associated with a useful sound event on the basis of the loudness information.

12. Source separator (1300) for generating an adjusted audio signal (1330) on the basis of an audio signal having at least two channels, the source separator (1300) comprising:

an activity pattern generator (1340, 1342) for generating a first activity pattern (110) at an auditory model of a first ear on the basis of a first channel (1310) of the audio signal, and for generating a second activity pattern (112) at an auditory model of a second ear on the basis of a second channel (1320) of the audio signal;

an apparatus (100; 200) for generating a filtered activity pattern (146) on the basis of the first activity pattern (110) and the second activity pattern (112) in accordance with any of claims 1 to 11;

a synthesizer (1350) for converting the filtered activity pattern (146) to a time representation, a frequency representation, or a subband representation so as to obtain the adjusted audio signal (1330).

**13.** Method (1200) for generating a filtered activity pattern (146) on the basis of a first activity pattern (110) at an auditory model of a first ear, and of a second activity pattern (112) at an auditory model of a second ear, the method comprising:

identifying (1210) a first trajectory (430, 432, 434) within the first activity pattern (110), and a second trajectory (440, 442, 444) within the second activity pattern (112), which are associated with same sound events;
determining a time shift between the two identified trajectories that are associated with the same sound event;
determining (1220) whether the two trajectories (430, 440; 432; 442; 434; 444) are associated with a sound event of a useful sound source (462); and
filtering (1230) the first activity pattern or the second activity pattern on the basis of a result (136) of determining (1220) whether a trajectory is associated with a sound event of the useful sound source (462), so that activity events which are associated with a sound event of the useful sound source will dominate, within a filtered activity pattern, over activity patterns which are not associated with a sound event of the useful sound source, or that the activity events which are not associated with a sound event of the useful sound source no longer exist within the filtered activity pattern;
wherein the first activity pattern (110) is based on a first audio signal processed by the auditory model of the first ear, and wherein the second activity pattern (112) is based on a second audio signal processed by the auditory model of the second ear;
wherein the first activity pattern and the second activity pattern describe two audio signals of different audio signal sources or audio signals of two channels of a multichannel audio signal;
wherein a trajectory within the activity pattern describes activity events within the activity pattern which belong together and are associated with a traveling wave on a basilar membrane of the ear model;
wherein identifying a first trajectory and a second trajectory comprises identifying a first curved trajectory within the first activity pattern and a second curved trajectory within the second activity pattern as trajectories belonging to a same sound event if the first trajectory and the second trajectory comprise a same curvature within a predefined tolerance range;
wherein determining whether the two identified trajectories (430, 440; 432, 442; 434, 444) are associated with a sound event of a useful sound source (462) occurs by means of the time shift.

**14.** Method (1400) for generating an adjusted audio signal (1330) on the basis of an audio signal having at least two channels, the method comprising:

generating (1410) a first activity pattern (110) at an auditory model of a first ear on the basis of a first channel (1310) of the audio signal, and generating (1420) a second activity pattern (112) at an auditory model of a second ear on the basis of a second channel (1320) of the audio signal;
generating a filtered activity pattern (146) on the basis of the first activity pattern (110) and the second activity pattern (112) as claimed in claim 13; and
converting (1440) the filtered activity pattern to a time representation, frequency representation, or subband representation so as to obtain the adjusted audio signal (1330).

**15.** Computer program for performing a method as claimed in claims 13 or 14, when the computer program runs on a computer.

**Revendications**

**1.** Dispositif (100; 200) pour générer un modèle d'activité filtré (146) sur base d'un premier modèle d'activité (110) sur un modèle auditif d'une première oreille, et d'un deuxième modèle d'activité (112) sur un modèle auditif d'une deuxième oreille, aux caractéristiques suivantes:

un identificateur (120) destiné à identifier une première trajectoire (430, 432, 434) dans le premier modèle d'activité et une deuxième trajectoire (440, 442, 444) dans le deuxième modèle d'activité, qui sont associées à un même événement sonore;
un déterminateur (130) destiné à déterminer si les deux trajectoires (430, 440; 432, 442; 434, 444) sont associées à un événement sonore d'une source sonore utile (462); et
un filtre (140) destiné à filtrer le premier modèle d'activité ou le deuxième modèle d'activité sur base d'un résultat (136) de la détermination de si une trajectoire (430, 432, 434, 440, 442, 444) est associée à un événement sonore de la source sonore utile, de sorte que dans un modèle d'activité filtré dominent des événements d'activité qui sont associés à un événement sonore de la source sonore utile, ou que les événements d'activité qui ne sont pas associés à un événement sonore de la source sonore utile ne sont plus présents dans le modèle d'activité filtré;

le premier modèle d'activité (110) se basant sur un premier signal audio traité par le modèle auditif de la première oreille, et le deuxième modèle d'activité (112) se basant sur un deuxième signal audio traité par le modèle auditif de la deuxième oreille,

le premier modèle d'activité et le deuxième modèle d'activité décrivant deux signaux audio de différentes sources de signal audio ou des signaux audio de deux canaux d'un signal audio multicanal,

une trajectoire dans le modèle d'activité décrivant des événements d'activité allant ensemble dans le modèle d'activité qui sont associés à une onde progressive sur une membrane basilaire du modèle d'oreille;

l'identificateur (120) étant réalisé de manière à identifier une première trajectoire courbée (430, 432, 434) dans le premier modèle d'activité et une deuxième trajectoire courbée (440, 442, 444) dans le deuxième modèle d'activité comme trajectoires appartenant à un même événement sonore, lorsque la première trajectoire et la deuxième trajectoire présentent, dans une plage de tolérances prédéterminée, une même courbure, et lorsque la première trajectoire et la deuxième trajectoire se produisent dans un laps de temps maximal prédéterminé;

l'identificateur (120) étant réalisé de manière à déterminer un décalage dans le temps ($\Delta t_1$, $\Delta t_2$, $\Delta t_3$) entre les deux trajectoires identifiées qui sont associées au même événement sonore; et

le déterminateur (130) étant réalisé de manière à constater, à l'aide du décalage dans le temps, si les deux trajectoires identifiées (430, 440; 432, 442; 434, 444) qui sont associées au même événement sonore sont associées à un événement sonore d'une source sonore utile (462).

2. Dispositif (100; 200) selon la revendication 1, dans lequel la première oreille est une oreille gauche, et dans lequel la deuxième oreille est une oreille droite, ou inversement,

le premier modèle d'activité (110) se basant sur un premier signal audio traité par le modèle auditif de la première oreille, et le deuxième modèle d'activité (112) se basant sur un deuxième signal audio traité par le modèle auditif de la deuxième oreille,

le premier signal audio décrivant un signal audio perceptible dans un environnement de la première oreille, et le deuxième signal audio décrivant un signal audio perceptible dans un environnement de la deuxième oreille, et le premier modèle d'activité (110) étant un modèle d'activité nerveuse dans le temps sur une pluralité de fibres de nerf (NF1, NF2, NF3, NF4) du modèle auditif de la première oreille qui décrit une réaction à la pluralité de fibres de nerf en cas de présence d'un premier signal audio à la première oreille, dans lequel le deuxième modèle d'activité (112) est un modèle d'activité nerveuse dans le temps sur une pluralité de fibres de nerf du modèle auditif de la deuxième oreille qui décrit une réaction à la pluralité de fibres de nerf en cas de présence d'un deuxième signal audio à la deuxième oreille, et

dans lequel le modèle d'activité filtré est un modèle d'activité nerveuse filtré.

3. Dispositif (100; 200) selon l'une des revendications 1 ou 2, dans lequel le premier modèle d'activité est une présence de vésicules de neurotransmetteur sur des cellules auditives internes (IHZ1, IHZ2, IHZ3, IHZ4) du modèle auditif de la première oreille qui décrit une réaction en cas de présence d'un premier signal audio à la première oreille, dans lequel le deuxième modèle d'activité est une présence de vésicules de neurotransmetteur sur des cellules auditives internes du modèle auditif de la deuxième oreille qui décrit une réaction à une présence d'un deuxième signal audio à la deuxième oreille, et dans lequel le modèle d'activité filtré est une présence de vésicules de neurotransmetteur filtrées.

4. Dispositif (100; 200) selon l'une des revendications 1 à 3, dans lequel l'identificateur (120) est réalisé de manière à déterminer un décalage dans le temps ($\Delta t_1$, $\Delta t_2$, $\Delta t_3$) entre les deux trajectoires identifiées qui sont associées au même événement sonore, et dans lequel le déterminateur (130) est réalisé de manière à indiquer que les deux trajectoires identifiées sont associées à un même événement sonore de la source sonore utile (462) lorsqu'un décalage dans le temps entre les deux trajectoires identifiées se situe dans une plage prédéterminée ou réglable fixe, et à indiquer, dans le cas contraire, que les deux trajectoires identifiées ne sont pas associées à un même événement sonore de la source sonore utile.

5. Dispositif (100; 200) selon l'une des revendications 1 à 4, dans lequel le premier modèle d'activité (110) est un modèle bidimensionnel qui est décrit par des évolutions dans le temps (302, 310, 315, 320; 342, 360, 365, 370) de signaux d'une première pluralité de signaux (392, 394, 396, 398; 510; 710), les signaux de la première pluralité de signaux étant associés à différents nerfs auditifs ou cellules auditives intérieures du modèle auditif de la première oreille, et les évolutions dans le temps des signaux de la première pluralité de signaux décrivant des évolutions d'une grandeur caractéristique aux nerfs auditifs ou aux cellules auditives intérieures ou dans les cellules auditives intérieures du modèle auditif de la première oreille;

dans lequel le deuxième modèle d'activité (112) est un modèle qui est décrit par des évolutions dans le temps (302, 310, 315, 320; 342, 360, 365, 370) de signaux d'une deuxième pluralité de signaux (392, 394, 396, 398; 512; 720), les signaux de la deuxième pluralité de signaux étant associés à différents nerfs auditifs ou cellules auditives intérieures du modèle auditif de la deuxième oreille, et les évolutions dans le temps des signaux de la première pluralité de signaux décrivant des évolutions d'une grandeur caractéristique aux nerfs auditifs ou aux cellules auditives intérieures ou dans les cellules auditives intérieures du modèle auditif de la deuxième oreille, et

l'identificateur (120) étant réalisé de manière à identifier des trajectoires de courbure au moins approximativement identique dans le premier modèle d'activité (110) et dans le deuxième modèle d'activité (112),

l'identificateur étant réalisé de manière à décaler le premier modèle d'activité et le deuxième modèle d'activité l'un par rapport à l'autre en ce qui concerne une direction de temps, pour identifier des endroits auxquels le premier modèle d'activité et le deuxième modèle d'activité présentent des coïncidences pour différents états de décalage, et à décider, sur base des endroits identifiés des coïncidences pour les différents états de décalage, si une trajectoire dans le premier modèle d'activité et une trajectoire dans le deuxième modèle d'activité présentent une courbure identique et, dans l'affirmative, à identifier, sur base des endroits identifiés des coïncidences, un décalage dans le temps des trajectoires dans le premier modèle d'activité et des trajectoires dans le deuxième modèle d'activité.

6. Dispositif (100; 200) selon la revendication 5, dans lequel l'identificateur (120) comporte un décaleur de modèle d'activité (500) qui est réalisé de manière à recevoir en parallèle le premier modèle d'activité (110) sous forme d'une pluralité de signaux dans le temps parallèles (510) et à le décaler de manière cadencée ou continue dans le temps dans une première direction, et qui est par ailleurs réalisé de manière à recevoir en parallèle le deuxième modèle d'activité (112) sous forme d'une pluralité de signaux dans le temps parallèles (512) et à le décaler de manière cadencée ou continue dans le temps dans une deuxième direction qui est opposée à la première direction,

le décaleur de modèle d'activité comportant une première pluralité de branchements parallèles qui sont réalisés de manière à décaler le premier modèle d'activité, et

le décaleur de modèle d'activité comportant par ailleurs une deuxième pluralité de branchements parallèles qui sont réalisés de manière à décaler le deuxième modèle d'activité,

une association existant entre un branchement de la première pluralité de branchements parallèles et un branchement de la deuxième pluralité de branchements parallèles,

une association existant entre des positions individuelles le long d'un premier branchement considéré de la première pluralité de branchements parallèles et des positions le long d'un deuxième branchement considéré s'y rapportant de la deuxième pluralité de branchements parallèles, de sorte qu'un événement d'activité alimenté vers le premier branchement considéré passe par les positions associées les unes aux autres au cours du temps selon un premier ordre, et qu'un événement d'activité alimenté vers le deuxième branchement considéré passe par les positions associées les unes aux autres au cours du temps selon un deuxième ordre qui est opposé au premier ordre,

l'identificateur présentant par ailleurs un moyen d'identification de coïncidence qui est réalisé de manière à identifier, pour une pluralité de branchements parallèles associés l'un à l'autre et pour une pluralité de positions associées l'une à l'autre dans les branchements parallèles associés l'un à l'autre lorsqu'à deux positions associées l'une à l'autre sont simultanément présents deux événements d'activité, et

l'identificateur comportant par ailleurs un moyen d'évaluation qui est réalisé de manière à identifier, sur base de l'information fournie par le moyen de détection de coïncidence, que deux trajectoires associées à un même événement sonore sont contenues dans le premier modèle d'activité et dans le deuxième modèle d'activité, et

le moyen d'évaluation étant réalisé de manière à déterminer séparément pour une pluralité de positions dans le sens du décalage des modèles d'activité le nombre total de coïncidences d'événements d'activité qui se sont produits dans le premier modèle d'activité (110) et d'événements d'activité qui se sont produits dans le deuxième modèle d'activité (112) pour une pluralité de branchements parallèles associés l'un à l'autre dans un laps de temps prédéterminé, pour identifier sur cette base une première trajectoire dans le premier modèle d'activité et une deuxième trajectoire dans le deuxième modèle d'activité qui sont associées à un même événement sonore et, par ailleurs, à déterminer un décalage dans le temps entre les deux trajectoires qui sont associées au même événement sonore.

7. Dispositif (100; 200) selon la revendication 6, dans lequel l'identificateur (120) est réalisé de manière à identifier, sur base d'une information du nombre total de coïncidences d'événements d'activité se sont produits dans le premier modèle d'activité et dans le deuxième modèle d'activité pour une pluralité de branchements parallèles associés l'un à l'autre en une position considérée le long de la direction de décalage des modèles d'activité dans un laps de temps prédéterminé, qu'une première trajectoire et une deuxième trajectoire associées à un même événement sonore sont présentes dans le premier modèle d'activité et le deuxième modèle d'activité lorsque l'information numérique indique la présence d'au moins un nombre minimum prédéterminé de coïncidences, et à déterminer, sur base de la position considérée long de la direction de décalage des modèles d'activité, le décalage dans le temps entre les deux trajectoires associées au même événement sonore.

8. Dispositif (100; 200) selon la revendication 5, dans lequel l'identificateur (120) comporte un identificateur de trajectoires (730, 732; 15000; 17000) et est, par ailleurs, réalisé de manière à alimenter le premier modèle d'activité (110) et le deuxième modèle d'activité (112) vers l'identificateur de trajectoires,

l'identificateur de trajectoires étant réalisé de manière à fournir, sur base du premier modèle d'activité, une information (740, 742, 744, 746) sur une trajectoire identifiée dans le premier modèle d'activité, l'information sur la trajectoire identifiée dans le premier modèle d'activité décrivant une position dans le temps et une courbure de la trajectoire identifiée;

l'identificateur de trajectoires étant par ailleurs réalisé de manière à fournir, sur base du deuxième modèle d'activité, une information (750, 752, 754, 756) sur une trajectoire identifiée dans le deuxième modèle d'activité, l'information sur la trajectoire identifiée dans le deuxième modèle d'activité décrivant une position dans le temps et une courbure de la trajectoire identifiée dans;

l'identificateur étant réalisé de manière à déterminer, sur base de l'information sur la trajectoire identifiée dans le premier modèle d'activité et de l'information sur la trajectoire identifiée dans le deuxième modèle d'activité, si la première trajectoire identifiée et la deuxième trajectoire identifiée sont associées à un même événement sonore; et l'identificateur étant par ailleurs réalisé de manière à déterminer, sur base de l'information sur la trajectoire identifiée dans le premier modèle d'activité et sur la trajectoire identifiée dans le deuxième modèle d'activité, un décalage dans le temps de la première trajectoire identifiée et de la deuxième trajectoire identifiée.

9. Dispositif (100; 200) selon l'une des revendications 5 à 8, dans lequel l'identificateur (120) présente un moyen de décalage (500) qui est réalisé de manière à décaler le premier modèle d'activité (110) et le deuxième modèle d'activité (112) en parallèle en direction opposée dans un champ de cellules de registre de décalage (580),

le champ de cellules de registre de décalage présentant une pluralité de rangées et une pluralité de colonnes;

le champ de cellules de registre de décalage étant réalisé de manière à recevoir le premier modèle d'activité comme une pluralité (510) de signaux de lignes parallèles comme signal d'entrée d'une première colonne, et à recevoir le deuxième modèle d'activité comme une pluralité de signaux de lignes parallèles comme signal d'entrée d'une dernière colonne;

une cellule de registre de décalage est disposée dans une ligne considérée et dans une colonne considérée, pour mémoriser une valeur d'information du premier modèle d'activité pouvant adopter un état actif et un état inactif, et une valeur d'information du deuxième modèle d'activité pouvant adopter un état actif et un état inactif, pour transmettre la valeur d'information appartenant au premier modèle d'activité à une cellule de registre de décalage adjacente de la ligne considérée et à une première colonne adjacente, et à transmettre la valeur d'information appartenant au deuxième modèle d'activité à une cellule de registre de décalage adjacente de la ligne considérée et d'une deuxième colonne adjacente;

une cellule de registre de décalage étant par ailleurs réalisée de manière à identifier un événement de coïncidence lorsque des valeurs d'information présentes dans la cellule de registre de décalage, appartenant au premier modèle d'activité et au deuxième modèle d'activité, indiquent simultanément un état actif; et

l'identificateur étant par ailleurs réalisé de manière à déterminer séparément, pour la pluralité de colonnes, le nombre d'événements de coïncidence qui se sont présentés, dans un intervalle de temps prédéterminé, dans une colonne considérée et à déterminer sur cette base si, et dans l'affirmative avec quel décalage dans le temps, des trajectoires associées à un même événement sonore se sont présentés dans les modèles d'activité, et

le premier identificateur (120) comportant une pluralité de compteurs, d'additionneurs ou d'intégrateurs qui sont associés à des colonnes du champ et qui sont réalisés de manière à déterminer le nombre d'événements de coïncidence qui se sont présentés dans les colonnes dans l'intervalle de temps considéré.

10. Dispositif (100; 200) selon l'une des revendications 8 à 9, dans lequel l'identificateur de trajectoires comporte un moyen d'identification de modèle qui est réalisé de manière à identifier, dans une représentation bidimensionnelle qui est formée dans le temps par le modèle d'activité, un modèle en forme de ligne droite ou courbée comme trajectoire, à déterminer la position dans le temps de la trajectoire et à fournir une information temporelle appartenant à la trajectoire,

le moyen d'identification de modèle étant réalisé de manière à déformer une représentation bidimensionnelle du modèle d'activité pas à pas dans le temps, pour obtenir une représentation bidimensionnelle déformée du modèle d'activité dans le temps, à identifier s'il est contenu dans la représentation bidimensionnelle déformée du modèle d'activité dans le temps une ligne approximativement droite, à identifier la ligne approximativement droite comme trajectoire, à déterminer la position dans le temps de la trajectoire, et à fournir l'information temporelle appartenant à la trajectoire,

le moyen d'identification de modèle étant réalisé de manière à déformer la représentation bidimensionnelle du modèle d'activité nerveuse pas à pas dans le temps, de sorte qu'une trajectoire courbée dans le modèle d'activité nerveuse soit redressée pas à pas par déformation pas à pas, un nombre d'étapes de déformation requises pour un redressement de la trajectoire courbée dépendant d'une courbure de la trajectoire courbe, et un nombre d'étapes de déformation requises pour un redressement de la trajectoire courbée comportant une indication sur une forme originale de la trajectoire,

le moyen d'identification de modèle comportant un moyen d'identification de courbe qui est réalisé de manière à recevoir en parallèle le modèle d'activité nerveux sous forme d'une pluralité de signaux, pour transmettre les signaux en parallèle à une vitesse différente à travers une pluralité d'étages connectés l'un après l'autre, au moins un étage prédéterminé présentant un moyen d'identification de seuil qui est réalisé de manière à identifier le moment où au moins un nombre prédéterminé de signaux sont simultanément actifs dans l'étage prédéterminé, et au moins un étage étant réalisé de manière à retarder de manière différente plusieurs signaux lors d'une transmission à travers l'étage.

**11.** Dispositif selon l'une des revendications 1 à 10, dans lequel le dispositif est réalisé de manière à recevoir, comme premier modèle d'activité, un modèle d'activité dans ou à des cellules auditives intérieures d'une première sensibilité de réaction au modèle auditif de la première oreille, à recevoir, comme deuxième modèle d'activité, un modèle d'activité dans ou à des cellules auditives intérieures de la première sensibilité de réaction au modèle auditif de la deuxième oreille,

à recevoir un troisième modèle d'activité dans ou à des cellules auditives intérieures d'une deuxième sensibilité de réaction au modèle auditif de la première oreille, et à recevoir un quatrième modèle d'activité dans ou à des cellules auditives intérieures de la deuxième sensibilité de réaction au modèle auditif de la deuxième oreille,

l'identificateur (120) étant réalisé de manière à identifier une troisième trajectoire dans le troisième modèle d'activité et une quatrième trajectoire dans le quatrième modèle d'activité qui sont associées à un même événement sonore;

le déterminateur étant réalisé de manière à déterminer si la première trajectoire, la deuxième trajectoire, la troisième trajectoire et la quatrième trajectoire sont associées à un événement sonore d'une source sonore utile (462),

le déterminateur étant réalisé de manière à déterminer, sur base d'un résultat d'une comparaison, si dans le premier modèle d'activité et dans le troisième modèle d'activité sont présentes, dans une plage de tolérances prédéterminée dans le temps, des trajectoires à courbure identique dans une plage de tolérances de courbure prédéterminée, une information d'intensité sonore pour les trajectoires et, de ce fait, pour les événements d'activité associées aux trajectoires,

et à déterminer l'information relative à si les trajectoires identifiées sont associées à un événement sonore utile, sur base de l'information d'intensité sonore.

**12.** Séparateur de sources (1300) pour générer un signal audio nettoyé (1330) sur base d'un signal audio avec au moins deux canaux, aux caractéristiques suivantes:

un générateur de modèle d'activité (1340, 1342) destiné à générer un premier modèle d'activité (110) sur un modèle auditif d'une première oreille sur base d'un premier canal (1310) du signal audio, et à générer un deuxième modèle d'activité (112) sur un modèle auditif d'une deuxième oreille sur base d'un deuxième canal (1320) du signal audio;

un dispositif (100; 200) destiné à générer un modèle d'activité filtré (146) sur base du premier modèle d'activité (110) et du deuxième modèle d'activité (112), selon l'une des revendications 1 à 11;

un synthétiseur (1350) destiné à convertir le modèle d'activité filtré (146) en une représentation dans le temps, une représentation en fréquence ou une représentation en sous-bandes, pour obtenir le signal audio nettoyé (1330).

**13.** Procédé (1200) pour générer un modèle d'activité filtré (146) sur base d'un premier modèle d'activité (110) sur un modèle auditif d'une première oreille et d'un deuxième modèle d'activité (112) sur un modèle auditif d'une deuxième oreille, aux étapes suivantes consistant à:

identifier (1210) une première trajectoire (430, 432, 434) dans le premier modèle d'activité (110) et une deuxième trajectoire (440, 442, 444) dans le deuxième modèle d'activité (112) qui sont associées à des événements sonores identiques;

déterminer un décalage dans le temps entre les deux trajectoires identifiées qui sont associées au même événement sonore;

déterminer (1220) si les deux trajectoires (430, 440; 432, 442; 434, 444) sont associées à un événement sonore d'une source sonore utile (462); et

filtrer (1230) le premier modèle d'activité ou le deuxième modèle d'activité sur base d'un résultat (136) de la détermination (1220) de si une trajectoire est associée à un événement sonore de la source sonore utile (462), de sorte que dans un modèle d'activité filtré dominent des événements d'activité associés à un événement sonore de la source sonore utile sur des événements d'activité qui ne sont pas associés à un événement sonore de la source sonore utile, ou que les événements d'activité qui ne sont pas associés à un événement sonore de la source sonore ne sont plus présents dans le modèle d'activité filtré;

le premier modèle d'activité (110) se basant sur un premier signal audio traité par le modèle auditif de la première oreille, et le deuxième modèle d'activité (112) se basant sur un deuxième signal audio traité par le modèle auditif de la deuxième oreille;

le premier modèle d'activité et le deuxième modèle d'activité décrivant deux signaux audio de sources de signal audio différentes ou des signaux audio de deux canaux d'un signal audio multicanal;

une trajectoire dans le modèle d'activité décrivant des événements d'activité allant ensemble dans le modèle d'activité qui sont associés à une onde progressive sur une membrane basilaire du modèle d'oreille;

l'identification d'une première trajectoire et d'une deuxième trajectoire comprenant une identification d'une première trajectoire courbée dans le premier modèle d'activité et d'une deuxième trajectoire courbée dans le deuxième modèle d'activité comme trajectoires appartenant à un même événement sonore lorsque la première trajectoire et la deuxième trajectoire présentent, dans une plage de tolérances prédéterminée, une même courbure; et

la détermination de si les deux trajectoires identifiées (430, 440; 432, 442; 434, 444) sont associées à un événement sonore d'une source sonore utile (462) ayant lieu à l'aide du décalage dans le temps.

**14.** Procédé (1400) pour générer un signal audio nettoyé (1330) sur base d'un signal audio avec au moins deux canaux, aux caractéristiques suivantes:

générer (1410) un premier modèle d'activité (110) sur un modèle auditif d'une première oreille sur base d'un premier canal (1310) du signal audio, et générer (1420) un deuxième modèle d'activité (112) sur un modèle auditif d'une deuxième oreille sur base d'un deuxième canal (1320) du signal audio;

générer un modèle d'activité filtré (146) sur base du premier modèle d'activité (110) et du deuxième modèle d'activité (112), selon la revendication 13; et

convertir (1440) le modèle d'activité filtré en une représentation dans le temps, une représentation en fréquence ou une représentation en sous-bandes, pour obtenir le signal audio nettoyé (1330).

**15.** Programme d'ordinateur pour réaliser un procédé selon la revendication 13 ou 14 lorsque le programme d'ordinateur est exécuté sur un ordinateur.

# FIG 1

100

1. Aktivitätsmuster        2. Aktivitätsmuster

110        112

t  122        t  124

120

alternativ        126        alternativ

130

136

140

146

# FIG 2

200

1. Aktivitätsmuster    2. Aktivitätsmuster

110        112

alternativ    122    st    124    alternativ

120

st    126

130

136

140

146

## FIG 3A

300

Anzahl an
Neurotransmitter-
Vesikeln

Anzahl an
Neurotransmitter-
Vesikeln

Anzahl an
Neurotransmitter-
Vesikeln

Anzahl an
Neurotransmitter-
Vesikeln

IHZ 1

IHZ 2

IHZ 3

IHZ 4

302

306

310

312

315

320

308

313

330

318

323

304

309

314

319

324

t

332

FIG 3B

340

| Nervenfaser-<br>Aktivität | Nervenfaser-<br>Aktivität | Nervenfaser-<br>Aktivität | Nervenfaser-<br>Aktivität |
|---|---|---|---|
| NF 1 | NF 2 | NF 3 | NF 4 |

342   346   360   365   370

348   380   363   368   373

344   350   364   369   373

t   382   374

FIG 3C

390

392   399a   394   396   398

399b

t

# FIG 3D

# FIG 4A

# FIG 4B

480

482

484

440

430

$st_1$

432

$st_2$

442

# FIG 5A

EP 2 024 022 B1

## FIG 5B

# FIG 5C

# FIG 6A

## FIG 6B

EP 2 024 022 B1

57

**FIG 7**

# FIG 8

15000

von Nervenzelle 1    von Nervenzelle 2    von Nervenzelle n
~15200                ~15220                ~15240

15100~

| Verzögerng 1,1 | Verzögerng 1,2 | Verzögerng 1,3 |

$\Sigma_1$

~15300

15320~        15340~        15360~

15120~

| Verzögerng 2,1 | Verzögerng 2,2 | Verzögerng 2,3 |

$\Sigma_2$

15140~

| Verzögerng i,1 | Verzögerng i,2 | Verzögerng i,3 |

$\Sigma_i$

EP 2 024 022 B1

# FIG 9

von Nerven-  von  von  von
Zelle 1  NZ 2  NZ 3  NZ 4  16140

16000

Eingänge
von Stufe 1

16100

16120

16120

16120

t

Σ=1

16120

---

von  von  von  von
NZ 1  NZ 2  NZ 3  NZ 4

nach Stufe 1
(Ausgänge der
1. Stufe=Eingänge
der 2. Stufe)

16240  16200

16220

16220

t

Σ=1

16220  16220

---

von  von  von  von
NZ 1  NZ 2  NZ 3  NZ 4

16300

nach Stufe 2
(Ausgänge der
2. Stufe=Eingänge
der 3. Stufe)

16340  Σ=4

t

trajektorie
geradegebogen

16320  16320  16320  16320

---

von  von  von  von
NZ 1  NZ 2  NZ 3  NZ 4

16400

nach Stufe 3

t

Σ=1

16440

16420  16420  16420

16420

# FIG 10

das Schaltbild des Hubel-Wiesel Netzes

Verzögerungselemente/Addierer

Schwellwertregister/
Komparatoren

EP 2 024 022 B1

## FIG 11

2000

2100 — Audiosignal

2140 — Mechanisch Schallwandlung im Außen-Ohr

2180 — Anregung des Trommelfells

2220 — Schallübertragung über Gehörknöchelchen

2260 — Anregung des ovalen Fensters zwischen Mittelohr und Cochlea

2300 — Berechnung der hydromechanischen Schwingungsanregung der Cochlea

2340 — Schwingungsanregung der Cochlea

2380 — Berechnung der Basilarmembran-Bewegung

2420 — Basilarmembran-Bewegung

2460 — Berechnung einer Auslenkung eines Stereoziliums

2520 — Auslenkung eines Stereoziliums

2560 — Berechnung einer Calcium-Konzentration

2600 — Calcium-Konzentration

2640 — Berechnung einer Transmitter-Freisetzungs-Rate

2680 — Transmitter-Freisetzungs-Rate

2720 — Berechnung einer Neurotransmitter-Versikel-Auftretens

2760 — Neurotransmitter-Versikel-Auftreten

2800 — Bestimmung eines Nervenaktivitätsmusters durch Bestimmung von den zu den Neurotransmitter-Versikel Auftreten gehörigen Aktionspotentialen

2840 — Nervenaktivitätsmuster

# FIG 12

1200

1210 — Identifizieren einer ersten Trajektorie in dem ersten Aktivitätsmuster und einer zweiten Trajektorie in dem zweiten Aktivitätsmuster, die einem gleichen Schallereignis zugeordnet sind

1220 — Bestimmen, ob die zwei Trajektorien einem Schallereignis einer Nutz-Schallquelle zugeordnet sind

1230 — Filtern des ersten Aktivitätsmusters oder des zweiten Aktivitätsmusters basierend auf einem Ergebnis des Bestimmens, ob eine Trajektorie einem Schallereignis der Nutz-Schallquelle zugeordnet ist, so daß in einem gefilterten Aktivitätsmuster Aktivitätsereignisse, die einem Schallereignis der Nutz-Schallquelle zugeordnet sind, dominieren, oder daß die Aktivitätsereignisse, die nicht einem Schallereignis der Nutz-Schallquelle zugeordnet sind, in dem gefilterten Aktivitätsmuster nicht mehr enthalten sind

# FIG 13

1300

Audiosignal
1. Kanal

Audiosignal
1. Kanal

1310

1310

| Aktivitätsmuster-Berechner (Gehörmodell) | Aktivitätsmuster-Berechner (Gehörmodell) |

1340

1340

110

112

t↓  122

t↓  124

126    120

136    130

140

146

1350

1330

# FIG 14

1400

**1410** — Erzeugen eines ersten Aktivitätsmusters an einem Gehörmodell eines ersten Ohrs basierend auf einem ersten Kanal des Audiosignals

**1420** — Erzeugen eines zweiten Aktivitätsmusters an einem Gehörmodell eines zweiten Ohrs basierend auf einem zweiten Kanal des Audiosignals

**1430** — Erzeugen eines gefilterten Aktivitätsmusters basierend auf dem ersten Aktivitätsmuster und dem zweiten Aktivitätsmuster

**1440** — Umwandeln des gefilterten Aktivitätsmusters in eine Zeit-Darstellung, Frequenz-Darstellung oder Subband-Darstellung, um das bereinigte Audiosignal zu erhalten

EP 2 024 022 B1

# FIG 15

# FIG 16

Charakteristische Frequenzen

Wanderwelle auf der Basilarmembran
der Cochlea

EP 2 024 022 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 200510030327
- WO 2007000231 A1
- DE 102005030327
- US 11172605 B
- US 6442510 B1

**In der Beschreibung aufgeführte Nicht-Patentliteratur**

- **T. Harczos.** *A revised neurobiologically parameterized model of the cochlea and an attached auditory image processing network*
- **T. Harczos ; F. Klefenz ; A. Kátai.** A neurobiologically inspired vowel recognizer using Hough-transform. *Proceedings VISAPP 2006,* 25. Februar 2006
- Neuronale Repräsentation des Hörvorgangs als Basis. **G. Szepannek ; F. Klefenz ; C. Weihs.** Informatik-Spektrum. Springer-Verlag, 28. September 2005
- **T. Harczos ; A. Katai ; F. Klefenz ; P. Schikowski ; G. Szepannek.** Feature Extraction for sound classification by means of a perceptionally motivated neurophysiologic parameterized auditory model. *veröffentlicht auf der Konferenz der Gesellschaft für Klassifikation GfKl 2006,* 08. Marz 2006